(19) Europäisches Patentamt
European Patent Office
Office européen des brevets

(11) EP 3 271 697 B1

(12) EUROPEAN PATENT SPECIFICATION

(45) Date of publication and mention of the grant of the patent:
**11.11.2020 Bulletin 2020/46**

(21) Application number: **16714642.2**

(22) Date of filing: **15.03.2016**

(51) Int Cl.:
***G01N 1/10*** *(2006.01)* ***B01D 46/00*** *(2006.01)*
***C12M 1/26*** *(2006.01)* ***C12Q 1/24*** *(2006.01)*
***C12M 1/12*** *(2006.01)* ***C12Q 1/04*** *(2006.01)*
***C12M 1/34*** *(2006.01)* ***G01N 1/40*** *(2006.01)*

(86) International application number:
**PCT/US2016/022411**

(87) International publication number:
**WO 2016/149235 (22.09.2016 Gazette 2016/38)**

# (54) METHODS, DEVICES, AND KITS FOR DETECTING MICROORGANISMS IN A FLUID SAMPLE

VERFAHREN, VORRICHTUNGEN UND KITS FÜR DEN NACHWEIS VON MIKROORGANISMEN IN EINER FLÜSSIGKEITSPROBE

PROCÉDÉS, DISPOSITIFS ET KITS DE DÉTECTION DE MICRO-ORGANISMES DANS UN ÉCHANTILLON DE FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.03.2015 US 201562135266 P**
**21.08.2015 US 201562208316 P**

(43) Date of publication of application:
**24.01.2018 Bulletin 2018/04**

(73) Proprietor: **3M Innovative Properties Company**
**St. Paul, MN 55133-3427 (US)**

(72) Inventors:
- **RAJAGOPAL, Raj**
  **Saint Paul, Minnesota 55133-3427 (US)**
- **KSHIRSAGAR, Manjiri T.**
  **Saint Paul, Minnesota 55133-3427 (US)**
- **BRUTINEL, Evan D.**
  **Saint Paul, Minnesota 55133-3427 (US)**
- **AYSTA, James E.**
  **Saint Paul, Minnesota 55133-3427 (US)**

(74) Representative: **Mathys & Squire**
**Mathys & Squire Europe LLP**
**Theatinerstraße 7**
**80333 München (DE)**

(56) References cited:
**WO-A1-2013/184186**
**WO-A2-2012/012172**
**US-A1- 2013 244 225**
**US-A1- 2013 260 370**

- **MARCIN SPYCHALA ET AL: "Bacteria in non-woven textile filters for domestic wastewater treatment", ENVIRONMENTAL TECHNOLOGY, vol. 36, no. 8, 21 October 2014 (2014-10-21), pages 937-945, XP055272123, GB ISSN: 0959-3330, DOI: 10.1080/09593330.2014.969326**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field

The present disclosure relates to methods of detecting microorganisms in a fluid sample and to devices and kits including the devices for use in detecting microorganisms.

### Background

Bacteria occur naturally in most aquatic systems and can cause problems in industrial and municipal applications using water such as cooling towers, heat exchangers, potable and wastewater applications, oil and gas exploration, and hydraulic fracturing operations. The bacteria need to be monitored to develop and implement an effective disinfection program to keep the bacteria within acceptable limits. The microbiological quality of water is typically assessed by traditional growth-based methods, which can take 24 to 48 hours to perform. Rapid methods based on ATP bioluminescence assays have been used to determine microbial contamination in water as they provide immediate results; however, the methods are limited by detection sensitivity because they require at least $1 \times 10^5$ colony forming units (cfu)/ml to elicit detectable responses. Interference from treatment chemicals or matrix components can affect the bioluminescence, leading to erroneous results and mismanagement of biocides, for instance. One can increase the sensitivity of the ATP bioluminescence assay by using a larger volume of sample (e.g., 100 ml), but such methods can be difficult to implement in the field.

US 2013/260370 A1 discloses a process for capturing or concentrating microorganisms for detection or assay comprises (a) providing a concentration device comprising (1) a porous fibrous nonwoven matrix and (2) a plurality of particles of at least one concentration agent that comprises a metal silicate, the particles being enmeshed in the porous fibrous nonwoven matrix; (b) providing a sample comprising at least one target cellular analyte; (c) contacting the concentration device with the sample such that at least a portion of the at least one target cellular analyte is bound to or captured by the concentration device; and (d) detecting the presence of at least one bound target cellular analyte.

US 2013/244225 A1 discloses process for capturing or concentrating microorganisms for detection or assay comprises (a) providing a concentration device comprising (1) a porous fibrous nonwoven matrix and (2) a plurality of particles of at least one concentration agent that comprises diatomaceous earth, the particles being enmeshed in the porous fibrous nonwoven matrix; (b) providing a sample comprising at least one target cellular analyte; (c) contacting the concentration device with the sample such that at least a portion of the at least one target cellular analyte is bound to or captured by the concentration device; and (d) detecting the presence of at least one bound target cellular analyte.

WO 2013/184186 A1 discloses a bismuth-containing concentration agent for microorganisms is provided. Additionally, articles that include the concentration agent and methods of concentrating a microorganism using the concentration agent are disclosed.

WO 2012/012172 A1 discloses a filter plate article and methods of using the same, the article comprising: a base member comprising a self-supporting water impervious substrate with first and second generally opposed major surfaces; a filter assembly defining a filter assembly aperture therein, and having a composite filter body mounted across the filter assembly aperture; wherein the composite filter body comprises: a microporous membrane, and a water-absorbent layer in fluid communication with the microporous membrane; and a cover sheet.

Spychala (2015, Environ Tech, 36(8), 937-945) discloses a culture based-method of concentrating and detecting of bacteria using devices comprising nonwoven filters in volumes of more than 300 mL.

### Summary

Devices and methods are provided that can be used to detect microorganisms in fluid samples, such as water or aqueous dispersions.

In a first aspect, a method of detecting microorganisms in a fluid sample is provided. The method includes providing a nonwoven article, providing a fluid sample suspected of containing at least one microorganism strain or target cellular analyte, and contacting the fluid sample with the nonwoven article such that at least a portion of the at least one microorganism strain or target cellular analyte is bound to the nonwoven article, and washing the at least one microorganism strain- or target cellular analyte-bound non-woven article. The nonwoven article includes a fibrous porous matrix and a plurality of concentration agent particles enmeshed in the fibrous porous matrix, wherein the concentration agent particles bind microorganisms and/or target cellular analytes from a fluid sample with a binding efficiency of at least 60 percent and wherein the target cellular analyte is a component of the nicroorganism. The method further includes placing the microorganism strain- or target cellular analyte-bound nonwoven article in contact with at least one detection reagent and detecting the presence of the bound microorganism strain or bound target cellular analyte.

In a second aspect, a use of a device for contacting a fluid sample with a nonwoven article in a method according

to the first aspect is provided. The device includes a sample container, a filter holder, a nonwoven article, and a first adaptor configured to interface the filter holder with a receptacle. The nonwoven article includes a fibrous porous matrix and a plurality of concentration agent particles enmeshed in the fibrous porous matrix, wherein the concentration agent particles bind microorganisms and/or target cellular analytes with a binding efficiency of at least 60 percent; and wherein the target cellular analyte is a component of the microorganism.

In a third aspect, a use of another device for contacting a fluid sample with a nonwoven article in a method according to the first aspect is provided. The device includes a sample container, a filter holder configured to interface with a receptacle, and a nonwoven article. The nonwoven article includes a fibrous porous matrix and a plurality of concentration agent particles enmeshed in the fibrous porous matrix, wherein the concentration agent particles bind microorganisms and/or target cellular analytes with a binding efficiency of at least 60 percent; and wherein the target cellular analyte is a component of the microorganism.

In a fourth aspect, a use of a kit in a method according to the first aspect is provided. The kit includes a device for contacting a fluid sample with a nonwoven article and a receptacle. The device includes a sample container, a filter holder, a nonwoven article, and a first adaptor. The first adaptor is configured to interface the filter holder with the receptacle. The nonwoven article includes a fibrous porous matrix and a plurality of concentration agent particles enmeshed in the fibrous porous matrix, wherein the concentration agent particles bind microorganisms and/or target cellular analytes with a binding efficiency of at least 60 percent ; and wherein the target cellular analyte is a component of the microorganism.

## Brief Description of the Drawings

Figure 1A is a schematic side view of an exemplary sample container according to the disclosure.

Figure 1B is a schematic cross-sectional view of an exemplary filter holder according to the disclosure.

Figure 1C is a schematic top view of an exemplary nonwoven article according to the disclosure.

Figure 1D is a schematic end view of the filter holder of Figure 1B.

Figure 1E is a schematic side view of the container of Figure 1A threaded onto the filter holder of Figure 1B, the filter holder containing the nonwoven article of Figure 1C.

Figure 2A a schematic side view of an exemplary sample container according to the disclosure.

Figure 2B is a schematic side view of an exemplary second adaptor according to the disclosure.

Figure 2C is a schematic side view of an exemplary filter holder threaded onto the second adaptor of Figure 2B according to the disclosure.

Figure 2D is a schematic side view of the container of Figure 2A threaded onto the filter holder and second adaptor combination of Figure 2C.

Figure 2E is a schematic side view of the device of 2D attached to a stopper.

Figure 3A a schematic side view of an exemplary sample container according to the disclosure.

Figure 3B is a schematic side view of another exemplary second adaptor according to the disclosure.

Figure 3C is a schematic side view of an exemplary filter holder threaded onto the second adaptor of Figure 3B according to the disclosure.

Figure 3D is a schematic side view of the container of Figure 3A threaded onto the filter holder and second adaptor combination of Figure 3C.

Figure 3E is a schematic side view of the device of 2D attached to a collection container.

Figure 4A is a schematic side view of a further exemplary device according to the disclosure.

Figure 4B is a schematic top view of the device of Figure 4A, further including an exemplary vacuum source.

Figure 5 is a schematic side view of yet another exemplary device according to the disclosure.

Figure 6 is an exploded perspective view of a sample preparation system according to one embodiment of the present disclosure, the sample preparation system including a lid.

Figure 7A is a schematic perspective view of another exemplary filter holder according to the disclosure.

Figure 7B is a schematic perspective view of an exemplary first adaptor according to the disclosure.

Figure 7C is a schematic perspective view of the filter holder of Figure 7A containing a first adaptor and a nonwoven article according to the disclosure.

Figure 7D is a schematic perspective view of a sample delivery system according to the disclosure.

Figure 7E is a schematic side view of the filter holder of Figure 7C containing the first adaptor of Figure 7B and connected to the sample delivery system of Figure 7D.

Figure 8A is a schematic perspective view of a detection device handle being inserted into a filter holder according to the disclosure.

Figure 8B is a schematic perspective view of the detection device handle of Figure 8A being removed from the filter

holder and removing a first adaptor and nonwoven article with the handle.

Figure 8C is a schematic side view of the detection device handle of Figure 8B being inserted in a detection device.

Figure 8D is a schematic side view of the detection device handle of Figure 8C being completely inserted in the detection device.

Figure 8E is a schematic side view of the detection device of Figure 8D operationally coupled to a luminometer.

Figure 9A is a schematic side view of a syringe adaptor.

Figure 9B is another schematic perspective view of a syringe adaptor.

Figure 9C is a schematic perspective view of a filter holder containing a first adaptor.

Figure 9D is a schematic perspective view of a filter holder containing a nonwoven article secured to a first adaptor with a gasket.

Figure 9E is a schematic perspective view of the syringe adaptor of Figure 9B coupled to the filter holder of Figure 9D.

Figure 9F is a schematic side view of a device including a syringe, the syringe adaptor of Figure 9A, and the filter holder of Figure 9D.

Figure 10A is a schematic perspective view of a first filter holder portion.

Figure 10B is another schematic perspective view of a first filter holder portion.

Figure 10C is a schematic perspective view of a first adaptor.

Figure 10D is a schematic perspective view of the first filter holder portion of Figure 10B containing the first adaptor of Figure 10C.

Figure 10E is a schematic perspective view of the first filter holder portion of Figure 10D further containing a nonwoven article and a gasket.

Figure 10F is a schematic perspective view of a second filter holder portion.

Figure 10G is an exploded side view of a device according to the disclosure.

Figure 10H is a schematic side view of the assembled device of Figure 10G.

Figure 11A is a schematic perspective view of a detection device handle being removed from a first filter holder portion and removing a first adaptor and nonwoven article with the handle.

Figure 11B is a schematic side view of the detection device handle of Figure 11A being inserted in a detection device.

Figure 11C is a schematic side view of the detection device handle of Figure 11B being completely inserted in the detection device.

## Detailed Description

Rapid methods for monitoring of microbial quality of fluid samples are provided. The methods combine devices that concentrate at least one microorganism or target cellular analyte by a nonwoven article, and detection of the microorganism or target cellular analyte. Devices according to the disclosure enable contact with large volumes of fluid samples, and also allow further optional washing to remove contaminants prior to detection. The nonwoven article is easily transferred to a receptacle for detection. Methods according to the disclosure are capable of readily detecting bacterial contamination in water in about 15 minutes. Accordingly, the methods and devices are suitable for field based detection of microorganisms and target cellular analytes in fluid samples.

For the following Glossary of defined terms, these definitions shall be applied for the entire application, unless a different definition is provided in the claims or elsewhere in the specification.

### Glossary

Certain terms are used throughout the description and the claims that, while for the most part are well known, may require some explanation. It should be understood that, as used herein:

The term "a", "an", and "the" are used interchangeably with "at least one" to mean one or more of the elements being described.

The term "and/or" means either or both. For example "A and/or B" means only A, only B, or both A and B.

As used in this specification, the recitation of numerical ranges by endpoints includes all numbers subsumed within that range (e.g. 1 to 5 includes 1, 1.5, 2, 2.75, 3, 3.8, 4, and 5).

Unless otherwise indicated, all numbers expressing quantities or ingredients, measurement of properties and so forth used in the specification and embodiments are to be understood as being modified in all instances by the term "about." Accordingly, unless indicated to the contrary, the numerical parameters set forth in the foregoing specification and attached listing of embodiments can vary depending upon the desired properties sought to be obtained by those skilled in the art utilizing the teachings of the present disclosure. At the very least, and not as an attempt to limit the application of the doctrine of equivalents to the scope of the claimed embodiments, each numerical parameter should at least be construed in light of the number of reported significant digits and by applying ordinary rounding techniques.

The terms "comprises" and variations thereof do not have a limiting meaning where these terms appear in the

description and claims.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure.

Unless specified or limited otherwise, the terms "supported," and "coupled" and variations thereof are used broadly and encompass both direct and indirect supports and couplings. It is to be understood that other embodiments may be utilized, and structural or logical changes may be made without departing from the scope of the present disclosure. Furthermore, terms such as "front," "rear," "top," "bottom," and the like are only used to describe elements as they relate to one another, but are in no way meant to recite specific orientations of the apparatus, to indicate or imply necessary or required orientations of the apparatus, or to specify how the invention described herein will be used, mounted, displayed, or positioned in use.

The term "cellular analyte" means an analyte of cellular origin (that is, a microorganism or a component thereof (for example, a cell or a cellular component such as deoxyribonucleic acid (DNA) or ribonucleic acid (RNA), proteins, nucleotides such as adenosine triphosphate (ATP), and the like, and combinations thereof); references to a microorganism or microorganism strain throughout this specification are meant to apply more generally to any cellular analyte).

The term "concentration agent" means a material or composition that binds microorganisms and/or cellular analytes from a fluid sample, having a cellular analyte capture or binding efficiency of at least about 60 percent (preferably at least about 70 percent, or at least about 80 percent, or at least about 90 percent), thereby concentrating the microorganisms and/or cellular analytes into a smaller volume than when present in the fluid sample.

The term "detection" means the identification of a cellular analyte (for example, at least a component of a target microorganism, which thereby determines that the target microorganism is present).

The term "enmeshed" (in regard to particles in a fibrous porous matrix) means that the particles are entrapped in and on the fibrous porous matrix (and, preferably, distributed within it), rather than solely being borne on its surface.

The term "fibrillated" (in regard to fibers or fibrous material) means treated (for example, by beating) in a manner that forms fibrils or branches attached to a fiber's main trunk.

The term "fibrous porous matrix" means a nonwoven web or medium, (i.e., not a woven or knitted fabric), comprising interlaid fibers, for example, a web comprising fibers that are interlaid by meltblowing, spunbonding, or other air laying techniques; carding; wet laying; or the like. Typically, the fibers have lengths of less than 100 millimeters and are uncrimped.

The term "filtering" is generally used to describe the process of separating matter by size, charge and/or function. For example, filtering can include separating soluble matter and a solvent (e.g., diluent) from insoluble matter, or it can include separating soluble matter, a solvent and relatively small insoluble matter from relatively large insoluble matter. A variety of filtration methods can be used, including, but not limited to, passing the liquid composition through a filter, settling followed by aspiration or decanting, other suitable filtration methods, and combinations thereof. "Settling" is used to refer to allowing the insoluble matter in the liquid composition to settle. Settling may occur by gravity or by centrifugation. The insoluble matter (or relatively large insoluble matter) can then be separated from the soluble matter (or soluble matter and relatively small insoluble matter) and solvent by aspirating the soluble matter and solvent from the insoluble matter, decanting the soluble matter and solvent, or a combination thereof.

The term "filtrate" is generally used to describe the liquid remaining after the insoluble matter (or at least the relatively large insoluble matter) has been removed from the liquid composition.

The term "fluid" means liquid, solution, or dispersion of solid or liquid in liquid.

The term "microorganism" means any cell or particle having genetic material suitable for analysis or detection (including, for example, bacteria, yeasts, viruses, and bacterial endospores).

The term "microorganism strain" means a particular type of microorganism that is distinguishable through a detection method (for example, microorganisms of different genera, of different species within a genera, or of different isolates within a species).

The terms "polymer" and "polymeric material" are used interchangeably and refer to materials formed by reacting one or more monomers.

The term "sample" means a substance or material that is collected (for example, to be analyzed).

The term "sample matrix" means the components of a sample other than microorganisms and/or cellular analytes.

The term "target cellular analyte" means any cellular analyte that is desired to be detected.

The term "target microorganism" means any microorganism that is desired to be detected.

Reference throughout this specification to "one embodiment," "certain embodiments," "one or more embodiments" or "an embodiment," whether or not including the term "exemplary" preceding the term "embodiment," means that a particular feature, structure, material, or characteristic described in connection with the embodiment is included in at least one embodiment of the certain exemplary embodiments of the present disclosure. Thus, the appearances of

the phrases such as "in one or more embodiments," "in some embodiments," "in certain embodiments," "in one embodiment," "in many embodiments" or "in an embodiment" in various places throughout this specification are not necessarily referring to the same embodiment of the certain exemplary embodiments of the present disclosure. Furthermore, the particular features, structures, materials, or characteristics may be combined in any suitable manner in one or more embodiments.

Various exemplary embodiments of the disclosure will now be described. Exemplary embodiments of the present disclosure may take on various modifications and alterations without departing from the scope of the invention. Accordingly, it is to be understood that the embodiments of the present disclosure are not to be limited to the following described exemplary embodiments, but are to be controlled by the limitations set forth in the claims and any equivalents thereof.

In a first aspect, a method of detecting microorganisms in a fluid sample is provided. The method includes providing a nonwoven article, providing a fluid sample suspected of containing at least one microorganism strain or target cellular analyte, and contacting the fluid sample with the nonwoven article such that at least a portion of the at least one microorganism strain or target cellular analyte is bound to the nonwoven article, and washing the at least one microorganism strain- or target cellular analyte-bound non-woven article. The nonwoven article includes a fibrous porous matrix and a plurality of concentration agent particles enmeshed in the fibrous porous matrix, wherein the concentration agent particles bind microorganisms from a fluid sample with a binding efficiency of at least 60 percent. The method further includes placing the microorganism strain- or target cellular analyte-bound nonwoven article in contact with at least one detection reagent and detecting the presence of the bound microorganism strain or bound target cellular analyte.

A variety of microorganisms can be concentrated and detected by using the methods of the disclosure, including, for example, bacteria, fungi, yeasts, protozoans, viruses (including both non-enveloped and enveloped viruses), fungal spores, bacterial endospores (for example, *Bacillus* (including *Bacillus anthracis, Bacillus cereus,* and *Bacillus subtilis*) and *Clostridium* (including *Clostridium botulinum, Clostridium difficile,* and *Clostridium perfringens*)), and the like, and combinations thereof, such as gram-negative bacteria, gram-positive bacteria, yeasts, fungi, and combinations thereof. Target cellular analyte that can be concentrated and detected by using the methods of the disclosure include nucleic acids, proteins, adenosine triphosphate (ATP), or combinations thereof.

Capturing or binding of microorganisms, cellular analytes, or combinations thereof is accomplished by contacting a fluid sample with a nonwoven article. In certain embodiments, contacting comprises filtration of the fluid sample through the nonwoven article. In select embodiments, contacting comprises passing the fluid sample at least twice through the nonwoven article, such as by sending a filtrate through the nonwoven article a second time or by placing the nonwoven article into a volume of the fluid sample and agitating the fluid sample such that sample passes through the nonwoven article multiple times. The method optionally further comprises passing the fluid sample through a coarse filter prior to the contacting the fluid sample with the nonwoven article. The use of such a filter can remove particulates from the fluid sample that might otherwise clog the nonwoven article. Suitable coarse filters include for example and without limitation, filters comprising pore sizes of at least 1 micrometer, at least 5 micrometers, at least 10 micrometers, at least 25 micrometers, or at least 50 micrometers.

Advantageously, the nonwoven articles of the present disclosure require only a very low pressure differential across the nonwoven article to effectively pass a fluid sample through the nonwoven article. This characteristic is particularly beneficial in environments, for instance, in a field situation, and/or when no or low power pumps are available for transporting a fluid sample. In an embodiment of the present disclosure, the contacting comprises passing the fluid sample through the laminated article at a pressure of 14.7 pounds per square inch (psi) (101.3 kilopascals (kPa)) or less, or 4.0 pounds per square inch (psi) (27.58 kilopascals (kPa)) or less, or 3.0 psi (20.68 kPa), or 2.0 psi (13.79 kPa), or 1.0 psi (6.9 kPa), or 0.9 psi (6.21 kPa), or 0.8 psi (5.52 kPa), or 0.7 psi (4.83 kPa), or 0.6 psi (4.14 kPa), or even 0.5 psi (3.45 kPa)or less, and at a pressure of at least 0.4 psi (2.76 kPa), or at least 0.5 psi (3.45 kPa).

The method further comprises washing the at least one microorganism strain- or target cellular analyte-bound nonwoven article prior to placing the at least one microorganism strain- or cellular analyte-bound nonwoven article in contact with at least one detection reagent. It has been discovered that contaminants such as residual sample matrix can be removed from the nonwoven article without significant loss of the bound or captured microorganisms and/or cellular analytes. In certain embodiments, washing includes for instance and without limitation, rinsing with sterile deionized water or bottled drinking water, or rinsing with aqueous salt or buffer solutions. Washing the nonwoven article tends to remove components that could otherwise interfere with detecting the presence of the bound microorganisms and/or cellular analytes, depending on the particular detection method employed.

In certain embodiments, placing the microorganism strain- or target cellular analyte-bound nonwoven article in contact with a reagent includes placing the nonwoven article in a receptacle that comprises a material through which a detection signal can be detected, wherein the receptacle contains at least one reagent. For instance, placing the microorganism strain- or target cellular analyte-bound nonwoven article in contact with a reagent optionally includes placing nonwoven the article in a receptacle configured to be operationally coupled to a luminometer, wherein the receptacle contains at least one reagent. Hence, in such an embodiment, detection is facilitated by disposing the receptacle in the luminometer for measurement of light generated from reaction of the bound microorganism strain and/or target cellular analyte with at least one reagent. Similarly, the receptacle can be interfaced with other types of equipment depending on the particular detection method. In certain embodiments, placing the microorganism strain- or target cellular analyte-bound nonwoven article in contact with a reagent includes pushing the nonwoven article into a receptacle containing the at least one reagent. It has been discovered that microorganism strain(s) and/or target cellular analyte(s) can be detected without requiring removal from being captured by the nonwoven article. The ability to detect microorganism strains and/or target cellular analytes attached to the nonwoven article is advantageous because it decreases the number of required method steps as compared to methods in which the microorganism strain(s) and/or target cellular analyte(s) need to be eluted from a nonwoven article prior to detection. Further, the nonwoven article concentrates the microorganism strains and/or target cellular analytes into the volume of the article, which is typically significantly smaller than the volume of the fluid sample contacted with the nonwoven article.

Microorganisms and/or cellular analytes that have been captured or bound (for example, by adsorption, absorption, or by sieving) by the nonwoven article can be detected by essentially any desired method that is currently known or hereafter developed. Such methods include, for example, culture-based methods, microscopy (for example, using a transmitted light microscope or an epifluorescence microscope, which can be used for visualizing microorganisms tagged with fluorescent dyes) and other imaging methods, immunological detection methods, and genetic detection methods. The detection process following microorganism and/or cellular analyte capture optionally can include washing to remove sample matrix components, staining, boiling or using elution buffers or lysis agents to release cellular analyte from the concentration device, or the like.

Immunological detection is detection of an antigenic material derived from a target organism, which is commonly a biological molecule (for example, a protein or proteoglycan) acting as a marker on the surface of bacteria or viral particles. Detection of the antigenic material typically can be by an antibody, a polypeptide selected from a process such as phage display, or an aptamer from a screening process.

Immunological detection methods are well-known and include, for example, immunoprecipitation and enzyme-linked immunosorbent assay (ELISA). Antibody binding can be detected in a variety of ways (for example, by labeling either a primary or a secondary antibody with a fluorescent dye, with a quantum dot, or with an enzyme that can produce chemiluminescence or a colored substrate, and using either a plate reader or a lateral flow device).

Detection can also be carried out by genetic assay (for example, by nucleic acid hybridization or primer directed amplification). The captured or bound microorganisms can be lysed to render their genetic material (e.g., cellular analytes) available for assay. Lysis methods are well-known and include, for example, treatments such as sonication, osmotic shock, high temperature treatment (for example, from about 50°C to about 100°C), and incubation with an enzyme such as lysozyme, glucolase, zymolose, lyticase, proteinase K, proteinase E, and viral enolysins.

Many commonly-used genetic detection assays detect the nucleic acids of a specific microorganism, including the DNA and/or RNA. The stringency of conditions used in a genetic detection method correlates with the level of variation in nucleic acid sequence that is detected. Highly stringent conditions of salt concentration and temperature can limit the detection to the exact nucleic acid sequence of the target. Thus microorganism strains with small variations in a target nucleic acid sequence can be distinguished using a highly stringent genetic assay. Genetic detection can be based on nucleic acid hybridization where a single-stranded nucleic acid probe is hybridized to the denatured nucleic acids of the microorganism such that a double-stranded nucleic acid is produced, including the probe strand. One skilled in the art will be familiar with probe labels, such as radioactive, fluorescent, and chemiluminescent labels, for detecting the hybrid following gel electrophoresis, capillary electrophoresis, or other separation method.

Particularly useful genetic detection methods are based on primer directed nucleic acid amplification. Primer directed nucleic acid amplification methods include, for example, thermal cycling methods (for example, polymerase chain reaction (PCR), reverse transcriptase polymerase chain reaction (RT-PCR), and ligase chain reaction (LCR)), as well as isothermal methods and strand displacement amplification (SDA) (and combinations thereof; preferably, PCR or RT-PCR). Methods for detection of the amplified product are not limited and include, for example, gel electrophoresis separation and ethidium bromide staining, as well as detection of an incorporated fluorescent label or radio label in the product. Methods that do not require a separation step prior to detection of the amplified product can also be used (for example, real-time PCR or homogeneous detection).

Bioluminescence detection methods are well-known and include, for example, adenosine triphosphate (ATP) detection methods including those described in U.S. Patent No. 7,422,868 (Fan et al.). Bioluminescence detection methods for ATP often include the known luciferin-luciferase system in which luciferase enzyme catalyzes the oxidation of luciferin in the presence of ATP and a divalent cation (such as magnesium or calcium). Other luminescence-based detection methods can also be utilized.

In many embodiments, detection comprises a culture-based detection method, an imaging detection method, a fluorescence-based detection method, a colorimetric detection method, an immunological detection method, a genetic detection method, a bioluminescence-based detection method, or a combination thereof.

As noted above, the nonwoven article comprises 1) a fibrous porous matrix and 2) a plurality of concentration

agent particles enmeshed in the fibrous porous matrix. The nonwoven, fibrous porous matrix is often in the form of a layer of interlaid fibers that are not woven or knitted together. The nonwoven, fibrous porous matrix can be prepared by any suitable process such as, for example, air laying techniques, spunlaid techniques such as meltblowing or spunbonding, carding, wetlaying, and combinations thereof. In many embodiments, the fibrous nonwoven matrix is prepared by wetlaid techniques.

Fibers suitable for use in preparing the nonwoven fibrous porous matrix are usually pulpable or extrudable fibers such as those that are stable to radiation and/or to a variety of solvents. Optionally, at least some of the polymeric fibers can be selected to exhibit a degree of hydrophilicity. Useful fibers include polymeric fibers, inorganic fibers, and combinations thereof. More particularly, the fibers include a plurality of different types of fibers, including polyolefin fibers and fiberglass fibers.

Suitable polymeric fibers include those made from natural polymers (those derived from animal or vegetable sources) and/or synthetic polymers, including thermoplastic and solvent-dispersible polymers. Useful polymers include polyolefins (for example, poly(ethylene) (e.g., low density polyethylene, medium density polyethylene, high density polyethylene, etc.), polypropylene, poly(1-butene), copolymers of ethylene and propylene, alpha olefin copolymers such as copolymers of ethylene or propylene with 1-butene, 1-hexene, 1-octene, and 1-decene such as poly(ethylene-co-1-butene), poly(ethylene-co-1-butene-co-1-hexene), and the like); polylactic acid; poly(isoprenes); poly(butadienes); polyamides (for example, nylon 6, nylon 6,6, nylon 6,12, poly(iminoadipoyliminohexamethylene), poly(iminoadipoyliminodecamethylene), polycaprolactam, and the like); polyimides (for example, poly(pyromellitimide) and the like); polyethers; poly(ether sulfones) (for example, poly(diphenylether sulfone), poly(diphenylsulfone-co-diphenylene oxide sulfone), and the like); poly(sulfones); poly(vinyl esters) such as poly(vinyl acetates); copolymers of vinyl acetate (for example, poly(ethylene-co-vinyl acetate), copolymers in which at least some of the acetate groups have been hydrolyzed to provide various poly(vinyl alcohols) including poly(ethylene-co-vinyl alcohol), and the like); poly(phosphazenes); poly(vinyl ethers); poly(vinyl alcohols); polyaramids (for example, para-aramids such as poly(paraphenylene terephthalamide) and fibers sold under the trade designation "KEVLAR" by DuPont Co., Wilmington, DE, pulps of which are commercially available in various grades based on the length of the fibers that make up the pulp such as, for example, "KEVLAR 1F306" and "KEVLAR 1F694", both of which include aramid fibers that are at least 4 mm in length; and the like); wool; silk; cellulosic polymers (for example, cellulose, cellulose derivatives such as rayon, and the like); acrylic polymers (for example, polyacrylonitrile); polyesters (for example, polyethylene terephthalate); fluorinated polymers (for example, poly(vinyl fluoride), poly(vinylidene fluoride), copolymers of vinylidene fluoride such as poly(vinylidene fluoride-co-hexafluoropropylene), copolymers of chlorotrifluoroethylene such as poly(ethylene-co-chlorotrifluoroethylene), and the like); chlorinated polymers; poly(carbonates); and the like; and combinations thereof. In certain embodiments, the polymeric fibers comprise a polyolefin, a polysulfone, a polyamide, or a combination thereof.

Suitable inorganic fibers include those that contain at least one inorganic material selected from glasses, ceramics, and combinations thereof. These fibers are often added to provide strength to the fibrous porous matrix. For example, porous matrix layers containing inorganic fibers are often capable of being bent, folded, or pleated without breaking apart. Useful inorganic fibers include, for example, fiberglass (for example, E-glass, S-glass, and the like), ceramic fibers (for example, fibers made of metal oxides (such as alumina), silicon carbide, boron nitride, boron carbide, and the like), and combinations thereof. Useful ceramic fibers can be at least partially crystalline (exhibiting a discernible X-ray powder diffraction pattern or containing both crystalline and amorphous (glass) phases). In some applications, the inorganic fibers include fiberglass.

To facilitate entrapment of the concentration agent particles and/or to ensure a high surface area, the fibers used to form the nonwoven, fibrous porous matrix often contain at least one fibrillated fiber (for example, in the form of a main fiber surrounded by many smaller attached fibrils). The main fiber generally can have a length in the range of 0.5 millimeters to 5 millimeters and a diameter in a range of 1 micrometer to 20 micrometers. The fibrils typically can have a sub-micrometer diameter. In many embodiments, the fibrillated fibers are prepared from a polyolefin such as poly(ethylene) or polypropylene, or from an acrylic polymer such as polyacrylonitrile.

Suitable polymeric fibers further include bi-component fibers, which typically assist in binding all of the matrix fibers together due to a difference in melting point of one of the materials in the bi-component fiber. Bi-component fibers can have, for example, a core-sheath structure, a side-by-side structure, an islands-in-the-sea structure, or a segmented-pie structure, or the like. An example side-by-side bi-component fiber is the polyolefin thermally bonded bi-component fiber that is commercially available from Chisso Corporation (Osaka, Japan) under the trade designation CHISSO (for example, CHISSO ES). An example core-sheath bi-component fiber is commercially available from Unitika Ltd. (Osaka, Japan) under the trade designation MELTY (for example, MELTY 4080) and those commercially available from Minifibers, Inc. (Johnson City, TN) made of ethyl vinyl acetate (sheath) and polypropylene (core), or made of a co-polyester of polyester and polyethylene terephthalate (PET) (sheath) and polyester (core).

The nonwoven fibrous porous matrix contains a plurality of different types of fibers. In some embodiments, the porous matrix can be formed using three, four, or even more different types of fibers. For example, a fiberglass fiber can be added for strength and integrity, while fibrillated poly(ethylene) can be added for entrapment of the particulates.

Additionally, nylon fibers provide hydrophilic character while fibrillated poly(ethylene) fibers provide hydrophobic character to the porous matrix. If fibrillated and non-fibrillated fibers are used in combination, the weight ratio of fibrillated fibers to non-fibrillated fibers is often at least 1:2, at least 1:1, at least 2:1, at least 3:1, at least 5:1, or even at least 8:1. In some embodiments, mixtures of hydrophobic and hydrophilic polymeric fibers are used. For example, the fibrous porous matrix can include a mixture of hydrophobic fibers such as polyolefins plus hydrophilic fibers such as polysulfones. In some specific examples, the polymeric fibers include polyolefin fibers, bi-component fibers, and fiberglass fibers.

In certain embodiments, the fibrous porous matrix is free of polyamide fibers. It has been discovered that the inclusion of nylon fibers in the fibrous porous matrix can result in lower luminescence than the fibrous porous matrix without the nylon fibers for a bioluminescent ATP detection method, as discussed in the Examples below.

Preferably, the fibers used to form the nonwoven fibrous porous matrix are uncrimped. In contrast to uncrimped fibers, crimped fibers may be identified as displaying repeating features (as manifested e.g., in a wavy, jagged, sinusoidal, etc., appearance of the fiber), by having a helical appearance (e.g., particularly in the case of crimped fibers obtained by thermal activation of bi-component fibers), and the like, and are readily recognizable by those of ordinary skill in the art. Exemplary crimped fibers are described in U.S. Pat. No. 4,118,531 to Hauser and U.S. Pat. No. 5,597,645 to Pike et al., and CA Patent 2612854 to Sommer et al.

The fibers used to form the nonwoven fibrous porous matrix can be of a length and diameter that can provide a porous matrix having sufficient structural integrity and sufficient porosity for a particular application (for example, passing a fluid sample through the matrix). The fiber lengths are often at least about 0.5 millimeter, at least 1 millimeter, at least 2 millimeters, at least 3 millimeters, at least 4 millimeters, at least 6 millimeters, at least 8 millimeters, at least 10 millimeters, at least 15 millimeters, or at least 20 millimeters, and up to 50 millimeters, up to 40 millimeters, up to 30 millimeters, or up to 25 millimeters. The diameter of the fibers can be, for example, at least 10 micrometers, at least 20 micrometers, or at least 30 micrometers. The fiber lengths and diameters will vary depending upon factors such as the nature of the fiber and the type of application.

The nonwoven fibrous porous matrix often includes a mixture of polyolefin fibers, glass fibers, and bi-component fibers. In some particular embodiments, the nonwoven fibrous porous matrix contains a mixture of fibrillated polyethylene fibers, glass fibers, and sheath-core bi-component fibers. In some examples, the nonwoven fibrous porous matrix contains 40 to 80 weight percent fibrillated polyethylene fibers, 5 to 20 weight percent glass fibers, and 5 to 20 weight percent bi-component fibers. In some examples, the nonwoven fibrous porous matrix contains 40 to 80 weight percent fibrillated polyethylene fibers, 10 to 30 weight percent nylon fibers, 5 to 20 weight percent glass fibers, and 5 to 20 weight percent bi-component fibers. In other examples, the nonwoven, fibrous porous matrix contains 50 to 70 weight percent fibrillated polyethylene fibers, 5 to 15 weight percent glass fibers, and 5 to 20 weight percent bi-component fibers. In still other examples, the fibrous porous matrix contains 55 to 65 weight percent fibrillated polyethylene fibers, 0 to 20 weight percent nylon fibers, 5 to 15 weight percent glass fibers, and 10 to 20 weight percent bi-component fibers.

In most embodiments, the fibrous porous matrix contains only fibers. For example, at least 90 weight percent, at least 95 weight percent, at least 98 weight percent, at least 99 weight percent, or at least 99.5 weight percent of a dry fibrous porous matrix is fibers. In certain embodiments, the nonwoven article comprises a thickness of at least 0.1 millimeters, or at least 0.15 millimeters, or at least 0.2 millimeters, or at least 0.3 millimeters, or at least 0.4 millimeters, or at least 0.5 millimeters, or at least 0.6 millimeters. The nonwoven article usually comprises a thickness of up to 1 millimeter, or up to 0.9 millimeters, or up to 0.8 millimeters, or up to 0.7 millimeters, or up to 0.55 millimeters. Stated differently, the nonwoven article may comprise a thickness of between 0.15 millimeters and 1 millimeter, or between 0.15 millimeters and 0.8 millimeters, or between 0.1 millimeters and 0.7 millimeters. In certain embodiments, a nonwoven article having a thickness towards the lower end of the thickness range is selected to minimize interference with detection of the microorganisms and/or cellular analytes, such as decreasing time required for a reagent to diffuse into the nonwoven article, or decreasing blockage of a generated detection signal.

The nonwoven article typically includes both the fibrous porous matrix and concentration agent particles distributed within the fibrous porous matrix. In most embodiments, the nonwoven article contains at least 10 weight percent concentration agent particles based on a total dry weight of the nonwoven article. If the amount of the concentration agent particles is lower than about 10 weight percent, the nonwoven article may not contain enough concentration agent particles to effectively capture microorganisms or cellular analytes from a fluid sample. In some examples, the nonwoven article contains at least 15 weight percent, at least 20 weight percent, at least 25 weight percent, or at least 30 weight percent concentration agent particles based on a total dry weight of the nonwoven article.

On the other hand, the nonwoven article usually contains no greater than 55 weight percent concentration agent particles based on the total dry weight of the nonwoven article. If the amount of the concentration agent particles is greater than about 55 weight percent, the nonwoven article may contain an insufficient amount of the fibrous porous matrix. That is, the strength of the nonwoven article may be insufficient to hold together when employed to capture microorganism strains and/or target cellular analytes. In some examples, the nonwoven article contains no greater than 50 weight percent, no greater than 45 weight percent, or no greater than 40 weight percent concentration agent particles based on a total weight of the nonwoven article.

Stated differently, the nonwoven article often contains 10 to 55 weight percent concentration agent particles and 45 to 90 weight percent fibrous porous matrix, 15 to 50 weight percent concentration agent particles and 50 to 85 weight percent fibrous porous matrix, 20 to 50 weight percent concentration agent particles and 50 to 80 weight percent fibrous porous matrix, 20 to 45 weight percent concentration agent particles and 55 to 80 weight percent fibrous porous matrix, 25 to 40 weight percent concentration agent particles and 60 to 75 weight percent fibrous porous matrix, or 30 to 40 weight percent concentration agent particles and 60 to 70 weight percent fibrous porous matrix. The amounts are based on the total dry weight of the nonwoven article.

In many embodiments, the nonwoven article (when dry) contains only concentration agent particles and fibrous porous matrix. For example, the nonwoven article contains at least 90 weight percent, at least 95 weight percent, at least 98 weight percent, at least 99 weight percent, or at least 99.5 weight percent combined concentration agent particles and fibrous porous matrix when dry.

Concentration agent particles are water-insoluble particulate materials that have been employed to non-specifically capture microorganism strains, cellular analytes, or a combination thereof, when contacted with fluid samples containing microorganisms and/or cellular analytes. The concentration agent particles typically comprise microparticles. The concentration agent particles typically comprise particles selected from the group consisting of amorphous metal silicates, guanidine-functionalized metal silicates, diatomaceous earth, surface-modified diatomaceous earth, guanidine-functionalized diatomaceous earth, gamma-FeO(OH), metal carbonates, metal phosphates, silica, perlite, guanidine-functionalized perlite, and combinations thereof.

In an embodiment, the concentration agent particles comprise particles of amorphous metal silicates, such as amorphous, spheroidized magnesium silicate, amorphous, spherical aluminum silicate, or a combination thereof. Amorphous, at least partially fused particulate forms of metal silicate can be prepared by any of the known methods of melting or softening relatively small feed particles (for example, average particle sizes up to about 25 micrometers) under controlled conditions to make generally ellipsoidal or spheroidal particles (that is, particles having magnified two-dimensional images that are generally rounded and free of sharp corners or edges, including truly or substantially circular and elliptical shapes and any other rounded or curved shapes). Such methods include atomization, fire polishing, direct fusion, and the like. A preferred method is flame fusion, in which at least partially fused, substantially glassy particles are formed by direct fusion or fire polishing of solid feed particles (for example, as in the method described in U.S. Patent No. 6,045,913 (Castle et al.). Most preferably, such methods can be utilized to produce amorphous, spheroidized metal silicates by converting a substantial portion of irregularly-shaped feed particles (for example, from about 15 to about 99 volume percent; preferably, from about 50 to about 99 volume percent; more preferably, from about 75 to about 99 volume percent; most preferably, from about 90 to about 99 volume percent) to generally ellipsoidal or spheroidal particles.

Some amorphous metal silicates are commercially available. For example, amorphous, spheroidized magnesium silicate was commercially available for use in cosmetic formulations (for example, "3M COSMETIC MICROSPHERES CM-111", available from 3M Company, St. Paul, MN). 3M COSMETIC MICROSPHERES CM-111 have a particle density of 2.3 g/cc, a surface area of 3.3 $m^2$/g, and have a particle size of: 90 percent less than 11 microns (i.e., $D_{90}$ = 11), 50 percent less than 5 microns, and 10 percent less than 2 microns. Amorphous aluminum silicate is commercially available for use in paints, primers, powder coatings, and other coatings, for example, "3M CERAMIC MICROSPHERES" from 3M Company, St. Paul, MN. The 3M CERAMIC MICROSPHERES are alkali alumino silicate ceramic microspheres shaped as solid spheres with particle density of 2.4 g/cc, and are commercially available in three grades: W-210, W-410, and W-610. W-210 particles have a surface area of 5 $m^2$/cc and a particle size of: 95 percent less than about 12 microns (i.e., $D_{95}$ = 12), 90 percent less than about 9 microns, 50 percent less than about 3 microns, and 10 percent less than about 1 micron. W-410 particles have a surface area of 3 $m^2$/cc and a particle size of: 95 percent less than about 24 microns (i.e., $D_{95}$ = 24), 90 percent less than about 15 microns, 50 percent less than about 4 microns, and 10 percent less than about 1 micron. W-610 particles have a surface area of 3 $m^2$/cc and a particle size of: 95 percent less than about 40 microns (i.e., $D_{95}$ = 40), 90 percent less than about 28 microns, 50 percent less than about 10 microns, and 10 percent less than about 1 micron.

In certain embodiments, the concentration agent particles comprise perlite particles. Perlite is a naturally-forming amorphous volcanic glass, containing about 70-75% silicon dioxide and 12-15% aluminum oxide, as well as smaller amounts of other metal oxides, including sodium oxide, potassium oxide, iron oxide, magnesium oxide, and calcium oxide. When perlite is expanded by heat it forms a lightweight aggregate. Examples of suitable perlite particles include the 4106 grade material, 4156 grade material and the 476 grade material, each commercially available from Dicaperl Minerals Corporation (Crawfordsville, IN).

In certain embodiments, the concentration agent particles comprise guanidine-functionalized metal silicate particles, guanidine-functionalized diatomaceous earth particles, or guanidine-functionalized perlite particles. A guanidine-functionalized particle can be made, for example, according to methods disclosed in commonly assigned International Application No. PCT/US2014/040861 (Kshirsagar et al.). A guanidine-functionalized metal silicate particle, guanidine-functionalized diatomaceous earth particle, or guanidine-functionalized perlite particle comprises at least one guanidine-containing ligand. The guanidine-containing ligand is formed by modifying the metal silicate, diatomaceous

earth, or perlite particle with a guanidine-containing silane having the structure shown in Formula 1:

$$X_{3-n}R^{a}_{n}Si–Y–G \qquad \text{Formula 1}$$

In Formula 1, Si is a silicon atom, and G denotes a guanidine group of the formula -NH-C(=NH)-$NH_2$. Y is a divalent group that is covalently bonded to the silicon atom at one end and to the G group at the other end. Each $R^a$ group, if any are present, is independently an alkyl, aralkyl, or aryl group, and is attached to the silicon atom. Each X is a leaving group covalently bonded to the silicon atom and is independently alkoxy or acyloxy, and *n* is 0, 1, or 2. A typical alkylene can be up to 20, up to 16, 12, 10, 8, 7, 6, 5, 4, or even up to 3 carbons, or even 2 carbons, inclusive of the terminal atoms of the divalent group. In some embodiments, Y is a divalent group comprising an alkylene of 3 to 6 carbons. In a preferred embodiment, Y is a divalent group having 3 carbons (i.e., propyl).

In Formula 1, each leaving group X is independently an alkoxy group of 1, 2, 3, 4, 5, 6, 7, 8, 9, or even up to 10 carbons, or is an acyloxy group of 2 carbons, or 3, 4, 5, 6, 7, 8, 9, or even up to 10 carbons, where the alkoxy or acyloxy group is bonded to the silicon through an oxygen atom.

In some embodiments, *n* is 0. When *n* is 0, no $R^a$ groups are present, and Formula 1 can be re-written more simply as shown in Formula 2 (where Si, G, Y, and X are as defined for Formula 1):

$$X_3Si–Y–G \qquad \text{Formula 2}$$

When the silane of Formula 1 (or Formula 2) reacts with an -OH group on the surface of a metal silicate or perlite particle, at least one X leaving group is replaced by a covalent bond of between the silicon atom and an oxygen atom on the surface of the metal silicate, diatomaceous earth, or perlite particle. An embodiment of a guanidine-functionalized metal silicate, guanidine-functionalized diatomaceous earth, or guanidine-functionalized perlite particle comprising a specific exemplary guanidine-containing ligand within the general type represented by Formula 1, wherein n = 0 (i.e., as in Formula 2), is shown in Formula 3 (the circle in Formula 3 represents a metal silicate or perlite particle):

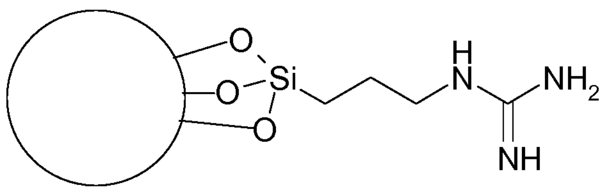

Formula 3

It will be understood that Formula 3 represents a specific embodiment wherein n is 3 and Y is a divalent group that is alkylene having 3 carbons. In each of Formulas 1 to 3, the ionization state of the guanidine group is omitted; however, it will be understood that in various environments such guanidine groups may be charged or uncharged (e.g., protonated or deprotonated), for example, according to the pH of a liquid medium in which the guanidine group is present.

The covalent bond(s) between the oxygen(s) of the ligand and the particle can be conveniently obtained, for example, by reacting a Si-bonded hydrolyzable group of the guanidine-containing precursor with a hydroxyl group of the particle. While the exemplary structure of Formula 3 shows three such bonded oxygen atoms (i.e., *n* = 3 in Formula 1), it will be appreciated that in various embodiments one, two or three such bonded oxygen atoms can be provided. If less than three such oxygen atoms are bonded to the silicon atom, other substituents (e.g., substituents that are not bonded to the particle, and which are not shown in Formula 1) may be present on the silicon atom. For example, the guanidine-containing ligand can include a polymeric structure involving formation of Si-O-Si (i.e., siloxane) groups, resulting from Si-O bonds being formed between two or more guanidine-containing ligand precursors. Without being bound by theory, it is thought that Si-O-Si groups may form in the presence of added water, or other aqueous solvents, or other agent that can hydrolyze bonds in Si-O-R groups, to give rise to more complex guanidine-containing ligand structures attached to particles.

A network of polymerized guanidine-containing ligands can form a coating on the surface of the metal silicate, diatomaceous earth, or perlite particle. In some embodiments it may be desirable to obtain the particle functionalized with polymerized guanidine-containing ligand (e.g., having at least one Si-O-Si group in the polymerized guanidine-containing ligand), as a means of increasing the loading of nitrogen-containing guanidine groups on the surface of the metal silicate, diatomaceous earth, or perlite particle. It is thought that in at least these types of polymerizations, a loading of nitrogen-containing guanidine groups on the surface of the metal silicate or perlite particle can attain levels of surface nitrogen content in a range from 1 to 10 atomic percent, as can be measured, for example, by X-ray photoelectron

spectroscopy.

Guanidine-functionalized particles of the present disclosure include metal silicate particles, diatomaceous earth particles, and perlite particles. Useful metal silicates include silicates of metals such as magnesium, calcium, zinc, aluminum, iron, titanium, and the like (preferably, magnesium and aluminum), and combinations thereof. Preferred are amorphous metal silicates in at least partially fused particulate form. In certain embodiments, more preferred are amorphous, spheroidized metal silicates; and even more preferably, amorphous, spheroidized magnesium silicate. In certain embodiments, more preferred are amorphous aluminum silicates. Metal silicates are known and can be chemically synthesized by known methods or obtained through the mining and processing of raw ores that are naturally-occurring. The metal silicate particle, such as a magnesium silicate particle, bears sufficient surface hydroxyl groups (typically, Si-OH groups) to enable a desired number of guanidine-containing ligands to be covalently attached thereto. Useful perlites include the 4106, 4156, and 476 grade materials from Dicaperl Minerals Corporation (Crawfordsville, IN). Useful diatomaceous earth particles can be obtained from natural sources, and are commercially available from Alfa Aesar (A Johnson Matthey Company, Ward Hill, MA).

The guanidine-functionalized metal silicate, diatomaceous earth, or perlite particles used in nonwoven articles of the present disclosure can be used in essentially any particulate form (preferably, a relatively dry or volatiles-free form) that is amenable to blending with fibers to form the nonwoven articles of the present disclosure. Preferably, the guanidine-functionalized particles are used in the form of a powder. Useful powders include those that comprise microparticles (preferably, microparticles having a particle size in the range of about 1 micrometer (more preferably, about 2 micrometers; even more preferably, about 3 micrometers; most preferably, about 4 micrometers) to about 100 micrometers (more preferably, about 50 micrometers; even more preferably, about 25 micrometers; most preferably, about 15 or 20 micrometers; where any lower limit can be paired with any upper limit of the range, as referenced above).

XPS is a technique that can provide information about the elemental and chemical (oxidation state and/or functional group) concentrations present on a solid surface. XPS typically provides an analysis of the outermost 3 to 10 nanometers (nm) of the specimen surface. XPS is sensitive to all elements in the periodic table except hydrogen and helium with detection limits for most species in the 0.1 to 1 atomic percent concentration range. In some cases, for example for CM-111 particles, a preferred surface composition assessment conditions for XPS can include a take-off angle of 90 degrees measured with respect to the sample surface with a solid angle of acceptance of ± 10 degrees. A person skilled in the art can select a suitable instrument setting for analysis of particles of the present disclosure.

In embodiments of the present disclosure, guanidine-functionalized metal silicate particles have a surface nitrogen content in a range from 1 atomic percent to 20 atomic percent, as measured by XPS. In some embodiments, the guanidine-functionalized metal silicate particles have a surface nitrogen content of at least 1 atomic percent, at least 2, at least 3, at least 4, or even at least 5 atomic percent, as measured by XPS. In some embodiments, the guanidine-functionalized metal silicate particles have a surface nitrogen content of up to 20 atomic percent, up to 15, up to 10, up to 9, up to 8, up to 7, or even up to 6 atomic percent, as measured by XPS. The surface nitrogen content of the guanidine-functionalized metal silicate particles, as measured by XPS, may be any combination of these lower and upper values, inclusive of the values thereof. A person skilled in the art would understand that in some embodiments it may be preferred to select higher or lower surface nitrogen content within these ranges, depending on the desired application. Suitable guanidine-functionalized perlite particles for use according to the present disclosure include those that comprise perlite and that have a surface composition having a surface nitrogen content of greater than 2 and less than or equal to about 12, as determined by XPS. Suitable guanidine-functionalized diatomaceous earth particles for use according to the present disclosure include those that comprise diatomaceous earth and have a surface composition having a surface nitrogen content of greater than 2 and less than or equal to about 12.

In some embodiments, particularly preferred are guanidine-functionalized magnesium silicate particles. Suitable guanidine-functionalized magnesium silicate particles for use according to the present disclosure include those that comprise an amorphous magnesium silicate and that have a surface composition having a metal atom to silicon atom ratio greater than 0.01 and less than or equal to about 0.5 (preferably, less than or equal to about 0.4; more preferably, less than or equal to about 0.3; most preferably, less than or equal to about 0.2), as determined by X-ray photoelectron spectroscopy ("XPS", also known as Electron Spectroscopy for Chemical Analysis ("ESCA")). In some embodiments, particularly preferred are guanidine-functionalized aluminum silicate particles. Suitable guanidine-functionalized aluminum silicate particles for use according to the present disclosure include those that comprise an amorphous aluminum silicate and that have a surface composition having a metal atom to silicon atom ratio greater than 6.7 and less than or equal to about 17.3, as determined by XPS (also known as ESCA). In some embodiments, particularly preferred are guanidine-functionalized perlite particles.

In an embodiment, the concentration agent particles comprise particles of diatomaceous earth, for instance particles of surface-modified diatomaceous earth. Diatomaceous earth (or kieselguhr) is a natural siliceous material produced from the remnants of diatoms, a class of ocean-dwelling microorganisms. Thus, it can be obtained from natural sources and is also commercially available (for example, from Alfa Aesar, A Johnson Matthey Company, Ward Hill, MA or Dicaperl Minerals Corporation, Crawfordsville, IN). Diatomaceous earth particles generally comprise small, open

networks of silica in the form of symmetrical cubes, cylinders, spheres, plates, rectangular boxes, and the like. The pore structures in these particles can generally be remarkably uniform.

Diatomaceous earth can be used in carrying out the process of the invention as the raw, mined material or as purified and optionally milled particles. Preferably, the diatomaceous earth is in the form of milled particles with sizes in the range of about 1 micrometer to about 50 micrometers in diameter (more preferably, about 3 micrometers to about 10 micrometers). The diatomaceous earth can optionally be heat treated prior to use to remove any vestiges of organic residues. If a heat treatment is used, it can be preferable that the heat treatment be at 500°C or lower, as higher temperatures can produce undesirably high levels of crystalline silica.

Surface-modified diatomaceous earth comprises diatomaceous earth bearing, on at least a portion of its surface, a surface treatment comprising titanium dioxide, ferric oxide, fine-nanoscale gold or platinum, or a combination thereof. Useful surface modifiers include fine-nanoscale gold; fine-nanoscale platinum; fine-nanoscale gold in combination with at least one metal oxide (preferably, titanium dioxide, ferric oxide, or a combination thereof); titanium dioxide; titanium dioxide in combination with at least one other (that is, other than titanium dioxide) metal oxide; and the like; and combinations thereof. Preferred surface modifiers include fine-nanoscale gold; fine-nanoscale platinum; fine-nanoscale gold in combination with at least ferric oxide or titanium dioxide; titanium dioxide; titanium dioxide in combination with at least ferric oxide; and combinations thereof. Surface-modified diatomaceous earth can be made, for example, according to methods disclosed in commonly assigned International Publication No. WO 2009/046191 (Kshirsagar et al.).

In an embodiment, the concentration agent particles comprise particles of gamma-FeO(OH) (also known as lepidocrocite). Specific examples of such concentration agent particles are disclosed in commonly assigned International Publication No. WO2009/046183 (Kshirsagar et al.). Gamma-FeO(OH) particles have been found to be surprisingly more effective than other iron-containing concentration agent particles in capturing gram-negative bacteria, which can be of great concern in regard to human bacterial infections.

Gamma-FeO(OH) is known and can be chemically synthesized by known methods (for example, by oxidation of ferrous hydroxide at neutral or slightly acidic pHs, as described for purposes of magnetic tape production in U.S. Pat. No. 4,729,846 (Matsui et al.)). Gamma-FeO(OH) is also commercially available (for example, from Alfa Aesar, A Johnson Matthey Company, Ward Hill, Mass., and from Sigma-Aldrich Corporation, St. Louis, Mo.).

In an embodiment, the concentration agent particles comprise particles of silica. A specific example of concentration agent silica particles is silicon dioxide microspheres having a mean diameter of about 2.5 microns that are commercially available from PolySciences, Inc., (Warrington, PA).

In an embodiment, the concentration agent particles comprise particles of metal carbonates. A specific example of concentration agent metal carbonate particles is calcium carbonate, such as calcium carbonate particles having a diameter range of 2.5-10 microns that are commercially available from Sigma-Aldrich, (St. Louis, MO).

In an embodiment, the concentration agent particles comprise particles of metal phosphates. A specific example of concentration agent metal phosphate particles is hydroxyapatite, such type-1 hydroxyapatite particles having particle sizes from 2-8 microns that are commercially available from Sigma-Aldrich, (St. Louis, MO).

In one specific method, the nonwoven article is prepared using a wet laying or "wetlaid" process. In this process, a dispersion is formed that contains (a) a plurality of fibers, (b) a plurality of concentration agent particles, (c) polymeric binder fibers, (d) and a dispersing liquid such as water, a water-miscible organic solvent, or a mixture thereof. The fibers and concentration agent particles can be dispersed together in the dispersing liquid. In some embodiments, the fibers (for example, hydrophobic fibers) have additives, surface treatments, or chemical groups that facilitate dispersion of the fibers in the dispersion liquid. For example, polyolefin-based fibers can have maleic anhydride or succinic anhydride functionality, or, during the melt-processing to prepare polyolefin-based fibers, a suitable surfactant can be added.

The wetlaid process additionally includes dewatering, followed by heating to finish the dewatering and optionally to bind some of the fibers together.

One or more adjuvants or additives can be used in preparing this type of nonwoven article. Useful adjuvants include process aids, materials that can enhance the overall performance of the resulting nonwoven article, and the like. When used, the amounts of such adjuvants can be present, for example, in an amount up 5 weight percent, up to 4 weight percent, up to 3 weight percent, up to 1 weight percent, or up to 0.5 weight percent based on a total dry weight of the nonwoven article (for example, fibers and concentration agent particles). The total amount of adjuvants is typically selected to be as low as possible so as to maximize the amount of concentration agent particles that can be included in the nonwoven article.

In one more specific wetlaid process, the fibers (for example, chopped fibers) can be blended in a container in the presence of the dispersing liquid (for example, water, a water-miscible organic solvent such as an alcohol, or a mixture thereof) to form a slurry. After formation of the slurry, the concentration agent particles and an optional precipitation agent (for example, a pH adjusting agent such as alum) can be added to the slurry.

When the wetlaid process is carried out by using hand-sheet methods known in the art, the order of addition of the components (i.e., fibers and concentration agent particles) to the dispersion has not been found to significantly affect the ultimate performance of the concentration device. After formation, the dispersion mixture can be poured into

a mold, the bottom of which can be covered by a screen. The dispersing liquid can be allowed to drain from the mixture (in the form of a wet sheet) through the screen. After sufficient liquid has drained, the wet sheet generally can be removed from the mold and dried by pressing, heating, or a combination of the two. Generally pressures are in a range of about 300 to about 600 kPa. Temperatures in a range of 90°C to 200°C, in a range of 100°C to 175°C, in a range of 100°C to 150°C, or in a range of 90°C to 120°C can be used for drying the wet sheet. Drying often removes all or most of the dispersing liquid (for example, up to 85 weight percent, up to 90 weight percent, up to 95 weight percent, up to 98 weight percent, or up to 99 weight percent of the dispersing liquid based on the amount of dispersing liquid added to form the dispersion).

The resulting nonwoven article is a dry sheet having an average thickness of at least 0.1 millimeter, at least 0.2 millimeters, at least 0.5 millimeters, at least 0.8 millimeters, at least 1 millimeter, at least 2 millimeters, at least 4 millimeters, or at least 5 millimeters. The average thickness is often up to 20 millimeters, up to 15 millimeters, up to 12 millimeters, or up to 10 millimeters. Calendering can be used to provide additional pressing or fusing, if desired, of the dry sheet.

In the nonwoven article, the concentration agent particles can be entrapped in the fibrous porous matrix through either chemical interactions (for example, chemical bonding) or physical interactions (for example, adsorption or mechanical entrapment), depending upon the nature of the fibers that are utilized. The concentration agent particles are often preferably distributed essentially uniformly throughout the fibrous porous matrix within the nonwoven article, for instance including at each surface and throughout the depth of the fibrous porous matrix.

Generally the average pore size of the dry nonwoven article can be in a range of 0.1 to 10 micrometers, as measured by scanning electron microscopy (SEM). Void volumes in the range of 20 to 80 volume percent or in a range of 40 to 60 volume percent can be useful. The porosity of the dry nonwoven article can be modified (increased) by using fibers of larger diameter or stiffness in the fiber mixture. The basis weight of the nonwoven article (in the form of sheet material) can be in the range of about 100 to about 350 grams per square meter ($g/m^2$), preferably, in the range of about 200 to about 300 $g/m^2$, such as about 250 $g/m^2$.

In certain embodiments, contacting comprises passing the fluid sample through the nonwoven article using a sample delivery system. One suitable sample delivery system comprises a freestanding container comprising a first reservoir and a deformable self-supporting receptacle dimensioned to be received in the first reservoir of the freestanding container and comprising a second reservoir. The freestanding container is more rigid than the deformable self-supporting receptacle, and the freestanding container includes a base comprising an aperture formed therein, through which the deformable self-supporting receptacle can be accessed. Accordingly, fluid sample is passed through the nonwoven article by applying external pressure to the deformable self-supporting receptacle via the aperture in the freestanding container to pass the fluid sample through the nonwoven article. The sample delivery system is discussed in detail below.

In a second aspect, a use of a device for contacting a fluid sample with a nonwoven article in a method according to the first aspect is provided. The device includes a sample container, a filter holder, a nonwoven article, and a first adaptor configured to interface the filter holder with a receptacle. Referring to FIG. 7A, a perspective schematic of an exemplary filter holder 14 is shown. The filter holder 14 is configured to interface with a sample delivery system. Referring to FIG. 7B, a first adaptor 18 is configured to interface the filter holder 14 with a receptacle (not shown). In certain embodiments, the first adaptor 18 comprises a hollow shape (e.g., a hollow cylinder) having a ledge 19 on which the nonwoven article 16 is placed. Referring to FIG. 7C, a perspective schematic of the filter holder 14 is shown, including a first adaptor 18 disposed inside the filter holder 14 and a nonwoven article 16 disposed on the ledge 19 of the first adaptor 18. Referring to FIG. 7D, a perspective schematic of an exemplary sample preparation system 100 is shown. The sample preparation system 100 includes a container 102, a liner 104, a lid 106, and a collar 108. The lid 106 comprises a port 132 extending from the center of the lid 106. The sample preparation system is described in detail below with respect to FIG. 6. Referring now to FIG. 7E, a side view schematic of an exemplary device 10 is shown, including the sample preparation system 100 of FIG. 7D. The device 10 further comprises the filter holder 14, nonwoven article 16, and first adaptor 18 of FIG. 7C disposed on the port 132 of the lid 106. In this embodiment, the lid 106 further comprises a plurality of protrusions 116 that cooperate with the filter holder 14 to fix the filter holder 14 in place on the lid 106.

It is not necessary for the filter holder to be a separate piece, but rather it could be integral to the lid. For instance, the filter holder and lid could be molded as one piece with no need for a port. In such a case the filter holder could have a cap to prevent contamination that would need to be removed before filtration. The lid could then be removed and the nonwoven article could be pushed from the top of the lid, crumpling the nonwoven article during its removal.

In a third aspect, a use of another device for contacting a fluid sample with a nonwoven article in a method according to the first aspect is provided. The device includes a sample container, a filter holder configured to interface with a receptacle, and a nonwoven article. The nonwoven article for each of the second and third aspects includes a fibrous porous matrix and a plurality of concentration agent particles enmeshed in the fibrous porous matrix. Typically, the filter holder comprises an exterior ledge that is configured to interface with the receptacle. In certain embodiments, the filter holder comprises an interior ledge configured to hold the nonwoven article.

Referring to FIGS. 1A-1E, an exemplary device 10 according to the disclosure, for contacting a fluid sample

with a nonwoven article is illustrated. The device 10 comprises a sample container 12; a filter holder 14; and a nonwoven article 16 comprising a fibrous porous matrix 15 and a plurality of concentration agent particles 17 enmeshed in the fibrous porous matrix 15. FIG. 1D shows a bottom plan schematic of the filter holder 14, in which an exterior ledge 11 is illustrated. The exterior ledge 11 of the filter holder 14 is configured to interface with a receptacle (not shown). The filter holder 14 further comprises an interior ledge 13 configured to hold the nonwoven article 16. FIG. 1B provides a cross-sectional schematic view of the filter holder 14 comprising the nonwoven article 16 disposed adjacent to the interior ledge 13. Hence, the sample container 12 can be unattached from the filter holder 14 and the nonwoven article 16 readily removed from inside the filter holder 14, such as pushed into a receptacle. Having the filter holder 14 configured to interface with a receptacle, such as the exterior ledge 11, simplifies transferring the nonwoven article 16 from the filter holder 14 to a receptacle. FIG. 1E shows the assembled device, including the sample container 12 attached to the filter holder 14 (i.e., threaded onto the filter holder) and the nonwoven article 16 is disposed in the filter holder 14. One of skill in the art will appreciate that other methods of attachment (e.g., press-fit engagement, snap-fit engagement, clamping engagement, etc.) may be employed to assemble and disassemble two or more components of the device.

As discussed above, it is not necessary for the filter holder to be a separate piece, but rather it could be integral to the sample container. The sample container could then be removed and the nonwoven article could be pushed from the inside of the sample container, crumpling the nonwoven article during its removal.

Referring to FIGS. 2A-2E, another exemplary device 10 according to the disclosure, for contacting a fluid sample with a nonwoven article is illustrated. The device 10 comprises a sample container 12; a filter holder 14 configured to interface with a receptacle (not shown); a nonwoven article 16 comprising a fibrous porous matrix 15 and a plurality of concentration agent particles 17 enmeshed in the fibrous porous matrix 15; and a second adaptor 22. The filter holder 14 is configured as described above with respect to FIGS. 1A-1E. FIG. 2B shows the second adaptor 22. In certain embodiments, the second adaptor 22 is configured to couple the device to a source of vacuum. FIG. 2C illustrates the filter holder 14 attached to the second adaptor 22 (i.e., threaded onto the adaptor). FIG. 2D shows a side view schematic of the container 12 attached to the filter holder 14, which is in turn attached to the second adaptor 22. (The nonwoven article disposed inside the filter holder 14 is not shown.) Referring to FIG. 2E, the second adaptor 22 is inserted into a stopper 23 configured to attach the device 10 to a vacuum flask (not shown).

Referring to FIGS. 3A-3E, a further exemplary device 10 according to the disclosure, for contacting a fluid sample with a nonwoven article is illustrated. The device 10 comprises a sample container 12; a filter holder 14 configured to interface with a receptacle (not shown); a nonwoven article 16 comprising a fibrous porous matrix 15 and a plurality of concentration agent particles 17 enmeshed in the fibrous porous matrix 15; and a second adaptor 22. The filter holder 14 is configured as described above with respect to FIGS. 1A-1E. FIG. 3B shows the second adaptor 22. In certain embodiments, the second adaptor 22 is configured to couple the device to a source of vacuum through a side arm. FIG. 3C illustrates the filter holder 14 attached to the second adaptor 22 (i.e., threaded onto the adaptor). FIG. 3D shows a side view schematic of the container 12 attached to the filter holder 14, which is in turn attached to the second adaptor 22. (The first nonwoven article disposed inside the filter holder 14 is not shown.) Referring to FIG. 3E, the second adaptor 22 is attaches the device 10 to a fluid sample collection container 21. Hence, in an embodiment, a fluid sample is contacted with the nonwoven article by putting the fluid sample in the sample container, pulling a vacuum through the second adaptor, and collecting fluid sample that has passed through the nonwoven article in a collection container.

Referring to FIG. 4A, a side view schematic of the device 10 of FIG. 3D is shown, further comprising a rotary pump 24. In this embodiment, the rotary pump 24 is attached to the second adaptor 22; however, in alternate embodiments a rotary pump may instead be attached to the filter holder 14. Referring to FIG. 4B, a top view schematic of the device of FIG. 4A is shown, further comprising a power drill 25 attached to the rotary pump 24. The power drill 25 is one example of a convenient tool that may be employed to create a vacuum, particularly when using the device in the field and a typical laboratory sink vacuum flask is unavailable.

Referring to FIG. 5, a side view schematic of yet another device 10 according to the disclosure, for contacting a fluid sample with a nonwoven article is illustrated. The device 10 comprises a sample container 12; a filter holder 14 configured to interface with a receptacle (not shown); a nonwoven article (not shown); a second adaptor 22; and a syringe 28. The filter holder 14 is disposed between the syringe 28 and the sample container 12, and connected via a tube 29. In this embodiment, the filter holder 14 is attached to the syringe 28 by the second adaptor 22. Including the syringe 28 provides a convenient way to swiftly contact a fluid sample with a nonwoven article, by simply pulling the plunger 27 of the syringe 28 and forcing the fluid sample through the nonwoven article with negative pressure. Alternatively, the syringe 28 could be directly connected to the filter holder 14 without using the second adaptor 22.

The sample container 12 can be formed of a variety of materials including, but not limited to, polymeric materials, metals, ceramics, glasses, and combinations thereof. Examples of suitable polymeric materials include for instance and without limitation, polyolefins (e.g., polyethylene, polypropylene, combinations thereof, etc.), polycarbonate, acrylics, polystyrene, high density polyethylene (HDPE), polypropylene, other suitable polymeric materials capable of forming a freestanding and/or self-supporting container, or a combination thereof. The container 12 can be translucent (or even transparent), or opaque, and can be any suitable size, depending on the type, amount and size of source to be analyzed.

For example, in some embodiments, the container 12 can have a capacity of up to 50 mL, 100 mL, 250 mL, 300 mL, 500 mL, 750 mL, or even up to 1 L.

Nonlimiting examples of suitable receptacles, for instance receptacles containing at least one reagent, include the 3M CLEAN-TRACE Surface ATP Swab available from 3M Company (St. Paul, Minn.), the AQUASNAP ATP Water Test available from Hygiena (Camarillo, Calif.), the ACCUPOINT 2 ATP Sanitation Monitoring System available from Neogen Corporation (Lansing, Mich.), and the PRO-CLEAN Rapid Protein Residue Test available from Hygiena.

In an alternate embodiment, a device is provided in which the sample container comprises a syringe. Using a syringe as a sample container allows for a convenient way to swiftly contact a fluid sample with a nonwoven article, by simply pressing the plunger of the syringe and forcing the fluid sample through the nonwoven article with positive pressure.

In any embodiment of the device, a gasket is disposed on or under the nonwoven article to minimize the ability of a volume of fluid sample to pass around the nonwoven article instead of through the nonwoven article. Suitable gaskets include, for instance, rubber gaskets sized to fit the perimeter of the nonwoven article, such as made from high-temperature resilient silicone foam, such as 1/16$^{th}$ inch or 1/8$^{th}$ inch thick foam commercially available from McMaster-Carr (Elmhurst, IL) (e.g., part number 1059N366).

Advantageously, a relatively large volume of fluid sample is optionally passed through the nonwoven article to concentrate the target microorganism and/or target cellular analyte, the container comprising a volume of at least 50 milliliters, or at least 100 milliliters, or at least 150 milliliters, or at least 300 milliliters, or at least 500 milliliters, or up to 1 liter.

FIG. 6 illustrates a sample delivery system 100 according to one embodiment of the present disclosure. As shown in FIG. 6, the sample delivery system 100 includes a container 102, a liner 104, a lid 106, a collar 108, and a cover 109. In some embodiments, one or more of the components of the sample delivery system 100 are sterile or sterilizable by sterilization and disinfection procedures such as steam, gamma radiation, ethylene oxide, hydrogen peroxide, peracetic acid, hydro-alcoholic solutions, bleach, and combinations thereof. A system having similar features to that of the sample delivery system 100 is described in PCT Publication No. WO 98/32539, U.S. Pat. Nos. 6,536,687 and 6,588,681, PCT Publication No. 2004/060574, PCT Publication No. 2004/060575, US Publication No. 2004/0164182, PCT Publication No. 2004/094072, PCT Publication No. WO 2007/079143, PCT Publication No. WO 2007/079188, and PCT Publication No. WO2009/067498.

The container 102 is freestanding and/or self-supporting and includes a base 127 and a sidewall 129. The term "freestanding" is generally used to refer to an object that is capable of standing on its own without collapsing or distorting, and without being held by another object. The term "self-supporting" is generally used to refer to an object that does not collapse or deform under its own weight. For example, a bag is typically not "self-supporting" in that it does not maintain its shape, but rather collapses or distorts, under its own weight. A self-supporting object is not necessarily freestanding.

The container 102 can be formed of the same materials as the sample container described above, and be of any of the same sizes.

In some embodiments, as shown in FIG. 6, the sample delivery system 100 includes a liner 104, which is shaped and dimensioned to be received within the container 102. The liner 104 can be disposable (e.g., made for one-time use), to allow the container 102 to be reused without substantial risk of contamination and without extensive cleaning required between uses.

As shown in FIG. 6, the container 102 defines a first reservoir 120, and the liner 104 defines a second reservoir 122. The liner 104 is shaped and dimensioned to be received within the first reservoir 120 of the container 102. In some embodiments, a fluid sample 114 can be added to the first reservoir 120, the fluid sample 114 comprising a sample matrix 113 and at least one microorganism strain and/or cellular analyte 112. In some embodiments, as shown in FIG. 6, the fluid sample 114 is positioned within the second reservoir 122 and the liner 104 is positioned within the first reservoir 120. In some embodiments, the liner 104 is freestanding and/or self-supporting, either of which can allow fluid sample 114 to be loaded into the liner 104 prior to positioning the liner 104 within the container 102, without the liner 104 collapsing or distorting.

In certain embodiments, the liner 104 is self-supporting and/or freestanding while also being deformable. The term "deformable" is used to refer to a structure that can be altered from its original shape or state by pressure (e.g., positive or negative) or stress. In embodiments employing a deformable liner 104, pressure can be applied to the liner 104 to reduce its size from its original (i.e., unstressed) dimensions. Such pressure can be used to promote removal of the fluid sample 114 (or a filtrate thereof) from the liner 104. In such embodiments, the liner 104 can serve as a deformable self-supporting receptacle that can contain the fluid sample 114. In some embodiments, the deformable self-supporting receptacle is also freestanding.

In the embodiment shown in FIG. 6, the container 102 includes an aperture 124 formed in its base 127, through which a user can access the liner 104 to apply pressure to the liner 104 to cause it to deform. Such pressure can be applied directly by hand, or by an additional device, and could be a manual or automated process.

In some embodiments, the liner 104 includes a relatively rigid base 126 and a relatively thin and deformable sidewall 128, such that when pressure is applied to the base 126 in a direction parallel to the longitudinal axis of the liner 104 (e.g., via the aperture 124 in the container 102), the liner 104 deforms in the longitudinal direction (e.g., by

virtue of the sidewall 128 collapsing rather than the base 126). Alternatively, or in addition, the base 126 can be thicker than the sidewall 128.

In certain embodiments, the lid 106 is removably coupled to the liner 104, and the collar 108 is employed to further secure the lid 106 to the container 102. For example, in FIG. 6, the container 102 includes threads 131 at the upper end of the outer surface of the sidewall 129, which are shaped and dimensioned for the collar 108 (having internal threads 133 capable of engaging with the threads 131 on the container 102) to be screwed onto the upper end of the container 102. As an alternative to using the collar 108 for securing the lid 106 to the container 102, other coupling means can be employed including clamping and/or any of the other coupling means. A variety of coupling means can be employed either between the lid 106 and the liner 104, the lid 106 and the container 102, and/or the collar 108 and the container 102 to allow the respective components to be removably coupled to one another, including, but not limited to, gravity (e.g., one component can be set atop another component, or a mating portion thereof), screw threads, press-fit engagement, snap-fit engagement, magnets, adhesives, heat sealing, other suitable removable coupling means, and combinations thereof.

In some embodiments, the sample delivery system 100 need not be reopened after the fluid sample 114 is added, such that the container 102, the liner 104, the lid 106 and the collar 108 need not be removably coupled to one another, but rather can be permanently or semi-permanently coupled to one another.

As shown in FIG. 6, the lid 106 further includes a port 132, which can be coupled to a filter 134, a cylindrical portion 136 that is dimensioned to be received within the liner 104, and a generally conical (e.g., frusto-conical) portion 138 that extends from the cylindrical portion 136 to the port 132. At the junction between the cylindrical portion 136 and the conical portion 138, the lid 106 further includes a lip 140 that extends radially outwardly from the cylindrical portion 136 and the conical portion 138.

In some embodiments, the filter 134 is coupled directly to the lid 106. In some embodiments, as shown in FIG. 6, the filter 134 can be supported by a frame 135 and coupled to the lid 106 via the frame 135. The frame 135 can form a portion of the filter 134, the frame 135 can be a part of the lid 106, or the frame 135 can be a separate element that is coupled to both the filter 134 and the lid 106. The frame 135 can be formed of a variety of materials, including, but not limited to, a variety of polymers, metals, ceramics, glasses, and combinations thereof.

As shown in FIG. 6, the collar 108 includes an inwardly-projecting lip 156, such that when the collar 108 is coupled to the container 102, the lip 156 of the collar 108 presses the lip 140 of the lid 106 into contact with the lip 146 of the liner 104, which is pressed into contact with the upper surface 146 of the container 102 (e.g., to form a higher integrity seal). The above-described means for assembling the sample delivery system 100 and for forming a seal between the components of the sample delivery system 100 are described and illustrated by way of example only. One of ordinary skill in the art will understand, however, that a variety of other mechanisms could be employed to assemble the components of the sample delivery system 100 and to form a seal (e.g., a liquid-tight seal, a hermetic seal, or a combination thereof), such that the sample delivery system 100 is inhibited from leaking under normal operating conditions.

The lid 106 can be formed of a variety of materials, including the materials listed above with respect to the sample container 12. The lid 106 can be translucent (or even transparent), or opaque, depending on the application of use.

The collar 108 can be formed of a variety of materials, including, but not limited to a variety of polymeric materials, metal materials, and combinations thereof. For example, the collar 108 can be formed of a molded plastic component, or a machined metal (such as aluminum) component. In some embodiments, the collar 108 is formed of a molded plastic component comprising glass fiber reinforced polypropylene.

As shown in FIG. 6, the port 132 of the lid 106 is generally cylindrical and tubular in shape, such that the port 132 defines a portion 152 of an inner surface of the lid 106 and an opening 154 in the lid 106. The lid 106 is hollow and is in fluid communication with the second reservoir 122 when the sample delivery system 100 is assembled. The port 132 does not need to be cylindrical and can instead take on any shape necessary for a given application. In the embodiment illustrated in FIG. 6, the filter 134 is coupled to the port 132 (i.e., via the frame 135) such that the filter 134 is in fluid communication with the lid opening 154, as well as the second reservoir 122.

In the embodiment shown in FIG. 6, the cover 109 is shaped and dimensioned to receive at least a portion of the port 132. As a result, the cover 109 can be coupled to the port 132 of the lid 106 to close the opening 154 in the lid 106 and to seal (e.g., hermetically seal) the sample delivery system 100 from the environment. The cover 109 can be coupled to the lid 106 using any suitable coupling means. The cover 109 can be integrally formed with the lid 106 (e.g., a flip-top snap-on cover), or the cover 109 can be separate from the lid 106 (e.g., a screw-on cover). The cover 109 can be formed of a variety of materials, including the materials listed above with respect to the container 102 or the collar 108.

The filter 134 can be of any geometrical shape to sufficiently filter the fluid sample 114. In some embodiments, the filter 134 is deformable and/or collapsible (i.e., such that the filter 134 folds under its own weight). In some embodiments, the filter 134 is rigid and retains its shape (i.e., does not fold under its own weight). The size and number of filters 134 used in a sample delivery system 100, and porosity thereof, may vary, depending on the desired analyte(s) and the insoluble matter in the sample matrix 113.

By way of example only, in some embodiments, the fluid sample 114 comprises a lysed bacterial cell culture, the desired analyte is one or more of DNA, RNA, a protein, ATP, or a metabolite, and the insoluble matter is cellular debris. In such embodiments, for example, the filter 134 can be selected or treated (e.g., derivatized with biomolecule-binding agents, such as antibodies) to retain and/or separate the cellular debris, while allowing the desired DNA, RNA, protein, ATP, and/or metabolite to pass through the filter 134 for subsequent capture by the nonwoven article.

The filter 134 can have a variety of pore sizes sufficient for retaining particles from the fluid sample 114, while allowing the desired analyte(s) (if present) in the fluid sample 114 to pass through the filter 134. In some embodiments, the filter 134 has an average pore or mesh size of at least 2 micrometers, in some embodiments, at least 5 micrometers, in some embodiments, at least 40 micrometers, in some embodiments, at least 80 micrometers, and in some embodiments, at least 120 micrometers. In some embodiments, the filter 134 has an average pore or mesh size of at most 2000 micrometers, in some embodiments, at most 1000 micrometers, in some embodiments, at most 500 micrometers, in some embodiments, at most 200 micrometers, in some embodiments, at most 50 micrometers, in some embodiments, at most 10 micrometers and in some embodiments, at most 1 micrometers (e.g., if it is desired to restrict bacteria from passing through the filter 134). Optionally, the filter 134 acts as a coarse filter.

The filter 134 can be formed from a variety of materials, including, but not limited to one or more of nylon, fluorinated polymers (e.g., polytetrafluoroethylene (PTFE)), cellulosics (e.g., modified celluloses such as cellulose esters (e.g., cellulose acetate) and nitrocelluloses), fiberglass, papers, and combinations thereof. In some embodiments, the filter 134 can be formed of a woven web, a nonwoven web, a molded structure, a foam, fabric, a fibrous web, and combinations thereof. The surface area of the filter 134 can be increased by pleating the filter 134, or by other similar techniques. The thickness of the filter 134 can be controlled by calendering or felting processes.

Following contact of a fluid sample with the nonwoven article, the device according to the disclosure is optionally at least partially disassembled and the first adaptor or the filter holder is interfaced with a receptacle. When interfaced with the receptacle, the nonwoven article is readily transferred from the device and placed in contact with at least one detection reagent. In certain embodiments, the nonwoven article can be moved using forceps, while in other embodiments the nonwoven article can be pushed through the hollow interior and/or off of the ledge of the first adaptor or filter holder, and into contact with one or more detection reagents.

Referring to FIGS 8A-8E, an exemplary method of detecting at least one bound microorganism strain or at least one bound cellular analyte is shown. FIG. 8A shows a filter holder 14 containing a first adaptor 18 and a nonwoven article 16 disposed on the first adaptor 18. A handle 32 of a detection device (not shown) is inserted into the first adaptor 18. FIG. 8B shows the handle 32 being removed from the filter holder 14, in which the first adaptor 18 and the nonwoven article 16 are also removed from the filter holder on an end 33 of the handle 32 of a detection device. FIG. 8C shows the end 33 of the handle 32 of a detection device 30 being inserted into the detection device 30. The first adaptor 18 fits inside a top opening of the detection device 30. FIG. 8D shows the handle 32 pushed down into the detection device 30. Due to the hollow configuration of the first adaptor 18, the end 33 of the handle 32 of the detection device 30 pushes the nonwoven article 16 down towards a lower end of the detection device 30 as the handle 32 is fully inserted into the detection device 30. In this embodiment, the detection device 30 comprises at least one reagent disposed in the lower end of the detection device 30. Microorganism strain(s) and target cellular analyte(s) bound to the nonwoven article 16 have an opportunity to react with the one or more reagents and generate a detectable signal. Referring to FIG. 8E, the detection device 30 is operationally coupled to a luminometer 50, which allows a generated luminescent signal to be detected.

The above-described means for transferring the non-woven article with bound microorganisms and/or cellular analytes is described and illustrated by way of example only. One of ordinary skill in the art will understand, however, that a variety of other mechanisms could be employed to transfer the non-woven article to a variety of detection devices depending on the configuration of the detection devices. In certain embodiments, for instance, a filter holder can be set on a detection device and the nonwoven article pushed through the filter holder into the detection device.

Referring to FIGS. 9A-9F, another exemplary device is illustrated. More particularly, FIG. 9A shows a syringe adaptor 92. The syringe adaptor may also be referred to as a second adaptor, for instance in a device for contacting a fluid sample with a nonwoven article including 1) a sample container, 2) a filter holder, 3) a nonwoven article, 4) a first adaptor configured to interface the filter holder with a receptacle, and 5) a second adaptor configured to attach the filter holder to the sample container. In the embodiment illustrated, the syringe (e.g., second) adaptor 92 advantageously includes a luer lock connector 95 for quick attachment and removal of a syringe from the rest of the device. The syringe adaptor 92 further includes a threaded portion 96 disposed on an exterior surface of the syringe adaptor 92, which imparts some flexibility to the exterior surface of the syringe adaptor 92 to assist in inserting at least some of the threaded portion 96 of the syringe adaptor 92 into the filter holder 94 of FIG. 9D (and being able to separate the syringe adaptor 92 from the filter holder 94 after use). Optionally, the filter holder 94 may have a complementary threaded portion located on an interior surface 91 so that the syringe adaptor 92 and the filter holder 94 could be specifically threaded together. Alternatively, other conventional methods of attaching two parts together may be employed, such as a twist and lock system, or compression fit parts, or the like. In addition, the syringe adaptor 92 includes at least one wing 97 (such as

the two wings 97 shown in FIG. 9A) which can optionally provide the dual functions of assisting in handling of the syringe adaptor 92 by a user and securing a ledge 98 on the filter holder 94. The wing 97 preferably includes a tab 101 configured to secure the ledge 98 of the filter holder 94. A different schematic perspective view of the syringe adaptor 92 is provided in FIG. 9B.

Referring to FIG. 9C, a filter holder 94 is illustrated. The filter holder 94 includes a first adaptor 18 disposed in the filter holder 94. Figure 9D is a schematic perspective view of a filter holder 94 containing a nonwoven article 16 secured to a first adaptor 18 with a gasket 93 (e.g., an O-ring).

Figure 9E is a schematic perspective view of the syringe adaptor 92 of Figure 9B coupled to the filter holder 94 of Figure 9D. Optionally, a luer lock connector (not shown) may also be provided at the aperture 99 present in the end of the filter holder 94 opposite the ledge 98, to attach a syringe and apply a vacuum to pull sample through the nonwoven article. Similarly, other sources of vacuum could be attached to the aperture 99, such as a pump or vacuum flask, and might be suitable for use to ensure all of a sample is passed through a nonwoven article in the device 90.

Figure 9F is a schematic side view of a device 90 including a syringe 28, the syringe adaptor 92 of Figure 9A, and the filter holder 94 of Figure 9D. In operation, a fluid sample is introduced into the syringe 28, then the syringe is attached to the rest of the device 90 using the luer lock connector 95 of the syringe adaptor 92. To capture microorganisms from the fluid sample, the plunger 27 of the syringe 28 is pressed, pushing the fluid sample to pass through the syringe adaptor 92 and the nonwoven article 16. The filter holder 94 may then be separated from the syringe adaptor 92 to provide access to the nonwoven article 16 for detection of bound microorganisms and/or cellular analytes.

Optionally, similar to the method demonstrated in FIGS. 8A-8E, the handle of a detection device may be inserted into the first adaptor 18. When the handle is subsequently removed from the filter holder 94, the first adaptor 18 and the nonwoven article 16 are also removed from the filter holder 94 on an end of the handle of a detection device. Next, the end of the handle of a detection device may be inserted into the detection device, in which the first adaptor 18 fits inside a top opening of the detection device. The handle is then pushed down into the detection device, and due to the hollow configuration of the first adaptor 18, the end of the handle of the detection device pushes the nonwoven article 16 down towards a lower end of the detection device as the handle is fully inserted into the detection device. Microorganism strain(s) and target cellular analyte(s) bound to the nonwoven article 16 thus have an opportunity to react with the one or more reagents and generate a detectable signal.

Referring to FIGS. 10A-10H, another exemplary device is illustrated, including 1) a sample container, 2) a filter holder, 3) a nonwoven article, and 4) a first adaptor configured to interface the filter holder with a receptacle. The filter holder 14a-14b includes two separable parts; a first filter holder portion 14a and a second filter holder portion 14b. FIG. 10A shows the first filter holder portion 14a. In the embodiment illustrated, the first filter holder portion 14a advantageously includes a luer lock connector 95 for quick attachment and removal of a container such as a syringe 28 from the rest of the device. As shown in FIG. 10B, the first filter holder portion 14a further includes an interior ledge 13 and a threaded portion 96 disposed on an interior surface of the first filter holder portion 14a, which allows connection of the first filter holder portion 14a with the second filter holder portion 14b of FIG. 10F (and allows separation of the first and second filter holder portions 14a and 14b from each other after use). Accordingly, the second filter holder portion 14b has a complementary threaded portion 96 located on an exterior surface of the second filter holder portion 14b. Alternatively, other conventional methods of attaching two parts together may be employed, such as a twist and lock system, or compression fit parts, or the like.

Referring to FIG. 10C, a first adaptor 18 is shown. In the illustrated embodiment, the first adaptor 18 is shaped to have a flat major surface 155 and an angled major surface 157 opposite the flat major surface 155, and to define an aperture that extends between the flat major surface 155 and the angled major surface 157. The first adaptor 18 is configured to be disposed in the first filter holder portion 14a (on the interior ledge 13), as shown in FIG. 10D, with the flat major surface 155 in contact with the interior ledge 13. Figure 10E is a schematic perspective view of a first filter holder portion 14a containing the first adaptor 18, a nonwoven article 16 disposed on the first adaptor 18, and a gasket 93 disposed on the nonwoven article 16. The gasket 93 is generally composed of a resilient material to assist in preventing a fluid from going around the edge of the nonwoven article 16 but instead through the nonwoven article 16. Depending on the dimensions of the interior of the first filter holder portion 14a and the second filter holder portion 14b, the first adaptor 18 may have the form of a short tube (similar to the tube shape of the first adaptor 18 shown in FIG. 7B).

As noted above, Figure 10F shows the second filter holder portion 14b. In the illustrated embodiment, the second filter holder portion 14b includes a fluid-permeable support 160 (which assists in securing the nonwoven article 16 in place in the filter holder 14a-14b), a threaded portion 96, and an outlet 158 that allows fluid to pass out of the filter holder 14a-14b. In alternate embodiments, no support 160 is provided; rather, the gasket 93 and first adaptor 18 cooperate to provide sufficient support to the nonwoven article 16. Optionally, a luer lock connector (not shown) may also be provided at the outlet 158 present at the end of the second filter holder portion 14b, to attach a syringe and apply a vacuum to pull sample through the nonwoven article 16. Similarly, other sources of vacuum could be attached to the outlet 158, such as a pump or vacuum flask, and might be suitable for use to ensure all of a sample is passed through a nonwoven article in the device 200.

Referring now to FIG. 10G, an exploded side view of the device 200 is provided. The device 200 includes the syringe 28 (e.g., the sample container), the first filter holder portion 14a, the first adaptor 18, the nonwoven article 16, the gasket 93, and the second filter holder portion 14b. Similarly, FIG. 10H provides a side view of the assembled device 200, including 1) a sample container (i.e., the syringe 28), 2) a filter holder (comprising a first filter holder portion 14a and a second filter holder portion 14b), 3) a nonwoven article 16, 4) a gasket 93, and 5) a first adaptor 18 configured to interface the filter holder 14a-14b with a receptacle. The filter holder 14a-14b contains the first adaptor 18 and the nonwoven article 16.

In operation, a fluid sample is introduced into the syringe 28, then the syringe is attached to the rest of the device 200 using the luer lock connector 95 of the filter holder 14a-14b. To capture microorganisms from the fluid sample, the plunger 27 of the syringe 28 is pressed, pushing the fluid sample to pass through the first filter holder portion 14a, the first adaptor 18, the nonwoven article 16, and the second filter holder portion 14b. The filter holder 14a-14b may then be separated into its first and second filter holder portions 14a and 14b to provide access to the nonwoven article 16 for detection of bound microorganisms and/or cellular analytes.

Referring now to FIGS. 11A-11C, similar to the method demonstrated in FIGS. 8A-8E, the handle 32 of a detection device 30 may be inserted into the first adaptor 18. When the handle 32 is subsequently removed from the first filter holder portion 14a, the first adaptor 18 and the nonwoven article 16 are also removed from the first filter holder portion 14a on an end 33 of the handle 32 of a detection device 30. Next, the end 33 of the handle 32 of the detection device 30 may be inserted into the detection device 30, in which the first adaptor 18 fits inside a top opening of the detection device 30. The handle 32 is then pushed down into the detection device 30, and due to the hollow configuration of the first adaptor 18, the end 33 of the handle 32 of the detection device 30 pushes the nonwoven article 16 down towards a lower end of the detection device 30 as the handle 32 is fully inserted into the detection device 30. Microorganism strain(s) and target cellular analyte(s) bound to the nonwoven article 16 thus have an opportunity to react with the one or more reagents and generate a detectable signal.

In a fourth aspect, a use of a kit in a method according to the first aspect is provided. The kit includes a device for contacting a fluid sample with a nonwoven article, and a receptacle. The device includes a sample container, a filter holder, a nonwoven article, and a first adaptor. The first adaptor is configured to interface the filter holder with the receptacle. The nonwoven article includes a fibrous porous matrix and a plurality of concentration agent particles enmeshed in the fibrous porous matrix.

Often, a second adaptor is provided and configured to attach the filter holder to a vacuum source or to a collection container. In certain embodiments, the device further includes a rotary pump attached to the filter holder or to the second adaptor. The second adaptor and rotary pump are as described above with respect to the first aspect. In many embodiments of the kit, the receptacle contains at least one reagent, although in alternate embodiments, one or more reagents can be added to the receptacle at the time of use of the kit. Typically, the receptacle is configured to be operationally connected to a detection instrument, such as a luminometer.

In many embodiments, the kit further includes instructions for using the kit. Such instructions typically include method details in accordance with the first aspect above.

Various embodiments are provided that include a method, and a use of a device and a kit.

Embodiment 1 is a method of detecting microorganisms in a fluid sample. The method includes a) providing a nonwoven article, b) providing a fluid sample suspected of containing at least one microorganism strain or target cellular analyte, and c) contacting the fluid sample with the nonwoven article such that at least a portion of the at least one microorganism strain or target cellular analyte is bound to the nonwoven article, and washing the at least one microorganism strain- or target cellular analyte-bound non-woven article. The nonwoven article includes 1) a fibrous porous matrix and 2) a plurality of concentration agent particles enmeshed in the fibrous porous matrix, wherein the concentration agent particles bind microorganisms from a fluid sample with a binding efficiency of at least 60 percent. The method further includes d) placing the at least one microorganism strain- or target cellular analyte-bound nonwoven article in contact with at least one detection reagent, and

e) detecting the presence of the at least one bound microorganism strain or bound target cellular analyte.

Embodiment 2 is the method of embodiment 1, wherein the detecting includes a culture-based detection method, an imaging detection method, a fluorescence-based detection method, a colorimetric detection method, an immunological detection method, a genetic detection method, a bioluminescence-based detection method, or a combination thereof.

Embodiment 3 is the method of embodiment 1, wherein the detecting includes a bioluminescence-based detection method.

Embodiment 4 is the method of embodiment 1 or embodiment 2, wherein the reagent includes luciferase.

Embodiment 5 is the method of embodiment 4, wherein the reagent further includes luciferin.

Embodiment 6 is the method of embodiment 4, wherein the reagent further includes a lysis agent.

Embodiment 7 is the method of any of embodiments 1 to 6, wherein the contacting includes filtration of the fluid

sample through the nonwoven article.

Embodiment 8 is the method of any of embodiments 1 to 7, further including passing the fluid sample through a coarse filter prior to the contacting the fluid sample with the nonwoven article.

Embodiment 10 is the method of any of embodiments 1 to 8, wherein the placing includes placing the at least one microorganism strain- or target cellular analyte-bound nonwoven article in a receptacle including a material through which a detection signal can be detected. The receptacle contains the at least one reagent.

Embodiment 11 is the method of any of embodiments 1 to 10, wherein the placing includes placing the at least one microorganism strain- or target cellular analyte-bound nonwoven article in a receptacle configured to be operationally coupled to a luminometer. The receptacle contains the at least one reagent.

Embodiment 12 is the method of any of embodiments 1 to 11, wherein the contacting includes pushing the at least one microorganism strain- or target cellular analyte-bound nonwoven article into a receptacle containing the at least one reagent.

Embodiment 13 is the method of any of embodiments 1 to 12, wherein the target cellular analyte includes at least one nucleic acid, protein, or adenosine triphosphate (ATP).

Embodiment 14 is the method of any of embodiments 1 to 13, wherein the contacting includes passing the fluid sample at least twice through the nonwoven article.

Embodiment 15 is the method of any of embodiments 1 to 14, wherein the contacting includes passing the fluid sample through the nonwoven article at a pressure of 14.7 pounds per square inch (psi) (101.3 kilopascals (kPa)) or less.

Embodiment 16 is the method of any of embodiments 1 to 15, wherein the contacting includes passing the fluid sample through the nonwoven article at a pressure of 4.0 psi (27.6 kPa) or less.

Embodiment 17 is the method of any of embodiments 1 to 16, wherein the contacting includes passing the fluid sample through the nonwoven article at a pressure of 0.5 psi (3.4 kPa) or less.

Embodiment 18 is the method of any of embodiments 1 to 17, wherein the microorganism strain is selected from strains of bacteria, fungi, protozoans, viruses, bacterial endospores, and combinations thereof.

Embodiment 19 is the method of any of embodiments 1 to 18, wherein the target cellular analyte includes ATP.

Embodiment 20 is the method of any of embodiments 1 to 19, wherein the fibrous porous matrix is a nonwoven fibrous layer and the concentration agent particles are distributed throughout the nonwoven fibrous layer.

Embodiment 21 is the method of any of embodiments 1 to 20, wherein the concentration agent particles include amorphous metal silicates, guanidine-functionalized metal silicates, diatomaceous earth, surface-modified diatomaceous earth, guanidine-functionalized diatomaceous earth, gamma-FeO(OH), metal carbonates, metal phosphates, silica, perlite, guanidine-functionalized perlite, or a combination thereof.

Embodiment 22 is the method of any of embodiments 1 to 21, wherein the contacting includes passing the fluid sample through the nonwoven article using a sample delivery system. The system includes a freestanding container including a first reservoir and a deformable self-supporting receptacle dimensioned to be received in the first reservoir of the freestanding container and having a second reservoir, the freestanding container being more rigid than the deformable self-supporting receptacle. The freestanding container includes a base having an aperture formed therein through which the deformable self-supporting receptacle can be accessed. The passing includes applying external pressure to the deformable self-supporting receptacle via the aperture in the freestanding container to pass the fluid sample through the nonwoven article.

Embodiment 23 is the method of any of embodiments 1 to 22, wherein the nonwoven article includes a thickness of between 0.15 millimeters and 1 millimeter.

Embodiment 24 is the method of any of embodiments 1 to 23, wherein the nonwoven article includes a thickness of between 0.15 millimeters and 0.8 millimeters.

Embodiment 25 is the method of any of embodiments 1 to 24, wherein the polymeric fibers include a polyolefin, a polysulfone, a polyamide, or a combination thereof.

Embodiment 26 is the method of any of embodiments 1 to 25, wherein the polymeric fibers include bi-component fibers.

Embodiment 27 is the method of any of embodiments 1 to 26, wherein the polymeric fibers include a fibrillated polyolefin fiber.

Embodiment 28 is the method of any of embodiments 1 to 27, wherein the inorganic fibers include glass fibers, ceramic fibers, or a combination thereof.

Embodiment 29 is the method of any of embodiments 1 to 28, wherein the inorganic fibers include glass fibers.

Embodiment 30 is the method of any of embodiments 1 to 29, wherein the inorganic fibers and polymeric fibers include an average length of less than 50 millimeters.

Embodiment 31 is the method of any of embodiments 1 to 30, wherein the nonwoven article includes 5 to 60 weight percent concentration agent particles based on a total dried weight of the nonwoven article and 40 to 95 weight percent fibrous porous matrix based on the total dried weight of the nonwoven article.

Embodiment 32 is the method of any of embodiments 1 to 31, wherein the nonwoven article includes 20 to 50 weight percent concentration agent particles based on a total dried weight of the nonwoven article and 50 to 80 weight percent fibrous porous matrix based on the total dried weight of the nonwoven article.

Embodiment 33 is the method of any of embodiments 1 to 32, wherein the fibrous porous matrix is a nonwoven fibrous layer including uncrimped polymeric fibers.

Embodiment 34 is the method of embodiment 33, wherein the nonwoven fibrous layer includes polyolefin fibers and glass fibers.

Embodiment 35 is the method of any of embodiments 1 to 34, wherein the fibrous porous matrix is free of polyamide fibers.

Embodiment 36 is the method of any of embodiments 1 to 35, wherein the concentration agent particles include particles of amorphous metal silicates.

Embodiment 37 is the method of embodiment 36, wherein the concentration agent particles include particles of amorphous, spheroidized magnesium silicate.

Embodiment 38 is the method of embodiment 36 or embodiment 37, wherein the concentration agent particles include particles of amorphous, spherical aluminum silicate.

Embodiment 39 is the method of any of embodiments 36 to 38, wherein the concentration agent particles include particles of guanidine-functionalized metal silicates.

Embodiment 40 is the method of embodiment 39, wherein the concentration agent particles include particles of guanidine-functionalized magnesium silicate.

Embodiment 41 is the method of embodiment 39, wherein the concentration agent particles include particles of guanidine-functionalized aluminum silicate.

Embodiment 42 is the method of any of embodiments 1 to 41, wherein the concentration agent particles include particles of diatomaceous earth.

Embodiment 43 is the method of any of embodiments 1 to 42, wherein the concentration agent particles include particles of surface-modified diatomaceous earth, particles of guanidine-functionalized diatomaceous earth, or combinations thereof.

Embodiment 44 is the method of embodiment 43, wherein the surface-modified diatomaceous earth includes diatomaceous earth bearing, on at least a portion of its surface, a surface treatment including titanium dioxide, ferric oxide, fine-nanoscale gold or platinum, or a combination thereof.

Embodiment 45 is the method of any of embodiments 1 to 44, wherein the concentration agent particles include particles of gamma-FeO(OH).

Embodiment 46 is the method of any of embodiments 1 to 45, wherein the concentration agent particles include particles of perlite.

Embodiment 47 is the method of any of embodiments 1 to 46, wherein the concentration agent particles include particles of guanidine-functionalized perlite.

Embodiment 48 is the method of any of embodiments 1 to 47, wherein the concentration agent particles include particles of hydroxyapatite.

Embodiment 49 is the method of any of embodiments 1 to 48, wherein the particles are microparticles.

Embodiment 50 is a use of a device for contacting a fluid sample with a nonwoven article in a method according to any of embodiments 1 to 49. The device includes 1) a sample container, 2) a filter holder, 3) a nonwoven article, and 4) a first adaptor configured to interface the filter holder with a receptacle. The nonwoven article includes a) a fibrous porous matrix and b) a plurality of concentration agent particles enmeshed in the fibrous porous matrix.

Embodiment 51 is the device of embodiment 50, further including a second adaptor configured to attach the filter holder to a vacuum source, to a collection container, or to the sample container.

Embodiment 52 is the use of a device according to embodiment 50 or embodiment 51, further including a rotary pump attached to the filter holder or to the second adaptor.

Embodiment 53 is the use of a device according to any of embodiments 50 to 52, further including a gasket disposed on the nonwoven article.

Embodiment 54 is the use of a device according to embodiment 50 or embodiment 51, wherein the sample container includes a syringe.

Embodiment 55 is the use of a device according to embodiment 50, further including a syringe, the filter holder disposed between the syringe and the sample container.

Embodiment 56 is the use of a device according to embodiment 50, wherein the sample container includes a freestanding container including a first reservoir and a deformable self-supporting receptacle dimensioned to be received in the first reservoir of the freestanding container and having a second reservoir, the freestanding container being more rigid than the deformable self-supporting receptacle. The freestanding container includes a base having an aperture formed therein through which the deformable self-supporting receptacle can be accessed. The passing includes applying external pressure to the deformable self-supporting receptacle via the aperture in the freestanding

container to pass the fluid sample through the nonwoven article.

Embodiment 57 is the use of a device according to any of embodiments 50 to 56, further including the receptacle, the receptacle configured to be operationally coupled to a luminometer. The receptacle contains at least one reagent.

Embodiment 58 is the use of a device according to any of embodiments 50 to 57, wherein the container has a volume of at least 50 milliliters.

Embodiment 59 is the use of a device according to any of embodiments 50 to 58, wherein the container has a volume of at least 150 milliliters.

Embodiment 60 is the use of a device according to any of embodiments 50 to 59, wherein the container has a volume of at least 300 milliliters.

Embodiment 61 is the use of a device according to any of embodiments 50 to 60, wherein the container has a volume of up to 1 liter.

Embodiment 62 is the use of a device according to any of embodiments 50 to 61, wherein the fibrous porous matrix consists essentially of inorganic fibers and polymeric fibers.

Embodiment 63 is the use of a device according to embodiment 62, wherein the polymeric fibers include a polyolefin, a polysulfone, a polyamide, or a combination thereof.

Embodiment 64 is the use of a device according to embodiment 62 or embodiment 63, wherein the polymeric fibers include bi-component fibers.

Embodiment 65 is the use of a device according to any of embodiments 62 to 64, wherein the polymeric fibers include a fibrillated polyolefin fiber.

Embodiment 66 is the use of a device according to any of embodiments 62 to 65, wherein the inorganic fibers include glass fibers, ceramic fibers, or a combination thereof.

Embodiment 67 is the use of a device according to embodiment 66, wherein the inorganic fibers include glass fibers.

Embodiment 68 is the use of a device according to any of embodiments 62 to 67, wherein the inorganic fibers and polymeric fibers include an average length of less than 50 millimeters.

Embodiment 69 is the use of a device according to any of embodiments 50 to 68, wherein the nonwoven article includes 5 to 60 weight percent concentration agent particles based on a total dried weight of the nonwoven article and 40 to 95 weight percent fibrous porous matrix based on the total dried weight of the nonwoven article.

Embodiment 70 is the use of a device according to any of embodiments 50 to 69, wherein the nonwoven article includes 20 to 50 weight percent concentration agent particles based on a total dried weight of the nonwoven article and 50 to 80 weight percent fibrous porous matrix based on the total dried weight of the nonwoven article.

Embodiment 71 is the use of a device according to any of embodiments 50 to 70, wherein the fibrous porous matrix is a nonwoven fibrous layer including uncrimped polymeric fibers.

Embodiment 72 is the use of a device according to any of embodiments 50 to 71, wherein the fibrous porous matrix is a nonwoven fibrous layer and the concentration agent particles are distributed throughout the nonwoven fibrous layer.

Embodiment 73 is the use of a device according to embodiment 72, wherein the nonwoven fibrous layer includes polyolefin fibers and glass fibers.

Embodiment 74 is the use of a device according to any of embodiments 50 to 73, wherein the fibrous porous matrix is free of polyamide fibers.

Embodiment 75 is the use of a device according to any of embodiments 50 to 74, wherein the concentration agent particles include amorphous metal silicates, guanidine-functionalized metal silicates, diatomaceous earth, surface-modified diatomaceous earth, guanidine-functionalized diatomaceous earth, gamma-FeO(OH), metal carbonates, metal phosphates, silica, perlite, guanidine-functionalized perlite, or a combination thereof.

Embodiment 76 is the use of a device according to any of embodiments 50 to 75, wherein the concentration agent particles include particles of amorphous metal silicates.

Embodiment 77 is the use of a device according to embodiment 75 or embodiment 76, wherein the concentration agent particles include particles of amorphous, spheroidized magnesium silicate.

Embodiment 78 is the use of a device according to any of embodiments 75 to 77, wherein the concentration agent particles include particles of amorphous, spherical aluminum silicate.

Embodiment 79 is the use of a device according to any of embodiments 75 to 78, wherein the concentration agent particles include particles of guanidine-functionalized metal silicates.

Embodiment 80 is the use of a device according to embodiment 79, wherein the concentration agent particles include particles of guanidine-functionalized magnesium silicate.

Embodiment 81 is the use of a device according to embodiment 79, wherein the concentration agent particles include particles of guanidine-functionalized aluminum silicate.

Embodiment 82 is the use of a device according to any of embodiments 50 to 81, wherein the concentration agent particles include particles of diatomaceous earth.

Embodiment 83 is the use of a device according to any of embodiments 50 to 82, wherein the concentration agent

particles include particles of surface-modified diatomaceous earth, guanidine-functionalized diatomaceous earth, or a combination thereof.

Embodiment 84 is the use of a device according to embodiment 83, wherein the surface-modified diatomaceous earth includes diatomaceous earth bearing, on at least a portion of its surface, a surface treatment including titanium dioxide, ferric oxide, fine-nanoscale gold or platinum, or a combination thereof.

Embodiment 85 is the use of a device according to any of embodiments 50 to 84, wherein the concentration agent particles include particles of gamma-FeO(OH).

Embodiment 86 is the use of a device according to any of embodiments 50 to 85, wherein the concentration agent particles include particles of perlite.

Embodiment 87 is the use of a device according to any of embodiments 50 to 86, wherein the concentration agent particles include particles of guanidine-functionalized perlite.

Embodiment 88 is the use of a device according to any of embodiments 50 to 87, wherein the concentration agent particles include particles of hydroxyapatite.

Embodiment 89 is the use of a device according to any of embodiments 50 to 88, wherein the particles are microparticles.

Embodiment 90 is a use of a kit in a method according to any of embodiments 1 to 49 including a) a device for contacting a fluid sample with a nonwoven article and b) a receptacle. The device includes 1) a sample container; 2) a filter holder; 3) a nonwoven article; and 4) a first adaptor. The first adaptor configured to interface the filter holder with the receptacle. The nonwoven article includes a) a fibrous porous matrix and b) a plurality of concentration agent particles enmeshed in the fibrous porous matrix.

Embodiment 91 is the use of a kit according to embodiment 90, further including a second adaptor configured to attach the filter holder to a vacuum source or to a collection container.

Embodiment 92 is the use of a kit according to embodiment 90 or embodiment 91, further including a rotary pump attached to the filter holder or to the second adaptor.

Embodiment 93 is the use of a kit according to any of embodiments 90 to 92, wherein the receptacle contains at least one reagent.

Embodiment 94 is the use of a kit according to any of embodiments 90 to 93, wherein the receptacle is configured to be operationally coupled to a luminometer.

Embodiment 95 is the use of a kit according to any of embodiments 90 to 94, further including instructions for using the kit.

Embodiment 96 is use of a device for contacting a fluid sample with a nonwoven article in a method according to any of embodiments 1 to 49. The device includes 1) a sample container; 2) a filter holder configured to interface with a receptacle; and 3) a nonwoven article. The nonwoven article includes a) a fibrous porous matrix and b) a plurality of concentration agent particles enmeshed in the fibrous porous matrix.

Embodiment 97 is the use of a device according to embodiment 96, wherein the filter holder comprises an interior ledge configured to hold the nonwoven article.

Embodiment 98 is the use of a device according to embodiment 96 or embodiment 97, wherein the filter holder comprises an exterior ledge that is configured to interface with the receptacle.

Embodiment 99 is the use of a device according to any of embodiments 96 to 98, further including an adaptor configured to attach the filter holder to a vacuum source, to a collection container, or to the sample container.

Embodiment 100 is the use of a device according to embodiment 99, further including a rotary pump attached to the adaptor.

Embodiment 101 is the use of a device according to any of embodiments 96 to 98, further including a rotary pump attached to the filter holder.

Embodiment 102 is the use of a device according to any of embodiments 96 to 101, further including a gasket disposed on the nonwoven article.

Embodiment 103 is the use of a device according to embodiment 96, further including a syringe, the filter holder disposed between the syringe and the sample container.

Embodiment 104 is the use of a device according to any of embodiments 96 to 103, further including the receptacle, the receptacle configured to be operationally coupled to a luminometer. The receptacle contains at least one reagent.

Embodiment 105 is the use of a device according to any of embodiments 96 to 104, wherein the container has a volume of at least 50 milliliters.

Embodiment 106 is the use of a device according to any of embodiments 96 to 105, wherein the container has a volume of at least 150 milliliters.

Embodiment 107 is the use of a device according to any of embodiments 96 to 106, wherein the container has a volume of at least 300 milliliters.

Embodiment 108 is the use of a device according to any of embodiments 96 to 107, wherein the container has a volume of up to 1 liter.

Embodiment 109 is the use of a device according to any of embodiments 96 to 108, wherein the fibrous porous matrix consists essentially of inorganic fibers and polymeric fibers.

Embodiment 110 is the use of a device according to embodiment 109, wherein the polymeric fibers include a polyolefin, a polysulfone, a polyamide, or a combination thereof.

Embodiment 111 is the use of a device according to embodiment 109 or embodiment 110, wherein the polymeric fibers include bi-component fibers.

Embodiment 112 is the use of a device according to any of embodiments 108 to 111, wherein the polymeric fibers include a fibrillated polyolefin fiber.

Embodiment 113 is the use of a device according to any of embodiments 109 to 112, wherein the inorganic fibers include glass fibers, ceramic fibers, or a combination thereof.

Embodiment 114 is the use of a device according to embodiment 113, wherein the inorganic fibers include glass fibers.

Embodiment 115 is the use of a device according to any of embodiments 109 to 114, wherein the inorganic fibers and polymeric fibers include an average length of less than 50 millimeters.

Embodiment 116 is the use of a device according to any of embodiments 96 to 115, wherein the nonwoven article includes 5 to 60 weight percent concentration agent particles based on a total dried weight of the nonwoven article and 40 to 95 weight percent fibrous porous matrix based on the total dried weight of the nonwoven article.

Embodiment 117 is the use of a device according to any of embodiments 96 to 116, wherein the nonwoven article includes 20 to 50 weight percent concentration agent particles based on a total dried weight of the nonwoven article and 50 to 80 weight percent fibrous porous matrix based on the total dried weight of the nonwoven article.

Embodiment 118 is the use of a device according to any of embodiments 96 to 117, wherein the fibrous porous matrix is a nonwoven fibrous layer including uncrimped polymeric fibers.

Embodiment 119 is the use of a device according to any of embodiments 96 to 118, wherein the fibrous porous matrix is a nonwoven fibrous layer and the concentration agent particles are distributed throughout the nonwoven fibrous layer.

Embodiment 120 is the use of a device according to embodiment 119, wherein the nonwoven fibrous layer includes polyolefin fibers and glass fibers.

Embodiment 121 is the use of a device according to any of embodiments 96 to 120, wherein the fibrous porous matrix is free of polyamide fibers.

Embodiment 122 is the use of a device according to any of embodiments 96 to 121, wherein the concentration agent particles include amorphous metal silicates, guanidine-functionalized metal silicates, diatomaceous earth, surface-modified diatomaceous earth, guanidine-functionalized diatomaceous earth, gamma-FeO(OH), metal carbonates, metal phosphates, silica, perlite, guanidine-functionalized perlite, or a combination thereof.

Embodiment 123 is the use of a device according to any of embodiments 96 to 122, wherein the concentration agent particles include particles of amorphous metal silicates.

Embodiment 124 is the use of a device according to embodiment 122 or embodiment 123, wherein the concentration agent particles include particles of amorphous, spheroidized magnesium silicate.

Embodiment 125 is the use of a device according to any of embodiments 122 to 124, wherein the concentration agent particles include particles of amorphous, spherical aluminum silicate.

Embodiment 126 is the use of a device according to any of embodiments 122 to 125, wherein the concentration agent particles include particles of guanidine-functionalized metal silicates.

Embodiment 127 is the use of a device according to embodiment 126, wherein the concentration agent particles include particles of guanidine-functionalized magnesium silicate.

Embodiment 128 is the use of a device according to embodiment 126, wherein the concentration agent particles include particles of guanidine-functionalized aluminum silicate.

Embodiment 129 is the use of a device according to any of embodiments 96 to 128, wherein the concentration agent particles include particles of diatomaceous earth.

Embodiment 130 is the use of a device according to any of embodiments 96 to 129, wherein the concentration agent particles include particles of surface-modified diatomaceous earth, guanidine-functionalized diatomaceous earth, or combinations thereof.

Embodiment 131 is the use of a device according to embodiment 130, wherein the surface-modified diatomaceous earth includes diatomaceous earth bearing, on at least a portion of its surface, a surface treatment including titanium dioxide, ferric oxide, fine-nanoscale gold or platinum, or a combination thereof.

Embodiment 132 is the use of a device according to any of embodiments 96 to 131, wherein the concentration agent particles include particles of gamma-FeO(OH).

Embodiment 133 is the use of a device according to any of embodiments 96 to 132, wherein the concentration agent particles include particles of perlite.

Embodiment 134 is the use of a device according to any of embodiments 96 to 133, wherein the concentration agent particles include particles of guanidine-functionalized perlite.

Embodiment 135 is the use of a device according to any of embodiments 96 to 134, wherein the concentration agent particles include particles of hydroxyapatite.

Embodiment 136 is the use of a device according to any of embodiments 96 to 135, wherein the particles are microparticles.

Embodiment 137 is the use of a device according to embodiment 96 or embodiment 99, wherein the sample container includes a syringe.

Embodiment 138 is the use of a device according to any of embodiments 50 to 89 or 96 to 137, wherein the nonwoven article has a basis weight in the range of about 150 to about 350 grams per square meter (g/$m^2$).

Embodiment 139 is the method of any of embodiments 1 to 49, wherein the nonwoven article has a basis weight in the range of about 150 to about 350 grams per square meter (g/$m^2$).

Embodiment 140 is the use of a kit according to any of embodiments 90 to 95, wherein the nonwoven article has a basis weight in the range of about 150 to about 350 grams per square meter (g/$m^2$).

## EXAMPLES

Unless otherwise noted, all chemicals used in the examples can be obtained from Sigma-Aldrich Corp. (Saint Louis, MO). Unless otherwise specified, all microbiological supplies and reagents were purchased as standard products from either Sigma-Aldrich or VWR.

| Material | Vendor |
|---|---|
| Fiber 1 - SHORT STUFF E380F ~0.7 mm average length, 15 microns diameter polyethylene fibers | MiniFIBERS, Inc.; Johnson City, TN |
| Fiber 2 - 6 denier 2 inches long chopped nylon fibers | MiniFIBERS, Inc.; Johnson City, TN |
| Fiber 3 - 1 denier bi-component ethylene vinyl acetate/polypropylene fibers | MiniFIBERS, Inc.; Johnson City, TN |
| Fiber 4 - long glass fibers (MICRO-STRAND 106-475 Glass Fiberglas) Schuller Inc. | Johns Mansville; Denver, CO |
| Fiber 5 - 2 denier 5 mm bicomponent copolyester fibers made of polyester as the core and PET as the sheath | MiniFIBERS, Inc.; Johnson City, TN |
| Fiber 6 - typical length 4.3 mm (range 4.5-7.5 mm), specific gravity 1.17 acrylonitrile fibers | CFF Fibrillated Fiber 114-3, from Sterling Fibers, Inc.; Pace, FL |
| CM-111 - Amorphous spheroidized magnesium silicate: Cosmetic Microspheres (CM-111) | 3M Company; St. Paul, MN |
| G-CM-111 Guanylated CM-111 made according to the method of Example E1-D in PCT/US2014/040861 | 3M Company; St. Paul, MN |
| CLEAN-TRACE lysis reagent - reagent for bioluminescence assay | 3M Company; Bridgend, UK |
| CLEAN-TRACE luciferin-luciferase enzyme reagent - reagent for bioluminescence assay | 3M Company; Bridgend, UK |
| CLEAN-TRACE ATP Water Test | 3M Company; St. Paul, MN |
| DI Water - Deionized filtered 18 megaohm water from a Milli-Q Gradient System | Millipore; Waltham, MA |
| ATP-free Water - HYPURE Molecular biology grade water catalog # SH30538.02 | Thermo Fisher Scientific; Waltham, MA |
| Tryptic Soy Broth - DIFCO Tryptic Soy Broth, prepared at 3% according to the manufacturer's instructions | Becton Dickenson; Sparks, MD |
| *E. coli*/coliform count plate - 3M PETRIFILM *E coli*/Coliform Count Plates; | 3M Company; St. Paul, MN |
| Aerobic count plate - 3M PETRIFILM Aerobic Count Plates | 3M Company; St. Paul, MN |
| Endo Agar plate - Premade agar plate, Catalog # 254016 | Becton Dickenson; Sparks, MD |

(continued)

| Material | Vendor |
|---|---|
| 3M Flip-Top Dilution Bottle with Butterfield's Buffer (Catalog Number FTBFD9966) | 3M Company; St. Paul, MN |
| 3M CLEAN-TRACE Water Plus - Total ATP, Catalog # AQT200 | 3M Company; St. Paul MN |
| 3M CLEAN-TRACE Surface ATP, Catalog # UXL100 | 3M Company; St. Paul MN |

*Preparation of nonwoven fibrous porous matrices containing calcined magnesium silicate*

**Examples 1, 2 and 3:** Fiber premixes were prepared by mixing various amounts of Fiber 1, Fiber 2, Fiber 3, and Fiber 4 as shown in Table 1 below. The fibers were added to 3 liters of cold deionized water in a 4 L blender (available from VWR, Radnor, PA, under the trade designation "WARING COMMERCIAL HEAVY DUTY BLENDER, MODEL 37BL84") and blended at low speed for 30 seconds. The mixture was examined for uniform dispersion of the fibers without nits or clumps. The additive particles, CM-111, were added with an additional liter of deionized water and mixed at low speed for 15 seconds.

A felt was prepared using a pad maker apparatus (obtained from Williams Apparatus, Watertown, NY, under the trade designation "TAPPI") that had a box measuring about 30 centimeters (12 inches) square and 30 centimeters (~12 inches) high with a fine mesh screen at the bottom and a drain valve. On the screen ~ a 14 inch (36 cm) x 12 inch (30 cm) piece of a polyethylene spunbond (PET Lutradur 7240 obtained from Fiberweb, Cincinnati, OH) was laid as scrim on the screen. The box was filled with tap water up to a height of about 1 centimeter above the screen. Each fiber and particle mixture was poured into the box and the valve was opened immediately which created a vacuum that pulled the water out of the box.

The fibrous nonwoven felts were each transferred from the apparatus onto a 20 centimeter square sheet of blotter paper (96-pound white paper, obtained from Anchor Paper, St. Paul, MN). Each felt was sandwiched between 2 to 4 layers of blotter paper, to blot excess water. The pressed felt was then transferred onto a fresh sheet of blotter paper and placed in an oven (obtained from SPX Thermal Product Solutions, White Deer, PA, under the trade designation "BLUE M STABIL-THERM OVEN, MODEL OV-560A2") set at 110°C for about 3 hours to remove residual water and to form a nonwoven fibrous porous matrix. The resulting fibrous porous matrices of Examples 1 and 2 were approximately 0.8-1 millimeter thick. The fibrous porous matrix of Example 3 was approximately 0.8-0.9 millimeters thick.

**Table 1: Compositions of Examples 1-3**

| Materials (in grams) | Example 1 | Example 2 | Example 3 |
|---|---|---|---|
| Fiber 1 | 11.08 | 10.98 | 8.83 |
| Fiber 2 | 3.01 | 0 | 2.44 |
| Fiber 3 | 2.30 | 2.29 | 1.82 |
| Fiber 4 | 0 | 1.8 | 1.4 |
| CM-111 | 5.15 | 5.07 | 5.03 |
| Basis weight ($g/m^2$) | 196.66 | 203.44 | 197.74 |

*Preparation of nonwoven fibrous porous matrices containing guanylated magnesium silicate*

**Examples 4, 5, 6, 7 and 8:** Nonwoven fibrous porous matrices of Examples 4, 5, 6, 7, and 8 were made using the procedure described above. The formulations are shown in Table 2 below.

**Table 2 Compositions of Examples 4-8**

| Materials (in grams) | Example 4 | Example 5 | Example 6 | Example 7 | Example 8 |
|---|---|---|---|---|---|
| Fiber 1 | 11.05 | 11.03 | 11.08 | 11.05 | 11.01 |
| Fiber 2 | 0 | 0 | 3.02 | 3.03 | 3.01 |
| Fiber 3 | 2.26 | 2.25 | 2.27 | 2.25 | 2.26 |

(continued)

| Materials (in grams) | Example 4 | Example 5 | Example 6 | Example 7 | Example 8 |
|---|---|---|---|---|---|
| Fiber 4 | 0 | 2.25 | 1.76 | 0 | 1.76 |
| Guanylated CM-111 | 5.00 | 5.00 | 5.01 | 5.00 | 5.00 |
| Basis weight (g/m$^2$) | 166.30 | 217.07 | 238.32 | 199.02 | 229.82 |

The thickness of the matrix of Example 4 was approximately 0.8-0.9 mm, Example 5 was approximately 0.6-0.8 mm thick, and Examples 6-8 were each approximately 0.8-1.0 mm thick.

**Example 9:** Bacteria used in examples

The various bacteria used in the examples (see Table 3 below) were obtained from ATCC (Manassas, VA).

**Table 3. Bacteria used in examples**

| Bacteria | ATCC No. |
|---|---|
| *Escherichia coli* | 51813 |
| *Staphylococcus aureus* | 6538 |
| *Enterobacter aerogenes* | 29007 |
| *Pseudomonas aeruginosa* | 9027 |

Pure cultures of the bacterial strains were inoculated into Tryptic Soy Broth (TSB, Becton, Dickinson and Company, Franklin Lakes, NJ) and were grown overnight at 37°C. The cultures were diluted serially in Butterfield phosphate buffer (3M Company, St. Paul, MN) to obtain desired amount of colony forming units (cfu) per ml for spiking into water samples. *E. coli* and *E. aerogenes* were quantified by plating appropriate dilutions on 3M PETRIFILM *E. coli*/Coliform Count Plates (3M Company) according to manufacturer's instructions and incubated overnight at 37°C. *S. aureus* and *P. aeruginosa* were quantified by plating appropriate dilutions on 3M PETRIFILM aerobic count plates (3M Company) and incubated overnight at 37°C. The plates were read using 3M PETRIFILM Plate Reader (3M Company) and colony forming units (cfu) were determined.

**Example 10:** Bacterial capture efficiency of nonwoven fibrous porous matrices

100 ml of either neutralized tap water (neutralized with 0.1% sodium thiosulfate) or 18 mega ohms double deionized water was spiked with various bacteria to get approximately 100 cfu/ml (total of approximately 10,000 cfu in 100 ml). A 14 mm disk was cut from the various nonwoven fibrous porous matrices described above and the disk was placed in a SWINNEX 13 Filter Holder (Catalog number SX0001300, EMD Millipore, Billerica, MA). The filter holder was closed and attached to a 60 mL BD syringe with BD LUER-LOK tip (Product Number 309653, Becton, Dickinson and Company). The plunger from the syringe was removed prior to attaching the filter holder. The syringe was connected to a 12-place Waters Millipore SEP-PAK vacuum manifold (Waters Corporation, Milford, MA). A collection tube was placed in the sample rack to collect the filtrate from each of the syringes. The vacuum manifold was attached to Air Cadet Vacuum/Pressure Station (model No. 420-3901, Barnant Company, Barrington, IL) and the solution was filtered through the fibrous porous matrix materials at a vacuum pressure of about 15 inches of mercury. The filtration took less than a minute. For manual filtration, the samples were also filtered by pushing the syringe plunger through the barrel.

The filtrate from each of the filtrations was collected in sterile tubes and 1 ml of each was plated on PETRIFILM plates to enumerate the amount of bacteria left over in the solution. The plates were incubated overnight at 37°C and colonies growing on the plates were enumerated. The input sample was also plated prior to filtration to determine the amount of bacteria in the sample. The samples were plated on a minimum of three plates and experiments were repeated at least three times. The number of bacteria in the input solution and the number of bacteria left over in the filtrate was used to calculate the percent capture efficiency. The bacterial capture ranged from 32 to 95 percent depending on the material (see Tables 4, 5, 6 and 7 below). The nonwoven fibrous porous matrices without fiberglass (Fiber 4, Examples 1 and 4) ranged from 32 to 70%. The percent capture of the nonwoven fibrous porous matrices without nylon (Fiber 2, Examples 2 and 5) ranged from 90 to 95%. Similar results were seen with *E. aerogenes* and *P. aeruginosa* (see Tables 4 and 6).

The percent capture of bacteria in nonwoven fibrous porous matrices without Fiber 2 (nylon) indicated that it was not essential for bacterial capture. However, Fiber 4 (fiberglass) was more important as the percent capture dropped to 32 to 70% when Fiber 4 (Examples 1 and 4) was removed. This was the case with nonwoven fibrous porous matrices containing either type of particles (calcined magnesium silicate or guanylated magnesium silicate).

**Example 11:** Detection of bacteria in nonwoven fibrous porous matrices by ATP bioluminescence.

Each nonwoven fibrous porous matrix containing bound bacteria was removed from the SWINNEX filter holder and transferred aseptically to a 1.5 ml microfuge tube (Plastibrand microtubes, Brand GmbH & Co. KG, Wertheim, Germany). 200 microliters of CLEAN-TRACE lysis reagent containing bacterial lysis reagent was added to the tube and vortexed for 1 min and allowed to sit at room temperature for additional 1 minute. 250 microliters of CLEAN-TRACE luciferin-luciferase enzyme reagent was added to the tube and mixed. The tube was placed immediately into a bench-top luminometer (20/20n single tube luminometer, Turner Biosystems, Sunnyvale, CA) and measurement of RLUs was recorded. The luminescence measurements were obtained from the luminometer using spreadsheet interface PC software that was provided with the luminometer. 100 microliters of buffer containing approximately 10,000 cfu of bacteria was pipetted into a 1.5 ml microfuge tube (Plastibrand microtubes) and upon addition of lysis mix (200 microliters) and ATP reagent (250 microliters), luminescence was measured similarly. The relative light units obtained from approximately 10,000 cfu was used to calculate percent recovery of ATP in nonwoven fibrous porous matrices (see Tables 4 and 5 below). The ATP recovery varied from 30 to 68% with uncut material and the matrix without Fiber 2 (Examples 2 and 5) gave the most recovery (55 to 68%).

**Table 4. Bacterial capture efficiency and percent recovery of ATP in uncut nonwoven fibrous porous matrices containing calcined magnesium silicate**

| Matrix | EC_10,000 cfu total | | SA_10,000 cfu total | |
|---|---|---|---|---|
| | % Capture | % ATP Recovery | % Capture | % ATP Recovery |
| Example 1 | 60 | 32 | 32 | 30 |
| Example 2 | 94 | 62 | 92 | 60 |
| Example 3 | 77 | 48 | 85 | 45 |
| | EA_10,000 cfu total | | PA_10,000 cfu total | |
| | % Capture | % ATP Recovery | % Capture | %ATP Recovery |
| Example 1 | 48 | 35 | 38 | 30 |
| Example 2 | 95 | 55 | 90 | 58 |
| Example 3 | 65 | 35 | 62 | 30 |

**Table 5. Bacterial capture efficiency and percent recovery of ATP in uncut nonwoven fibrous porous matrices containing guanylated magnesium silicate**

| Matrix | EC_10.000 cfu total | | SA_10,000 cfu total | |
|---|---|---|---|---|
| | % Capture | % ATP Recovery | % Capture | % ATP Recovery |
| Example 4 | 60 | 56 | 70 | 55 |
| Example 5 | 93 | 65 | 95 | 68 |
| Example 6 | 85 | 46 | 91 | 49 |

In another experiment, the nonwoven fibrous porous matrix was aseptically cut into small pieces and then transferred to 1.5 ml microfuge tube (Plastibrand microtubes). 200 microliters of the lysis reagent was added to the tube and vortexed for 1 minute and allowed to sit at room temperature for additional 1 minute. 250 microliters of the luciferin-luciferase enzyme reagent was added to the tube and mixed. The luminescence was measured as described above. The percent recovery of ATP in nonwoven fibrous porous matrices is shown in Tables 6 and 7 below. The ATP recovery varied from 52 to 77% with uncut material and the wet-laid without Fiber 2 (Examples 2 and 5) gave the most recovery (65 to 77%).

**Table 6. Bacterial capture efficiency and percent recovery of ATP in cut fibrous porous matrices containing calcined magnesium silicate**

| Matrix | EC_10,000 cfu total | | SA_10,000 cfu total | |
|---|---|---|---|---|
| | % Capture | % ATP Recovery | % Capture | % ATP Recovery |
| Example 1 | 55 | 60 | 45 | 55 |
| Example 2 | 92 | 77 | 90 | 70 |
| Example 3 | 75 | 65 | 82 | 72 |
| | EA_10,000 cfu total | | PA_10,000 cfu total | |
| | % Capture | % ATP Recovery | % Capture | % ATP Recovery |
| Example 1 | 45 | 55 | 35 | 40 |
| Example 2 | 90 | 68 | 90 | 65 |
| Example 3 | 68 | 52 | 65 | 55 |

**Table 7. Bacterial capture efficiency and percent recovery of ATP in cut fibrous porous matrices containing guanylated magnesium silicate**

| Matrix | EC_10,000 cfu total | | SA_10,000 cfu total | |
|---|---|---|---|---|
| | % Capture | % ATP Recovery | % Capture | % ATP Recovery |
| Example 4 | 62 | 55 | 68 | 57 |
| Example 5 | 91 | 68 | 90 | 70 |
| Example 6 | 83 | 52 | 88 | 45 |

**Example 12:** Bacterial capture and detection of ATP with integrated devices

The container, filter holder and second adaptors were constructed from a CAD drawing using a UV curable epoxy composition (ACCURA 60 epoxy resin) in a stereolithography machine (SLA Model SLA-250/40 machine) according to the manufacturer's instructions. The devices were formed according to (FIGS. 2E and 3E) but did not include first adaptors. The epoxy composition and the machine were manufactured by 3D Systems Corporation; Rock Hill S.C.).

Each filtration device was formed as three separate parts. The filter holder holds a 12 mm disk and has threads to screw it to the container. The second adaptor has threads to connect it to the filter holder and the entire device assemblies are shown in FIGS. 2E and 3E. The second adaptor 22 in FIG. 2E has a long stem and was attached to a stopper to connect the entire device assembly to a vacuum flask or a manifold. The second adaptor 22 in FIG 3E has threads to connect it to a collection container and has a side arm to connect it to a vacuum source.

The filtration device according to FIG. 2E (not including a first adaptor) was assembled using parts made by SLA and a 12 mm nonwoven fibrous porous matrix disk (one of the matrices of Examples 1, 2, or 3) was placed at the bottom of the filter holder before connecting the filter holder to the second adaptor. The filter holder with the second adaptor was connected to the container and the entire device was connected to a vacuum flask. A 100 ml fluid sample containing approximately 10,000 cfu total (approximately 100 cfu/ml) of bacteria was added to the container and filtered using the vacuum station (Air Cadet Vacuum/Pressure Station, model No. 420-3901, Barnant Company). The filtrate was collected and plated to determine capture efficiency as described in Example 10. The assembly was disconnected from the vacuum flask, the filter holder was removed and the filter holder was interfaced to the 3M CLEAN-TRACE Water Plus - Total ATP detection device. The handle from the ATP detection device was removed and the filter holder was placed on top of the tube. The fibrous porous matrix disk (i.e., filter) was pushed down into the tube using the handle and then the filter holder was removed. The handle was returned to the detection device and the disk was pushed all the way down into the detection chamber. The device was shaken for about 2 minutes and then inserted into a 3M CLEAN-TRACE NG luminometer to determine the RLU. The results are shown in Table 8 below.

**Table 8. Bacterial capture efficiency and percent recovery of ATP in fibrous porous matrices containing calcined magnesium silicate using filtration device of FIG. 2E.**

| Matrix | EC_10,000 cfu total | | SA_10,000 cfu total | |
|---|---|---|---|---|
| | % Capture | % ATP Recovery | % Capture | % ATP Recovery |
| Example 1 | 55 | 35 | 45 | 28 |
| Example 2 | 88 | 60 | 90 | 55 |
| Example 3 | 82 | 45 | 80 | 40 |

The filtration device according to FIG. 3E (not including a first adaptor) was assembled and contained a 12 mm wet-laid disk (one of the matrices of Examples 1, 2, or 3). The assembled device was connected to a bottle and a 100 ml fluid sample containing approximately 10,000 cfu total (approximately 100 cfu/ml) of bacteria was added to the container and filtered using the vacuum station (Air Cadet Vacuum/Pressure Station, model No. 420-3901, Barnant Company). The filtrate was collected and plated to determine capture efficiency as described in Example 10. The assembly was disconnected from the vacuum flask, the filter holder was removed and the filter holder was interfaced to the 3M CLEAN-TRACE Water Plus - Total ATP detection device. The handle from the ATP detection device was removed and the filter holder was placed on top of the tube. The fibrous porous matrix disk (i.e., filter) was pushed down into the tube using the handle and then the filter holder was removed. The handle was returned to the detection device and the disk was pushed all the way down into the detection chamber. The device was shaken for about 2 minutes and then inserted into a 3M CLEAN-TRACE NG luminometer to determine the RLU. The results are shown in Table 9 below.

**Table 9. Bacterial capture efficiency and percent recovery of ATP in fibrous porous matrices containing calcined magnesium silicate using the filtration device of FIG. 3E.**

| | EC_10,000 cfu total | | SA_10,000 cfu total | |
|---|---|---|---|---|
| Matrix | % Capture | % ATP Recovery | % Capture | % ATP Recovery |
| Example 1 | 48 | 30 | 40 | 26 |
| Example 2 | 85 | 65 | 88 | 58 |
| Example 3 | 75 | 40 | 84 | 42 |

**Example 13:** Testing of fibrous porous matrices with various formulations for removal *of E. coli* from water samples by filtration

A streaked culture *of E coli* (ATCC 11229) on a TSA was incubated overnight at 37°C. From the plate an isolated colony was removed and inoculated into 5 ml of TSB using a standard microbiology loop and incubated in a shaking incubator (INNOVA 44 from New Brunswick Scientific) at 37°C for 20-22 hours. The overnight culture that contained ~ 2-3 x $10^9$ cfu/ml was serially diluted in Butterfield's Buffer to obtain an inoculum with approximately 1 x $10^6$ cfu/mL.

A test sample was prepared by inoculating 200 ml deionized of water (MilliQ Gradient system, Millipore, Ma) a 1:100 dilution of the $10^6$ cfu/mL inoculum resulting in water test sample containing approximately $10^4$ cfu/ml (~ 4 Log cfus/ml).

A disk 47 mm in diameter was die punched from each of the nonwoven fibrous porous matrices of Examples 4, 5, 7, and 8, and each placed into a sample holder which was a custom device fabricated from polycarbonate. The device had three parts and was cylindrically shaped measuring about 60 cm in diameter by about 45 cm high. The lower part contained a support screen for the filter disk and a sample outlet port. The top portion was enclosed except for the sample inlet port through PVC tubing connected to the Cole Parmer peristaltic pump (Model No. 7553-70) and was vented on the upstream side to allow for of purging air. O-ring seals were used to prevent leakage on both the upstream and downstream sides. Internal threads provided closure pressure. The 47 mm disk was placed on top of the support screen, an O ring was added on top and holder was closed.

The fibrous porous matrices were tested individually, without replicates. A pre-filtration sample was pumped through the sample holder containing the nonwoven matrix disk using a Cole Parmer peristaltic pump (Model No. 7553-70) using 1/8" wall thick PVC tubing (VWR catalog # 60985-522). The spiked water was pumped through the fibrous porous matrix at a flow rate of 12 ml/minute. Filtrates were collected in 250 ml sterile glass bottles. The first 100 ml filtrate was collected and discarded. The second 100 ml filtrate was collected for further processing. After each filtration test, the holder was disassembled to remove the fibrous porous matrix using sterile forceps. Between testing of the matrices the

filtration device was rinsed with filtered sterilized 500 ml deionized water.

A 10 ml volume of the second 100 ml filtrate was added to a 100 ml containing Butterfield's Buffer flip-top bottle to obtain a 1:10 dilution. The bottle was capped and mixed manually by shaking for 10 seconds. A 10 ml volume was removed and added to another flip-top bottle to obtain a 1:100. Similarly the filtrate was further diluted to 1:1000 and 1:10000. These 100 ml diluted filtrates were vacuum filtered through 0.45 micron filters. After each filtration, the vacuum apparatus was rinsed with filtered sterilized 500 ml deionized water and blotted dry with Kimwipes (Kimtech Science Delicate Task Wipers, Kimberly-Clark Professional, Roswell, GA).

The matrices were removed from the apparatus with sterile forceps plates and placed on Endo Agar plates, grid side up. The plates were incubated at 37°C for 18-20 hours. Colony counts were obtained from plates by manual counting. Pre-filtration samples were also diluted and filtered as the procedure above. The cfu/ml colony counts were converted to Log cfu/ml values. Log Reduction Values (LRV) were calculated based on counts obtained from the plated filtrate and pre-filtration samples by using the formula below.

LRV= (Log of cfus/ml in pre-filtration sample) - (Log of cfus/ml in filtrate sample) Filtration testing was done on 47 mm disks of the matrices of Examples 4, 5, 7, and 8. The results are shown in Table 10 below.

**Table 10. Removal of E. coli with fibrous porous matrices containing guanylated magnesium silicate**.

| Matrix | Log cfus in pre-filtration sample | LRV |
|---|---|---|
| Example 4 | 4.53 | 3.53 |
| Example 5 | 4.53 | 3.53 |
| Example 7 | 4.53 | 3.53 |
| Example 8 | 4.53 | 4.53 |

*Preparation of nonwoven fibrous porous matrices containing guanylated perlite or calcined magnesium silicate*

**Examples 14,15,16,17 and 18:** Nonwoven fibrous porous matrices of Examples 14, 15, 16, 17 and 18 were made using the procedure described in Examples 1, 2 and 3. The formulations are shown in Table 11 below. The thickness of the matrix of Example 14 was approximately 2.0 to 2.3 mm, Example 15 was approximately 1.2 to 1.5 mm, Example 16 was approximately 0.65 to 0.85 mm, Example 17 was approximately 0.6 to 0.8 mm, and Example 18 was approximately 0.6 to 0.8 mm thick.

**Table 11: Compositions of Examples 14-18**

| Materials (in grams) | Example 14 | Example 15 | Example 16 | Example 17 | Example 18 |
|---|---|---|---|---|---|
| Fiber 1 | 11.08 | 8.44 | 8.45 | 6.6 | 6.6 |
| Fiber 2 | 3.01 | 2.45 | 0 | 0 | 0 |
| Fiber 3 | 2.30 | 1.89 | 1.86 | 1.34 | 1.38 |
| Fiber 4 | 1.84 | 1.47 | 1.47 | 1.07 | 1.06 |
| CM-111 | 0 | 0 | 0 | 0 | 4.0 |
| Guanylated perlite | 5.0 | 4.09 | 4.0 | 3.99 | 0 |

*Preparation of nonwoven fibrous porous matrices containing guanylated perlite with autoclaved components*

**Examples 19, 20 and 21:** Nonwoven fibrous porous matrices of Examples 19, 20 and 21 were made using the procedure described in Examples 1, 2 and 3. The formulations are shown in Table 12 below. The components were autoclaved at 121°C for 30 minutes (AMSCO CENTURY Scientific model SV-120 pressure sterilizer, Steris Corporation, Mentor, OH) and then mixed with sterile water for preparation of matrix as described before. Fiber 3 (bi-component ethylene vinyl acetate/polypropylene fiber) lost its consistency during autoclaving and its addition gave a matrix which was very fragile. The thickness of the matrix of Example 19 was approximately 0.4 to 0.6 mm, Example 20 was approximately 0.4 to 0.6 mm, and Example 21 was approximately 0.8 to 1.0 mm thick.

**Table 12: Compositions of Examples 19-21**

| Materials (in grams) | Example 19 | Example 20 | Example 21 |
|---|---|---|---|
| Fiber 1 (autoclaved) | 8.44 | 8.46 | 8.46 |
| Fiber 2 (autoclaved) | 0 | 0 | 0 |
| Fiber 3 (autoclaved) | 1.87 | 1.89 | 0 |
| Fiber 3 | 0 | 0 | 1.87 |
| Fiber 4 (autoclaved) | 1.47 | 1.47 | 1.48 |
| Guanylated perlite (autoclaved) | 3.97 | 0 | 4.01 |
| Guanylated perlite | 0 | 4.0 | 0 |

**Example 22:** Bacterial capture efficiency of nonwoven fibrous porous matrices from Example 14 to Example 21

The capture efficiency of nonwoven fibrous porous matrices prepared in Examples 14 to 21 were tested as described in Example 10. The number of bacteria in the input solution and the number of bacteria left over in the filtrate was used to calculate the percent capture efficiency. The bacterial capture ranged from 88 to 99 percent depending on the material (see Table 13 below). Again, fibrous matrices without Fiber 2 (nylon) did not show any appreciable difference in percent capture. The matrices prepared with autoclaved materials showed similar recoveries as non-autoclaved material.

**Example 23:** Detection of bacteria in nonwoven fibrous porous matrices by ATP bioluminescence.

ATP bioluminescence from each nonwoven fibrous porous matrix (Examples 14 to 21) containing bound bacteria was tested as described in Example 11. The relative light units obtained from input bacteria (approximately 10,000 cfu) was used to calculate percent recovery of ATP in the nonwoven fibrous porous matrices (see Table 13 below). The ATP recovery varied from 38 to 82% and the matrices without Fiber 2 (Examples 16 to 21) gave the most recovery (60 to 82%).

**Table 13. Bacterial capture efficiency and percent recovery of ATP with nonwoven fibrous porous matrices**

| | EC_10,000 cfu total | | SA_10,000 cfu total | |
|---|---|---|---|---|
| Matrix | % Capture | % ATP Recovery | % Capture | % ATP Recovery |
| Example 14 | 99 | 45 | 98 | 38 |
| Example 15 | 96 | 48 | 92 | 42 |
| Example 16 | 94 | 72 | 95 | 68 |
| Example 17 | 96 | 82 | 95 | 73 |
| Example 18 | 99 | 65 | 92 | 58 |
| Example 19 | 99 | 75 | 93 | 62 |
| Example 20 | 97 | 68 | 90 | 60 |
| Example 21 | 91 | 65 | 88 | 65 |

**Example 24:** Bacterial capture and detection of ATP with paint preparation device

A 3M PPS (paint preparation system) containing cup, collar, disposable liner and lid was obtained from 3M Company. A filter adapter was constructed from a CAD drawing using a UV curable epoxy composition (ACCURA 60 epoxy resin) in a stereolithography machine (SLA Model SLA-250/40 machine) according to the manufacturer's instructions. The devices were formed according to FIGS. 7D and 7E. The epoxy composition and the machine were manufactured by 3D Systems Corporation).

The filter adapter holds a 14 mm disk and couples to the disposable lid. The filter adaptor has a built-in cylindrical cavity to hold a cylinder beneath the fibrous porous matrix. The PPS device with filter adaptor containing a fibrous porous matrix (of Example 14, 15, 16 or 17) was assembled according to FIGS. 7D and 7E. A 100 ml fluid sample containing approximately 10,000 cfu total (~100 cfu/ml) of bacteria was added to the liner in the cup, closed with the lid coupled

with the filter adaptor, and sealed with the collar. The device was inverted and the liner was pushed using a plunger from a syringe, to push the fluid sample through the fibrous porous matrix. The filtrate was collected and plated to determine capture efficiency as described in Example 10.

The filter adaptor was removed from PPS, the fibrous porous matrix was pushed into the cylinder using a 3M CLEAN-TRACE Surface ATP swab, and the cylinder with the fibrous matrix was lifted with the handle of the swab. The CLEAN-TRACE Surface ATP swab contains lysis reagent in the swab. The swab was introduced into a 3M CLEAN-TRACE Water Plus - Total ATP Detection Device (3M Company) and pushed into the detection chamber (Figs. 8A to 8D). The detection device containing the fibrous matrix was shaken for 1 to 2 min and then inserted into a 3M CLEAN-TRACE NG luminometer (3M Company) (FIG. 8E) for measuring bioluminescence.

The bacterial capture ranged from 92 to 99 percent and was similar to using a SWINNEX 13 Filter Holder (see Table 14 below). The ATP recovery varied from 32 to 75% and the matrices without Fiber 2 (Examples 16 and 17) gave the most recovery (65 to 75%).

**Table 14. Bacterial capture efficiency and percent recovery of ATP in fibrous porous matrices using the filtration device of FIG. 7D.**

| Matrix | EC_10,000 cfu total | | SA_10,000 cfu total | |
|---|---|---|---|---|
| | % Capture | % ATP Recovery | % Capture | % ATP Recovery |
| Example 14 | 99 | 32 | 98 | 33 |
| Example 15 | 96 | 45 | 95 | 38 |
| Example 16 | 92 | 70 | 93 | 65 |
| Example 17 | 95 | 75 | 92 | 68 |

**Examples 25-28 and Comparative Examples 1 and 2:** Bacterial capture and detection of ATP with device including a syringe adaptor

Produced water samples were obtained from an oil well in Canada. A device as illustrated in Figure 9F was assembled, in which 10 ml of a produced water sample D (i.e., Comparative Example 1) was loaded into a 10 cubic centimeter syringe and a 14-mm diameter disk of the nonwoven fibrous porous matrix of Example 1 was affixed to the first adaptor using an O-ring gasket. The 10 ml of produced water was filtered through the nonwoven fibrous porous matrix in a dropwise manner, which took about one minute. Advantageously, the turbid produced water sample passed from the small diameter of the luer lock portion of the syringe adaptor to the larger diameter of the threaded portion of the syringe holder and the filter holder without generating back pressure sufficient to require applying increasing pressure on the syringe plunger (rather than the constant pressure that was applied). The filtrate was discarded. To ensure that all of the produced water sample passed through the disk, a syringe was filled with 5 ml of air, connected to the syringe adaptor, then the air was pushed through the nonwoven fibrous porous matrix. Next, the syringe holder was removed from the filter holder by unthreading the two parts from each other, thereby providing access to the nonwoven fibrous porous matrix. An ATP Water Test was obtained and the foil seal on the test was punctured and kept on the side. The sample collection stick from the ATP Water Test was used to pick up the nonwoven fibrous porous matrix by making a few circular turns of the stick on the matrix. The sample stick having the nonwoven fibrous porous matrix stuck on its end was inserted into the pre-punctured test and analyzed according to the manufacturer's instructions on a NG luminometer. The resulting signal in Relative Light Units (RLUs) generated the 'Filter-no wash' Example 25 reading.

For the 'Filter-wash' test (i.e., Example 26), a 10 ml sample of the produced water sample D was filtered through a 14 mm disk of the nonwoven fibrous porous matrix of Example 1 as described above for Example 25. After pushing the 5 ml of air through the disk, a new syringe containing 5 ml of ATP free water was attached to the syringe holder. The filter was washed with the ATP free water and the filtrate was discarded. The filter was removed from the holder using the sample collection stick and analyzed, both as described as above for Example 25. The matrices were tested in duplicate.

Produced water sample D was tested using the device but without having any nonwoven fibrous porous matrix in the filter holder, using the ATP Water Test for generating Comparative Example 1 RLU readings. The improvement in ATP signal from captured bacteria over a CLEAN-TRACE test (without concentrating the bacteria in a nonwoven fibrous porous matrix) was calculated using the formula below. Data is shown in Table 15 below.

Fold increase in ATP signal over current test = (RLUs from post filtration nonwoven matrix disk/RLUs from the unfiltered sample tested using the CLEAN-TRACE ATP water test).

14 mm disks of the nonwoven fibrous porous matrix of Example 1 were tested with produced water sample E

(i.e., Comparative Example 2) as described above for Examples 25 and 26. Example 27 was the 'Filter-no wash' while Example 28 was the 'Filter-wash'. Data is shown in Table 15 below.

**Table 15. Bacterial capture efficiency and percent recovery of ATP in fibrous porous matrices (of Example 1) using the filtration device of FIG. 9F.**

| Example # | Average ATP signal in RLUs | Fold increase over current test |
|---|---|---|
| Comparative Example 1 | 202 (12%) | N/A |
| Example 25 | 1311 (24%) | 6.5 |
| Example 26 | 889(34%) | 4.4 |
| Comparative Example 2 | 4 (20%) | N/A |
| Example 27 | 50 (25%) | 12.5 |
| Example 28 | 15452 | 3863 |
| N=2, stdev less than 10% unless otherwise noted in parentheses. | | |

The variation in tests indicates matrix interference. The improvement in ATP signal after washing indicates the decrease in carryover of inhibitory substances, which allows the ATP assay to be used for accurate rapid monitoring of produced water samples. In the case where washing did not improve the signal indicated that the sample contained fewer sources of microbial ATP and more sources of innate ATP.

**Examples 29 and 30:** Making thin nonwoven fibrous porous matrices containing calcined magnesium silicate

Two fiber premixes were prepared by mixing various amounts of Fiber 1, Fiber 4, Fiber 5 and Fiber 6 as shown in Table 16 below.

For Example 29, the Fiber 6 was added to 3 liters of cold deionized water in a 4 L blender (available from VWR, Radnor, PA, under the trade designation "WARING COMMERCIAL HEAVY DUTY BLENDER, MODEL 37BL84") and blended at medium speed for 30 seconds. All other fibers were added and blended for additional 1 minute at low speed. The mixture was examined for uniform dispersion of the fibers without nits or clumps. The particulate additive, CM-111, was added with an additional liter of deionized water and mixed at low speed for 15 seconds.

A nonwoven fibrous porous felt was prepared using a pad maker apparatus (obtained from Williams Apparatus, Watertown, NY, under the trade designation "TAPPI") that had a box measuring about 30 centimeters (12 inches) square and 30 centimeters (12 inches) high with a fine mesh screen at the bottom and a drain valve. On the screen ~ a 14 inch (36 cm) x 12 inch (30 cm) piece of a polyethylene spunbond (PET Lutradur 7240 obtained from Fiberweb, Cincinnati, OH) was laid as scrim on the screen. The box was filled with tap water up to a height of about 1 centimeter above the screen. The fiber and particle additive mixture was poured into the box and the valve was opened immediately which created a vacuum that pulled the water out of the box.

The fibrous nonwoven felt was transferred from the apparatus onto a 20 centimeter square sheet of blotter paper (96-pound white paper, obtained from Anchor Paper, St. Paul, MN). The felt was sandwiched between 2 to 4 layers of blotter paper, to blot excess water. The pressed felt of Example 29 was then transferred onto a fresh sheet of blotter paper and placed in an oven (HERATHERM oven series OMS100 obtained from Thermo Fisher Scientific, Waltham, MA) set at 125°C for about 3 hours to remove residual water and to form a nonwoven fibrous porous matrix. The resulting fibrous porous matrix of Example 29 was approximately 0.8 to 0.90 millimeters thick.

For Example 30, the fiber mixture was prepared as described for Example 29, except as noted, using the amounts shown in Table 16 below. The pad maker apparatus was filled with additional 4 liters of cold deionized water. No spunbond scrim was used on the screen of the apparatus. The fiber and particle additive mixture was poured into the apparatus while opening the valve and draining the water. The fibrous nonwoven felt was removed from the screen by pressing a -14 inch (36 cm) x 12 inch (30 cm) piece of a polyethylene spunbond onto the wet felt and lifting the scrim. The resulting dried nonwoven fibrous porous matrix was approximately 0.8 to 0.90 millimeters thick.

**Table 16**

| Material (grams) | Example 29 | Example 30 |
|---|---|---|
| Fiber 1 | 6.02 | 6.02 |
| Fiber 4 | 1.07 | 1.06 |

(continued)

| Material (grams) | Example 29 | Example 30 |
|---|---|---|
| Fiber 5 | 1.30 | 1.32 |
| Fiber 6 | 1.05 | 2.05 |
| CM-111 | 4.03 | 4.08 |
| Basis Weights in g/$m^2$ | 126.05 | 122.17 |

*Testing of nonwoven fibrous porous matrices with metal silicate for bacterial capture of E. coli*

A single colony from a streak culture *of E. coli* (ATCC 11229, a Gram negative organism) was inoculated into 10 ml of TSB (Tryptic Soy Broth, 3% by weight from Difco) incubated overnight for about 20 hours at 37 degrees C. The resulting bacterial stock contained about 1 x $10^9$ cfus/ml. That stock was serially diluted in deionized water to make working stocks of 10 cfus/ml, 1 x $10^2$ cfus/ml, and 1 x $10^3$ cfus/ml.

Example 31: 14 mm disks of the nonwoven fibrous porous matrix of Example 29 were die punched and inserted into 13 mm filter holders (SWINNEX holders obtained from Millipore). 10 ml of 10 cfus/ml working stock was filtered through each disk using a 10 cc syringe. After filtration the disks were discarded. The filtrate was collected in sterile 15 ml polypropylene tubes. A one ml volume of the filtrate was plated on PAC plates.

**Example 32:** 14 mm disks of the nonwoven fibrous porous matrix of Example 29 were die punched and inserted into 13 mm filter holders (SWINNEX holders obtained from Millipore) and tested for *E. coli* capture using 10 ml of 1x $10^2$ cfus/ml working stock according to the same procedure as Example 31.

**Example 33:** 14 mm disks of the nonwoven fibrous porous matrix of Example 29 were die punched and inserted into 13 mm filter holders (SWINNEX holders obtained from Millipore) and tested for *E. coli* capture using 10 ml of 1x$10^3$ cfus/ml working stock according to the same procedure as Example 31.

**Examples 34 to 36:** 14 mm disks of the nonwoven fibrous porous matrix of Example 30 were tested according to the same procedures as Examples 31, 32, and 33, to generate Examples 34, 35 and 36, respectively.

The *E. coli* stock solutions were also plated on PAC. These were the "100% control" samples. Plate counts were measured per the manufacturer's instructions using a PETRIFILM Plate Reader. Capture efficiency was calculated using the formulas below. The results are shown in Table 17.

% Control= (CFUs in plated filtrates) x 100/CFUs in 100% Control
% Capture Efficiency = 100- % Control

**Table 17. Capture of *E. coli***

| Sample | Example 31 | Example 32 | Example 33 | Example 34 | Example 35 | Example 36 |
|---|---|---|---|---|---|---|
| % Capture Efficiency | 70.7 (48) | 53.4(11) | 67.1 (13) | 78.8 (26) | 71.3 | 82.8 |
| n=3, % std deviation < 10% unless indicated in parenthesis. The plated 100% control had total 205 cfus/ml (24% stdev), 1895 cfus and 18450 cfus. | | | | | | |

*Testing of nonwoven fibrous porous matrices with metal silicate for bacterial capture of S. aureus*

A single colony from a streak culture *of S. aureus* (ATCC 6538, a Gram positive organism) was inoculated into 10 ml of TSB (Tryptic Soy Broth, 3% by weight from Difco) incubated overnight for about 20 hours at 37 degrees C. The resulting bacterial stock contained about 1 x $10^9$ cfus/ml. That stock was serially diluted in deionized water to make working stocks of 10 cfus/ml, 1 x $10^2$ cfus/ml, and 1 x $10^3$ cfus/ml.

**Example 37:** 14 mm disks of the nonwoven fibrous porous matrix of Example 29 were die punched and inserted into 13 mm filter holders (SWINNEX holders obtained from Millipore). 10 ml of 10 cfus/ml working stock was filtered through each disk using a 10 cc syringe. After filtration the disks were discarded. The filtrate was collected in sterile 15 ml polypropylene tubes. A one ml volume of the filtrate was plated on PAC plates.

**Example 38:** 14 mm disks of the nonwoven fibrous porous matrix of Example 29 were die punched and inserted into 13 mm filter holders (Swinnex holders obtained from Millipore) and tested for *S. aureus* capture using 10 ml of 1x$10^2$ cfus/ml working stock according to the same procedure as Example 37.

**Example 39:** 14 mm disks of the nonwoven fibrous porous matrix of Example 29 were die punched and inserted

into 13 mm filter holders (Swinnex holders obtained from Millipore) and tested for *S. aureus* capture using 10 ml of $1x10^3$ cfus/ml working stock according to the same procedure as Example 37.

**Examples 40 to 42:** 14 mm disks of the nonwoven fibrous porous matrix of Example 30 were tested according to the same procedures as Examples 37, 38, and 39, to generate Examples 40, 41, and 42, respectively.

The *S. aureus* stock solution was also plated on PAC. These were the "100% control" samples. Plate counts were measured per the manufacturer's instructions using a PETRIFILM Plate Reader. Capture efficiency was calculated using the formulas below. The results are shown in Table 18.

% Control= (CFUs in plated filtrates) x 100/CFUs in 100% Control

% Capture Efficiency = 100- % Control

**Table 18. Capture of *S. aureus***

| Sample | Example 37 | Example 38 | Example 39 | Example 40 | Example 41 | Example 42 |
|---|---|---|---|---|---|---|
| % Capture Efficiency | 96.6 | 95.2 | 96.5 | 98.3 | 95.7 | 96.5 |
| n=3, % std deviation < 10 % unless indicated in parentheses. The plated 100% control had total 195 cfus/ml (40% stdev), 1380 cfus (12% stdev) and 15950 cfus. | | | | | | |

While the specification has described in detail certain exemplary embodiments, it will be appreciated that those skilled in the art, upon attaining an understanding of the foregoing, may readily conceive of alterations to, variations of, and equivalents to these embodiments. Various exemplary embodiments have been described. These and other embodiments are within the scope of the following claims.

## Claims

**1.** A method of detecting microorganisms in a fluid sample, the method comprising:

a) providing a nonwoven article comprising 1) a fibrous porous matrix and 2) a plurality of concentration agent particles enmeshed in the fibrous porous matrix, wherein the concentration agent particles bind microorganisms and/or target cellular analytes from a fluid sample with a binding efficiency of at least 60 percent; and wherein the target cellular analyte is a component of the microorganism ;
b) providing a fluid sample suspected of containing at least one microorganism strain or target cellular analyte;
c) contacting the fluid sample with the nonwoven article such that at least a portion of the at least one microorganism strain or target cellular analyte is bound to the nonwoven article, and washing the at least one microorganism strain- or target cellular analyte-bound nonwoven article;
d) placing the at least one microorganism strain- or target cellular analyte-bound nonwoven article in contact with at least one detection reagent; and
e) detecting the presence of the at least one bound microorganism strain or bound target cellular analyte.

**2.** The method of claim 1, wherein the reagent further comprises a lysis agent.

**3.** The method of claim 2, wherein the reagent comprises luciferase.

**4.** The method of any of claims 1 to 3, wherein the placing comprises placing the at least one microorganism strain- or target cellular analyte-bound nonwoven article in a receptacle comprising a material through which a detection signal can be detected, the receptacle containing the at least one reagent.

**5.** The method of any of claims 1 to 4, wherein the target cellular analyte comprises at least one nucleic acid, protein, or adenosine triphosphate (ATP).

**6.** The method of any of claims 1 to 5, wherein the contacting comprises passing the fluid sample through the nonwoven article at a pressure of 4.0 psi (27.6 kPa) or less.

**7.** The method of any of claims 1 to 6, wherein the nonwoven article comprises a thickness of between 0.15 millimeters and 2 millimeters.

8. Use of a device for contacting a fluid sample with a nonwoven article in a method according to any of the preceding claims, the device comprising: 1) a sample container; 2) a filter holder; 3) a nonwoven article comprising a) a fibrous porous matrix wherein the concentration agent particles bind microorganisms and/or target cellular analytes with a binding efficiency of at least 60 percent; and wherein the target cellular analyte is a component of the microorganism ; and b) a plurality of concentration agent particles enmeshed in the fibrous porous matrix; and 4) a first adaptor configured to interface the filter holder with a receptacle.

9. Use of a device according to claim 8, wherein the sample container comprises: a freestanding container comprising a first reservoir; a deformable self-supporting receptacle dimensioned to be received in the first reservoir of the freestanding container and comprising a second reservoir, the freestanding container being more rigid than the deformable self-supporting receptacle, the freestanding container including a base comprising an aperture formed therein through which the deformable self-supporting receptacle can be accessed.

10. Use of a device according to claim 8, further comprising a second adaptor configured to attach the filter holder to a vacuum source, to a collection container, or to the sample container.

11. Use of a device according to any of claims 8 to 10, wherein the fibrous porous matrix is a nonwoven fibrous layer comprising uncrimped polymeric fibers.

12. Use of a device according to any of claims 8 to 11, wherein the fibrous porous matrix is free of polyamide fibers.

13. Use of a device according to any of claims 8 to 12, wherein the concentration agent particles comprise amorphous metal silicates, guanidine-functionalized metal silicates, diatomaceous earth, surface-modified diatomaceous earth, guanidine-functionalized diatomaceous earth, gamma-FeO(OH), metal carbonates, metal phosphates, silica, perlite, guanidine-functionalized perlite, or a combination thereof.

14. Use of a device for contacting a fluid sample with a nonwoven article in a method according to any of the preceding claims, the device comprising: 1) a sample container; 2) a filter holder configured to interface with a receptacle; and 3) a nonwoven article comprising a) a fibrous porous matrix and b) a plurality of concentration agent particles enmeshed in the fibrous porous matrix, wherein the concentration agent particles bind microorganisms and/or target cellular analytes with a binding efficiency of at least 60 percent; and wherein the target cellular analyte is a component of the microorganism.

15. Use of a kit in a method according to any of the preceding claims, the kit comprising:

a. a device for contacting a fluid sample with a nonwoven article comprising: 1) a sample container; 2) a filter holder; 3) a nonwoven article comprising i) a fibrous porous matrix and ii) a plurality of concentration agent particles enmeshed in the fibrous porous matrix; wherein the concentration agent particles bind microorganisms and/or target cellular analytes with a binding efficiency of at least 60 percent; and wherein the target cellular analyte is a component of the microorganism ; and 4) a first adaptor; and
b. a receptacle, the first adaptor configured to interface the filter holder with the receptacle.

## Patentansprüche

1. Ein Verfahren zum Nachweisen von Mikroorganismen in einer Fluidprobe, wobei das Verfahren Folgendes umfasst:

a) Bereitstellen eines Vliesartikels, umfassend 1) eine faserige poröse Matrix und 2) eine Mehrzahl von Konzentrationsmittelteilchen, die in die faserige poröse Matrix eingebunden sind, wobei die Konzentrationsmittelteilchen Mikroorganismen und/oder Zielzellanalyten aus einer Fluidprobe mit einer Bindungseffizienz von mindestens 60 Prozent binden; und wobei der Zielzellanalyt ein Bestandteil des Mikroorganismus ist;
b) Bereitstellen einer Fluidprobe, von der angenommen wird, dass sie mindestens einen Mikroorganismenstamm oder einen Zielzellanalyten enthält;
c) Inkontaktbringen der Fluidprobe mit dem Vliesartikel, sodass mindestens ein Teil des mindestens einen Mikroorganismenstammes oder Zielzellanalyten an den Vliesartikel gebunden ist, und Waschen des mindestens einen Vliesartikels, an den ein Mikroorganismenstamm oder ein Zielzellanalyt gebunden ist;
d) Inkontaktbringen des mindestens einen Vliesartikels, an den ein Mikroorganismenstamm oder ein Zielzellanalyt gebunden ist, mit mindestens einem Nachweisreagens; und

e) Nachweisen des Vorhandenseins des mindestens einen gebundenen Mikroorganismenstamms oder gebundenen Zielzellanalyten.

**2.** Das Verfahren nach Anspruch 1, wobei das Reagens ferner ein Lysemittel umfasst.

**3.** Das Verfahren nach Anspruch 2, wobei das Reagens Luciferase umfasst.

**4.** Das Verfahren nach einem der Ansprüche 1 bis 3, wobei das Einbringen das Einbringen des mindestens Vliesartikels, an den ein Mikroorganismenstamm oder ein Zielzellanalyt gebunden ist, in ein Behältnis umfasst, das ein Material umfasst, durch das ein Nachweissignal nachgewiesen werden kann, wobei das Behältnis das mindestens eine Reagens enthält.

**5.** Das Verfahren nach einem der Ansprüche 1 bis 4, wobei der Zielzellanalyt mindestens eine Nukleinsäure, ein Protein oder Adenosintriphosphat (ATP) umfasst.

**6.** Das Verfahren nach einem der Ansprüche 1 bis 5, wobei das Inkontaktbringen das Durchleiten der Fluidprobe durch den Vliesartikel bei einem Druck von 4,0 psi (27,6 kPa) oder weniger umfasst.

**7.** Das Verfahren nach einem der Ansprüche 1 bis 6, wobei der Vliesartikel eine Dicke zwischen 0,15 Millimeter und 2 Millimeter umfasst.

**8.** Verwendung einer Vorrichtung zum Inkontaktbringen einer Fluidprobe mit einem Vliesartikel in einem Verfahren nach einem der vorstehenden Ansprüche, wobei die Vorrichtung Folgendes umfasst: 1) einen Probenbehälter; 2) einen Filterhalter; 3) einen Vliesartikel, umfassend a) eine faserige poröse Matrix, wobei die Konzentrationsmittelteilchen Mikroorganismen und/oder Zielzellanalyten mit einer Bindungseffizienz von mindestens 60 Prozent binden; und wobei der Zielzellanalyt ein Bestandteil des Mikroorganismus ist; und b) eine Mehrzahl von Konzentrationsmittelteilchen, die in der faserigen porösen Matrix eingebunden sind; und 4) einen ersten Adapter, der konfiguriert ist, den Filterhalter mit einem Behältnis zu verbinden.

**9.** Verwendung einer Vorrichtung nach Anspruch 8, wobei der Probenbehälter Folgendes umfasst: einen freistehenden Behälter, umfassend ein erstes Reservoir; ein verformbares, selbsttragendes Behältnis, das so bemessen ist, dass es in dem ersten Reservoir des freistehenden Behälters aufgenommen werden kann, und das ein zweites Reservoir umfasst, wobei der freistehende Behälter steifer ist als der verformbare, selbsttragende Behälter, wobei der freistehende Behälter eine Basis mit einer darin ausgebildeten Öffnung aufweist, durch die das verformbare, selbsttragende Behältnis zugänglich ist.

**10.** Verwendung einer Vorrichtung nach Anspruch 8, ferner umfassend einen zweiten Adapter, der konfiguriert ist, den Filterhalter an einer Vakuumquelle, an einem Sammelbehälter oder an dem Probenbehälter zu befestigen.

**11.** Verwendung einer Vorrichtung nach einem der Ansprüche 8 bis 10, wobei es sich bei der faserigen porösen Matrix um eine Vliesfaserschicht handelt, die ungekräuselte Polymerfasern umfasst.

**12.** Verwendung einer Vorrichtung nach einem der Ansprüche 8 bis 11, wobei die faserige poröse Matrix frei von Polyamidfasern ist.

**13.** Verwendung einer Vorrichtung nach einem der Ansprüche 8 bis 12, wobei die Konzentrationsmittelteilchen amorphe Metallsilikate, Guanidin-funktionalisierte Metallsilikate, Diatomeenerde, oberflächenmodifizierte Diatomeenerde, Guanidin-funktionalisierte Diatomeenerde, gamma-FeO(OH), Metallcarbonate, Metallphosphate, Siliciumdioxid, Perlit, Guanidin-funktionalisiertes Perlit oder eine Kombination davon umfassen.

**14.** Verwendung einer Vorrichtung zum Inkontaktbringen einer Fluidprobe mit einem Vliesartikel in einem Verfahren nach einem der vorstehenden Ansprüche, wobei die Vorrichtung Folgendes umfasst: 1) einen Probenbehälter; 2) einen Filterhalter, der konfiguriert ist, mit einem Behältnis verbunden zu werden; und 3) einen Vliesartikel, umfassend a) eine faserige poröse Matrix und b) eine Mehrzahl von Konzentrationsmittelteilchen, die in die faserige poröse Matrix eingebunden sind, wobei die Konzentrationsmittelteilchen Mikroorganismen und/oder Zielzellanalyten mit einer Bindungseffizienz von mindestens 60 Prozent binden; und wobei der Zielzellanalyt ein Bestandteil des Mikroorganismus ist.

**15.** Verwendung eines Kits in einem Verfahren nach einem der vorhergehenden Ansprüche, wobei das Kit Folgendes umfasst:

a. eine Vorrichtung zum Inkontaktbringen einer Fluidprobe mit einem Vliesartikel, umfassend: 1) einen Probenbehälter; 2) einen Filterhalter; 3) einen Vliesartikel, umfassend i) eine faserige poröse Matrix und ii) eine Mehrzahl von Konzentrationsmittelteilchen, die in der faserigen porösen Matrix eingebunden sind; wobei die Konzentrationsmittelteilchen Mikroorganismen und/oder Zielzellanalyten mit einer Bindungseffizienz von mindestens 60 Prozent binden; und wobei der Zielzellanalyt ein Bestandteil des Mikroorganismus ist; und 4) einen ersten Adapter; und
b. ein Behältnis, wobei der erste Adapter konfiguriert ist, den Filterhalter mit dem Behältnis zu verbinden.

**Revendications**

**1.** Procédé de détection de micro-organismes dans un échantillon de fluide, le procédé comprenant :

a) la fourniture d'un article non tissé comprenant 1) une matrice poreuse fibreuse et 2) une pluralité de particules d'agent de concentration emmaillées dans la matrice poreuse fibreuse, dans lequel les particules d'agent de concentration lient des micro-organismes et/ou des analytes cellulaires cibles d'un échantillon de fluide ayant une efficacité de liaison d'au moins 60 pour cent ;
et dans lequel l'analyte cellulaire cible est un composant du micro-organisme ;
b) la fourniture d'un échantillon de fluide soupçonné de contenir au moins une souche de micro-organisme ou un analyte cellulaire cible ;
c) la mise en contact de l'échantillon de fluide avec l'article non tissé de telle sorte qu'au moins une partie de l'au moins une souche de micro-organisme ou un analyte cellulaire cible soit liée à l'article non tissé, et le lavage de l'au moins un article non tissé lié à une souche de micro-organisme ou à un analyte cellulaire cible ;
d) l'entrée en contact de l'au moins un article non tissé lié à une souche de micro-organisme ou à un analyte cellulaire cible avec au moins un réactif de détection ; et
e) la détection de la présence de l'au moins une souche de micro-organisme liée ou un analyte cellulaire cible lié.

**2.** Procédé selon la revendication 1, dans lequel le réactif comprend en outre un agent de lyse.

**3.** Procédé selon la revendication 2, dans lequel le réactif comprend de la luciférase.

**4.** Procédé selon l'une quelconque des revendications 1 à 3, dans lequel la mise en place comprend la mise en place de l'au moins un article non tissé lié à une souche de micro-organisme ou à un analyte cellulaire cible dans un réceptacle comprenant un matériau à travers lequel un signal de détection peut être détecté, le réceptacle contenant l'au moins un réactif.

**5.** Procédé selon l'une quelconque des revendications 1 à 4, dans lequel l'analyte cellulaire cible comprend au moins un acide nucléique, une protéine, ou de l'adénosine triphosphate (ATP).

**6.** Procédé selon l'une quelconque des revendications 1 à 5, dans lequel la mise en contact comprend le passage de l'échantillon de fluide à travers l'article non tissé à une pression de 4,0 psi (27,6 kPa) ou moins.

**7.** Procédé selon l'une quelconque des revendications 1 à 6, dans lequel l'article non tissé comprend une épaisseur entre 0,15 millimètres et 2 millimètres.

**8.** Utilisation d'un dispositif pour la mise en contact d'un échantillon de fluide avec un article non tissé dans un procédé selon l'une quelconque des revendications précédentes, le dispositif comprenant : 1) un récipient d'échantillon ; 2) un porte-filtre ; 3) un article non tissé comprenant a) une matrice poreuse fibreuse dans laquelle les particules d'agent de concentration lient des micro-organismes et/ou des analytes cellulaires cibles avec une efficacité de liaison d'au moins 60 pour cent ; et dans laquelle l'analyte cellulaire cible est un composant du micro-organisme ; et b) une pluralité de particules d'agent de concentration emmaillées dans la matrice poreuse fibreuse ; et 4) un premier adaptateur configuré pour raccorder le porte-filtre à un réceptacle.

**9.** Utilisation d'un dispositif selon la revendication 8, dans laquelle le récipient d'échantillon comprend : un récipient indépendant comprenant un premier réservoir ; un réceptacle autoporteur déformable dimensionné pour être reçu

dans le premier réservoir du récipient indépendant et comprenant un deuxième réservoir, le récipient indépendant étant plus rigide que le réceptacle autoporteur déformable, le récipient indépendant incluant une base comprenant une ouverture formée dans celle-ci à travers laquelle le réceptacle autoporteur déformable peut être accédé.

**10.** Utilisation d'un dispositif selon la revendication 8, comprenant en outre un deuxième adaptateur configuré pour fixer le porte-filtre à une source de vide, à un récipient de collecte, ou au récipient d'échantillon.

**11.** Utilisation d'un dispositif selon l'une quelconque des revendications 8 à 10, dans laquelle la matrice poreuse fibreuse est une couche fibreuse non tissée comprenant des fibres polymères non crêpées.

**12.** Utilisation d'un dispositif selon l'une quelconque des revendications 8 à 11, dans laquelle la matrice poreuse fibreuse est exempte de fibres de polyamide.

**13.** Utilisation d'un dispositif selon l'une quelconque des revendications 8 à 12, dans laquelle les particules d'agent de concentration comprennent des silicates de métal amorphes, des silicates de métal fonctionnalisés par la guanidine, de la terre à diatomées, de la terre à diatomées modifiée en surface, de la terre à diatomées fonctionnalisée par la guanidine, gamma-FeO(OH), des carbonates de métal, des phosphates de métal, du silicium, de la perlite, de la perlite fonctionnalisée par la guanidine, ou une combinaison de ceux-ci.

**14.** Utilisation d'un dispositif pour la mise en contact d'un échantillon de fluide avec un article non tissé dans un procédé selon l'une quelconque des revendications précédentes, le dispositif comprenant : 1) un récipient d'échantillon ; 2) un porte-filtre configuré pour être raccordé à un réceptacle ; et 3) un article non tissé comprenant a) une matrice poreuse fibreuse et b) une pluralité de particules d'agent de concentration emmaillées dans la matrice poreuse fibreuse, dans laquelle les particules d'agent de concentration lient des micro-organismes et/ou des analytes cellulaires cibles avec une efficacité de liaison d'au moins 60 pour cent ; et dans laquelle l'analyte cellulaire cible est un composant du micro-organisme.

**15.** Utilisation d'une trousse dans un procédé selon l'une quelconque des revendications précédentes, la trousse comprenant :

a. un dispositif pour mettre en contact un échantillon de fluide avec un article non tissé comprenant : 1) un récipient d'échantillon ; 2) un porte-filtre ; 3) un article non tissé comprenant i) une matrice poreuse fibreuse et ii) une pluralité de particules d'agent de concentration emmaillées dans la matrice poreuse fibreuse ; dans laquelle les particules d'agent de concentration lient des micro-organismes et/ou des analytes cellulaires cibles avec une efficacité de liaison d'au moins 60 pour cent ; et dans laquelle l'analyte cellulaire cible est un composant du micro-organisme ; et 4) un premier adaptateur ; et
b. un réceptacle, le premier adaptateur configuré pour raccorder le porte-filtre au réceptacle.

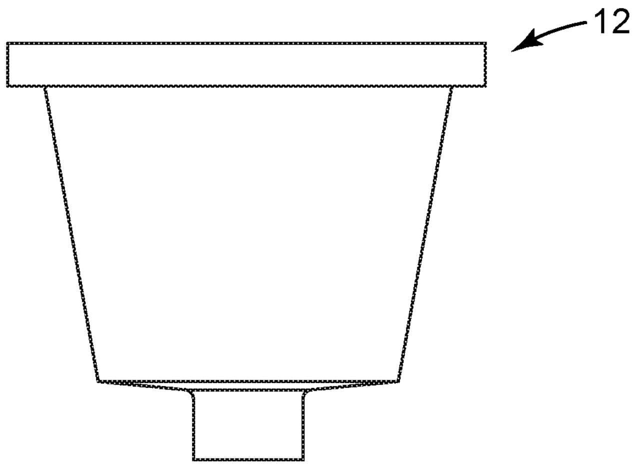


Fig. 1A

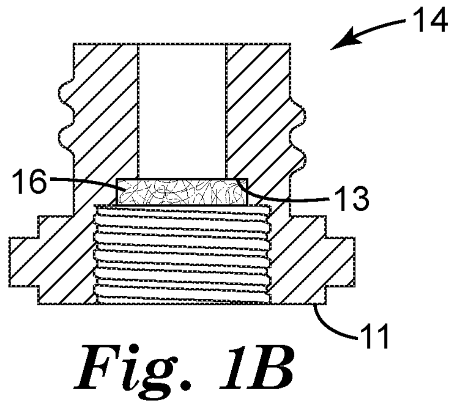


Fig. 1B

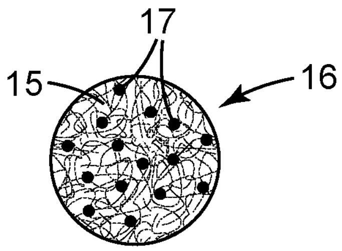


Fig. 1C

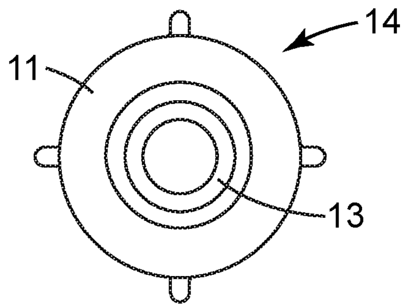


Fig. 1D

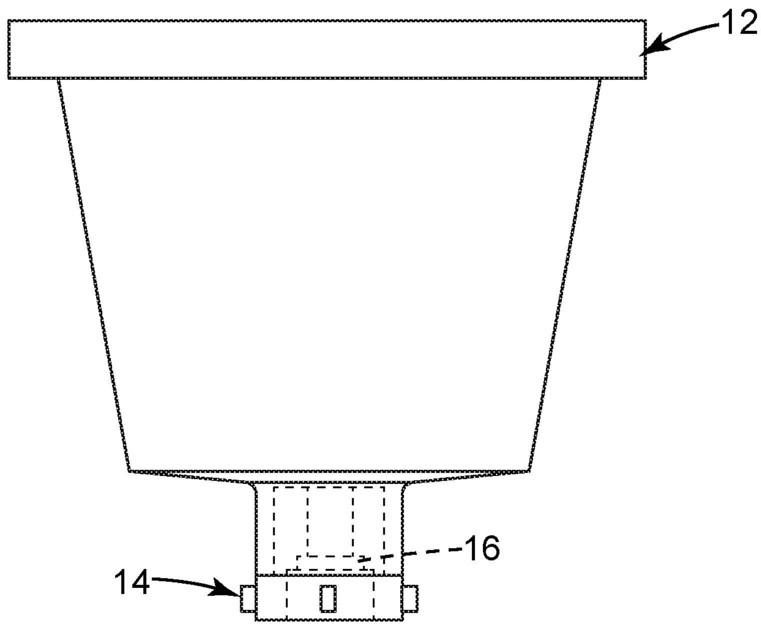


Fig. 1E

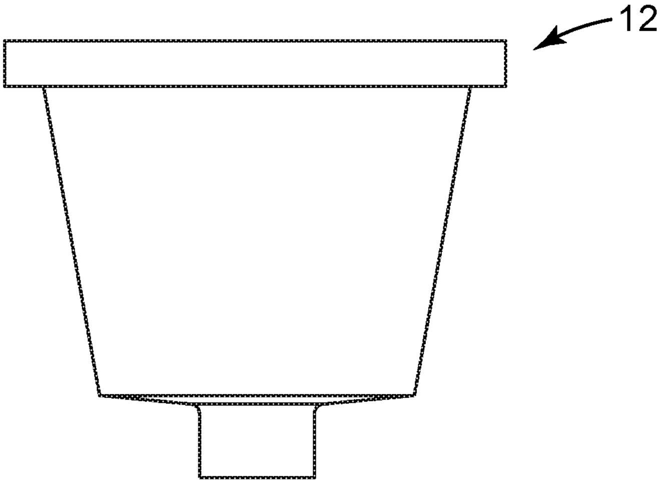


Fig. 2A

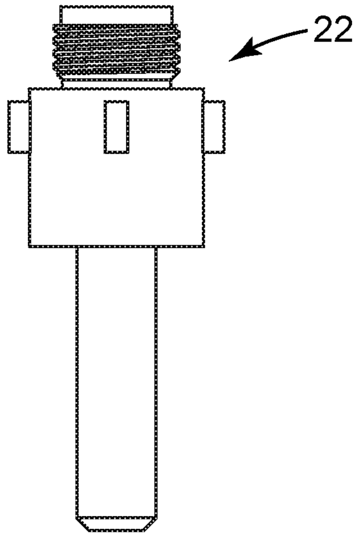


Fig. 2B

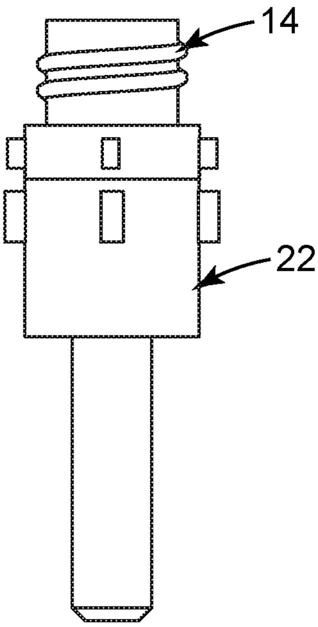


Fig. 2C

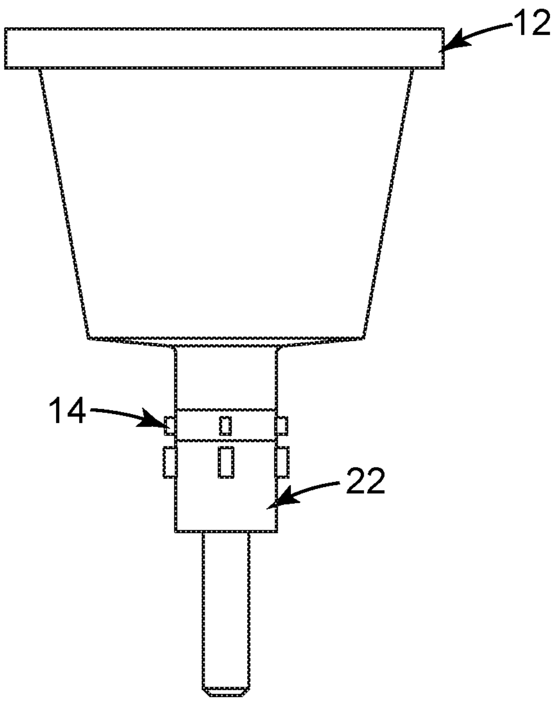


Fig. 2D

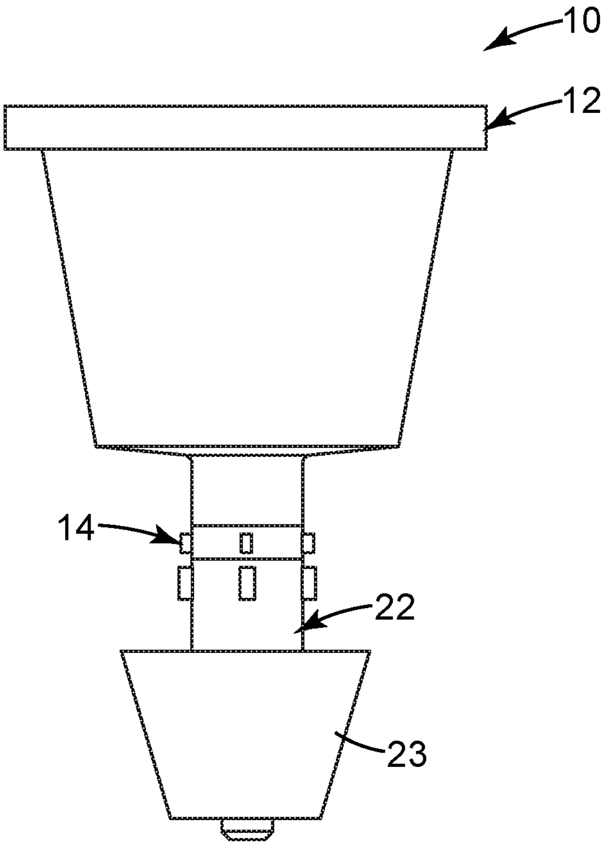


*Fig. 2E*

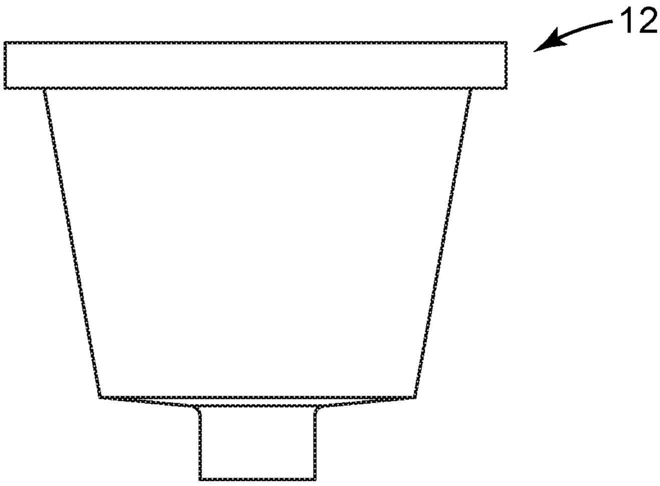


Fig. 3A

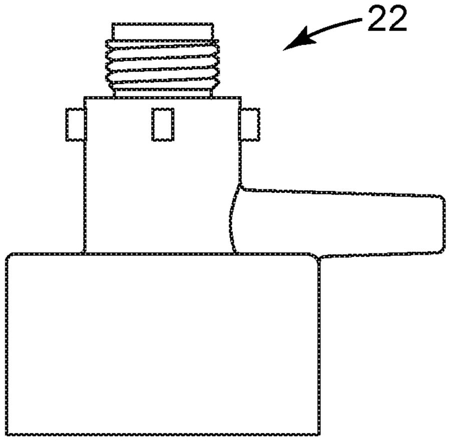


Fig. 3B

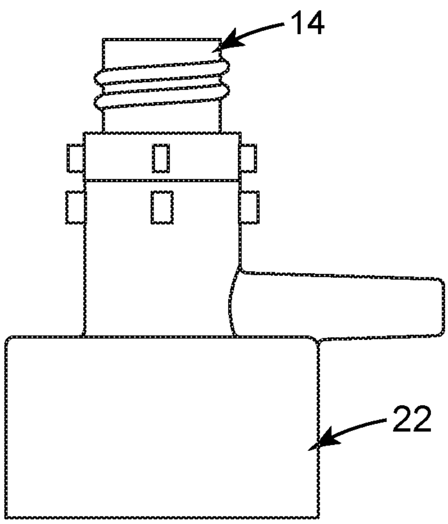


Fig. 3C

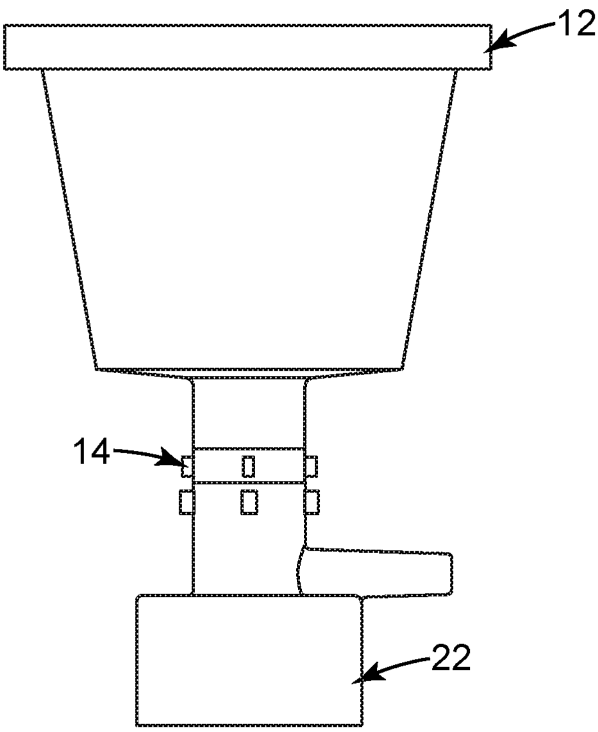


Fig. 3D

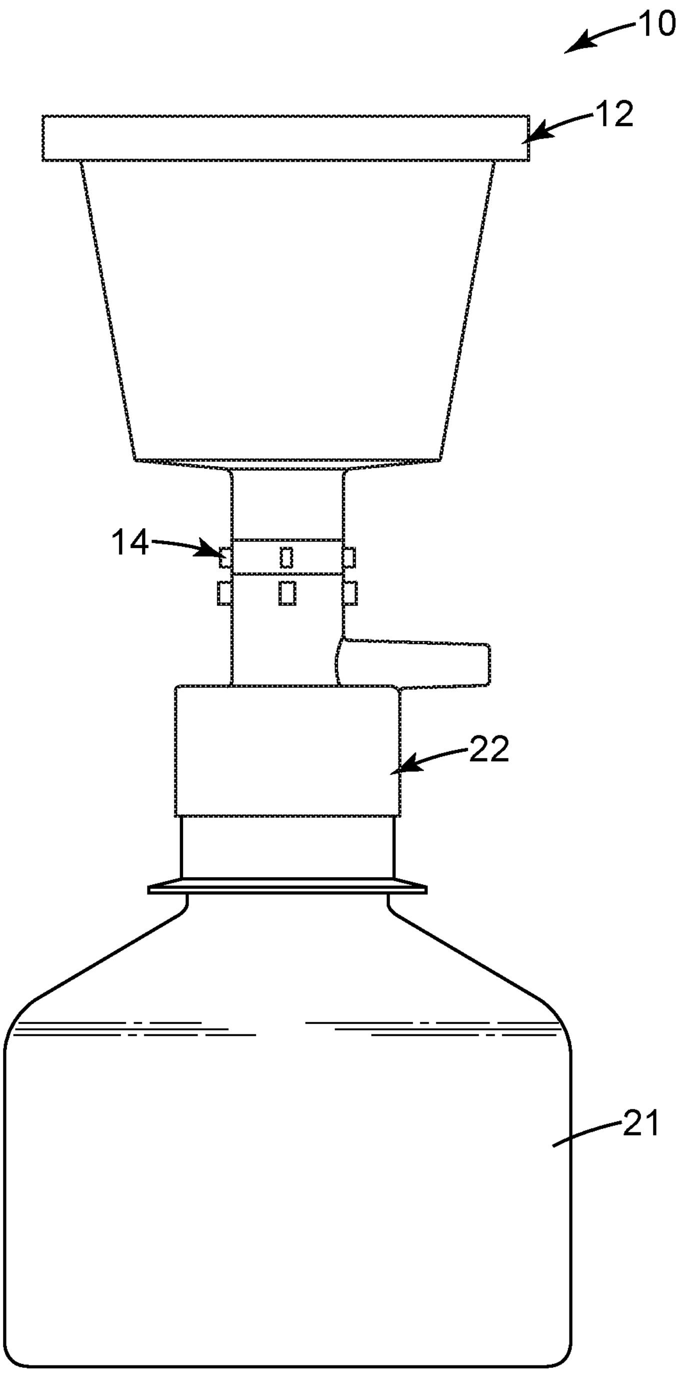


Fig. 3E

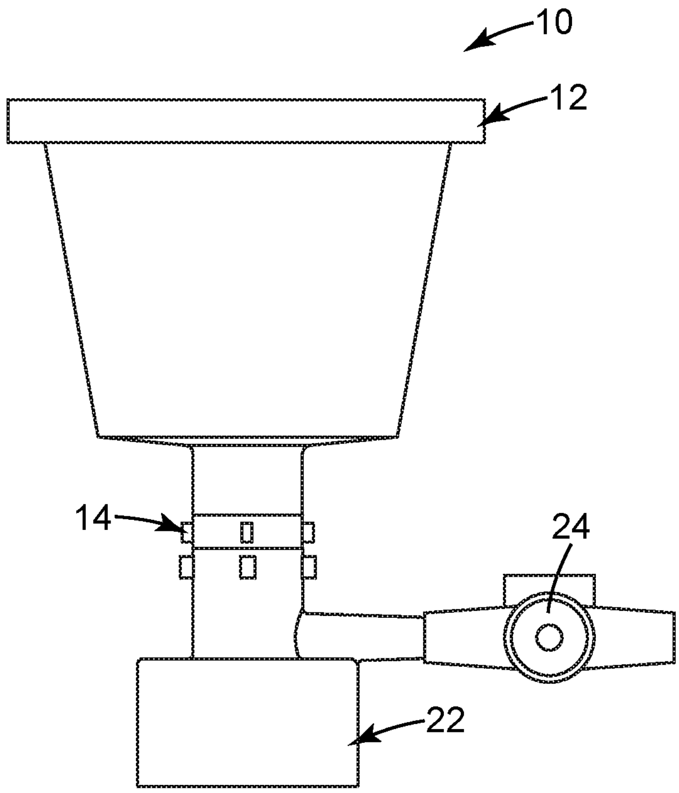


*Fig. 4A*

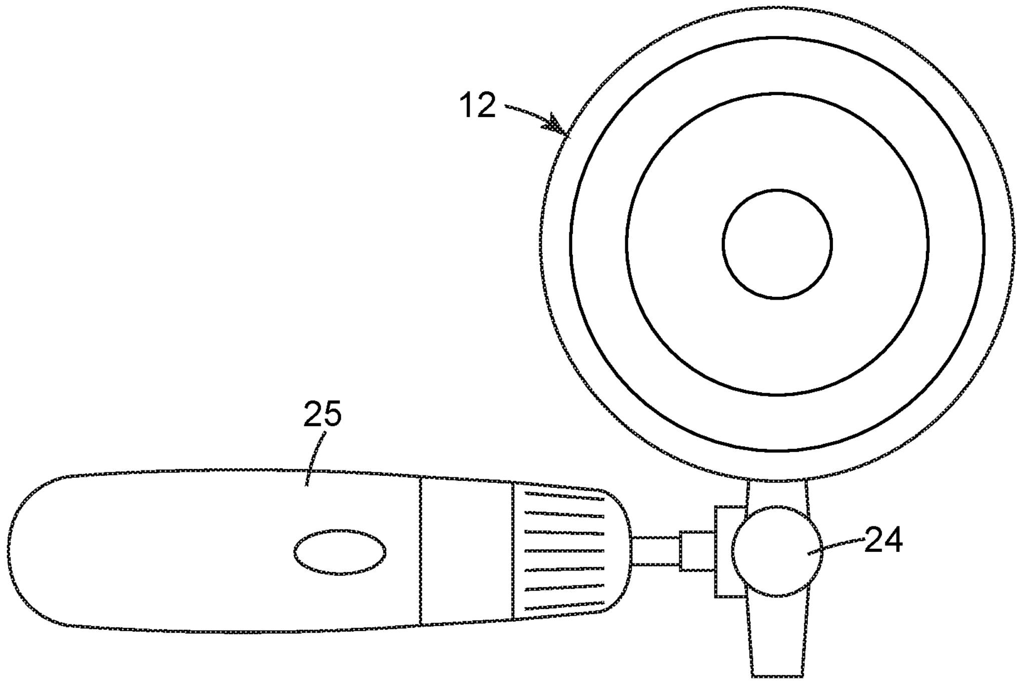


*Fig. 4B*

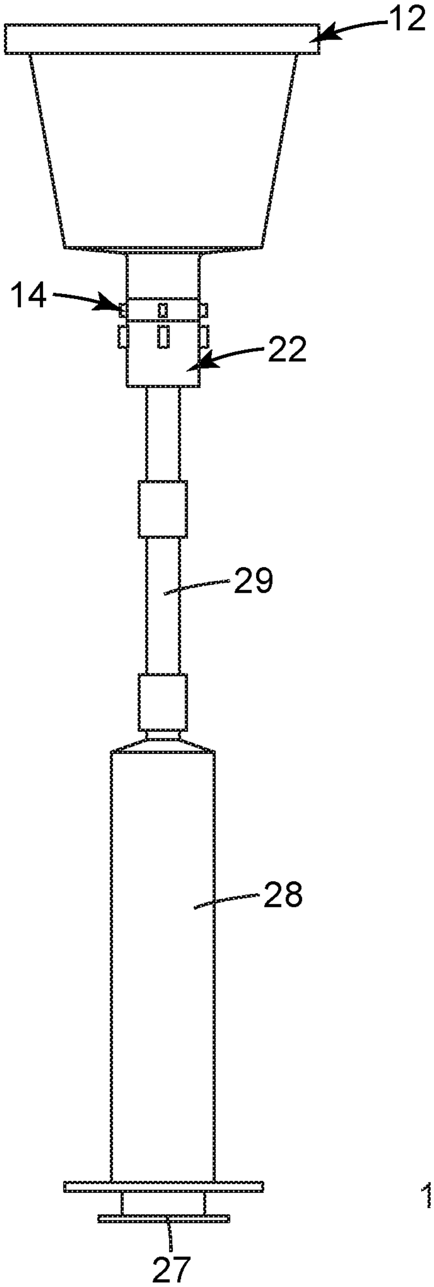
12
14
22
29
28
27

*Fig. 5*

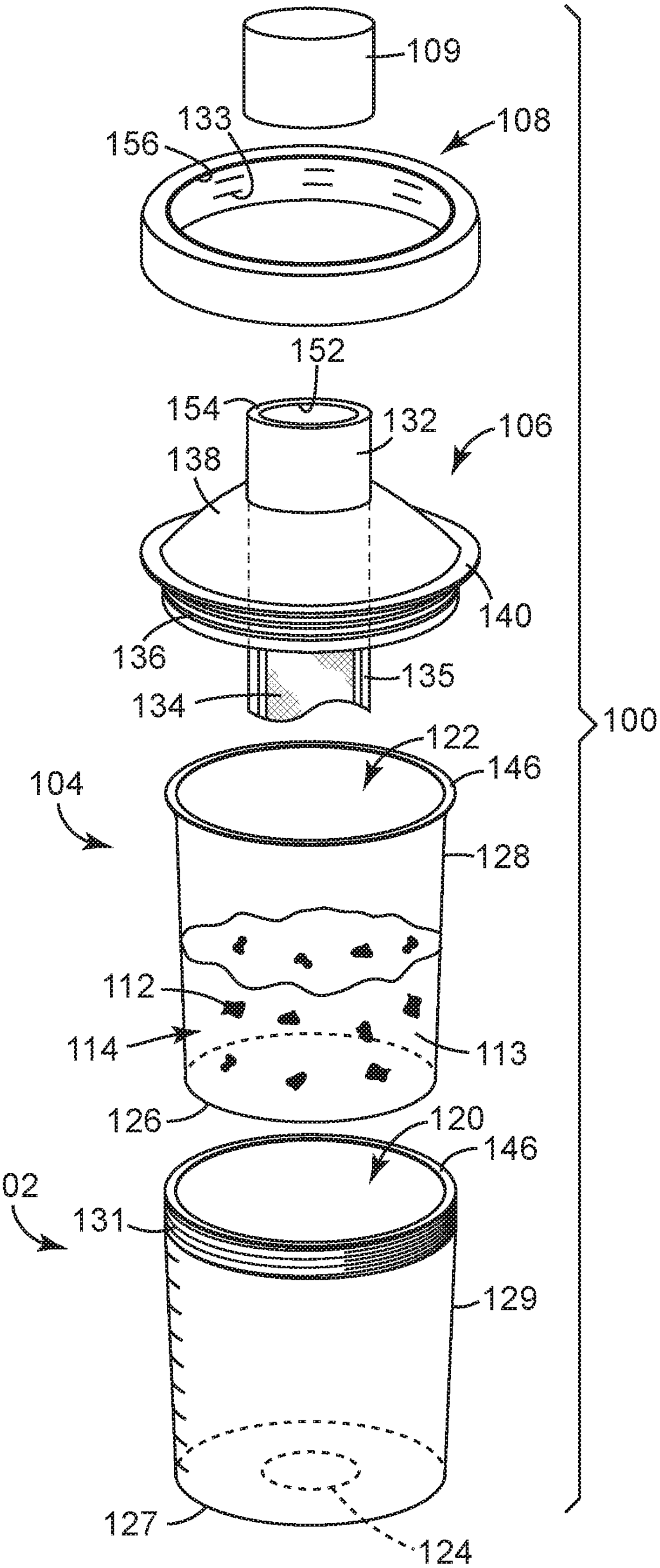
109
108
133
156
152
154
132
106
138
140
136
135
134
100
122
146
104
128
112
114
113
126
120
146
102
131
129
127
124

*Fig. 6*

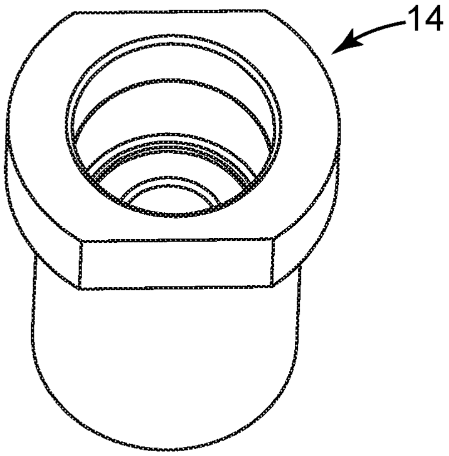


Fig. 7A

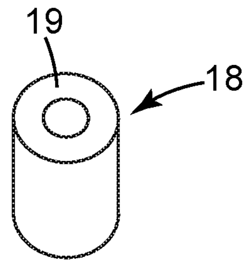


Fig. 7B

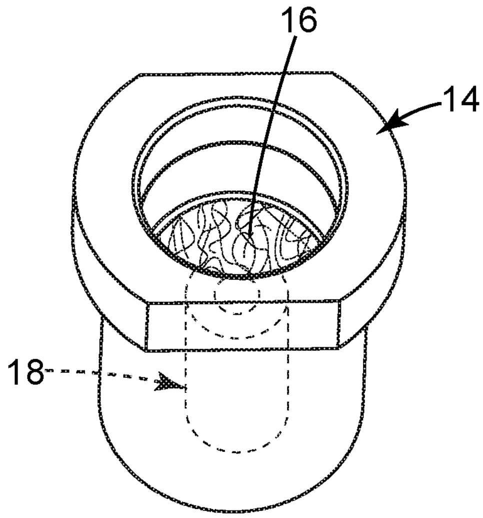


Fig. 7C

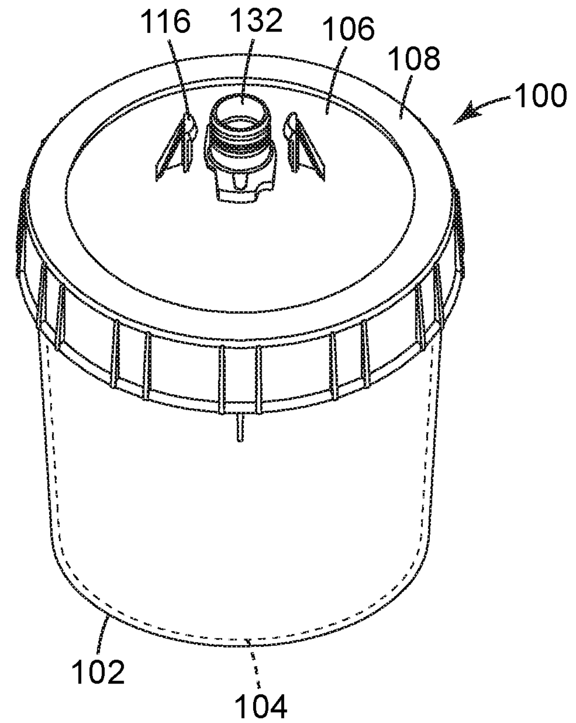


*Fig. 7D*

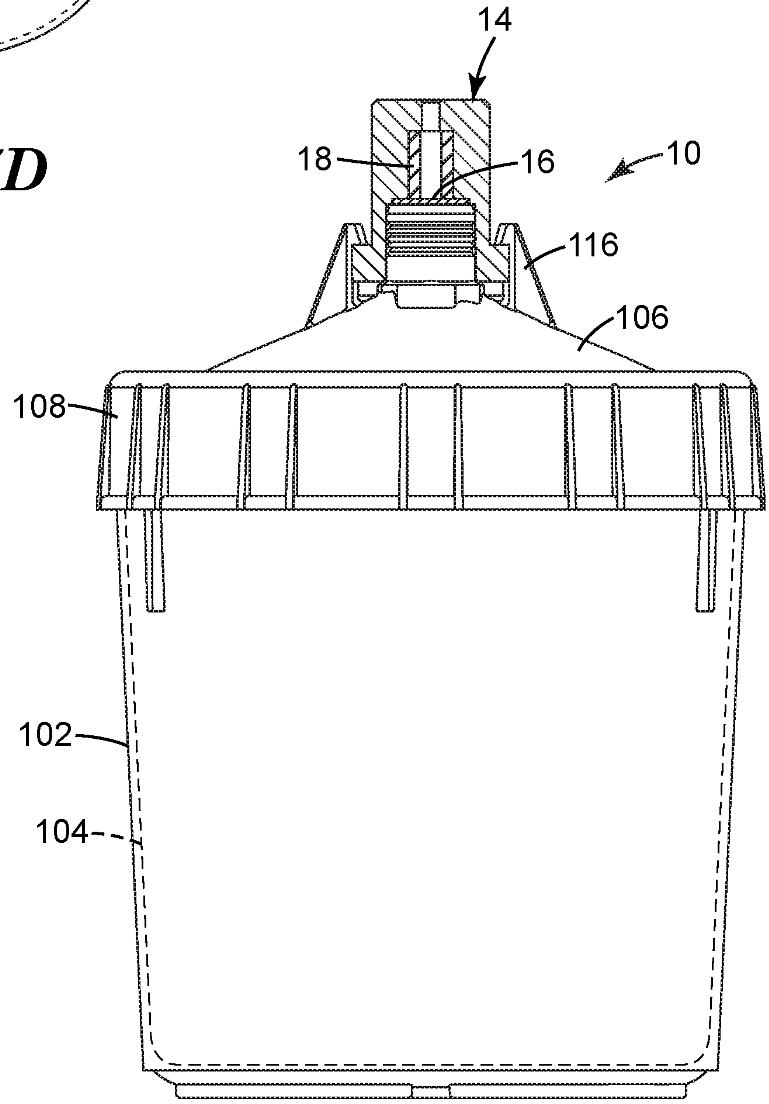


*Fig. 7E*

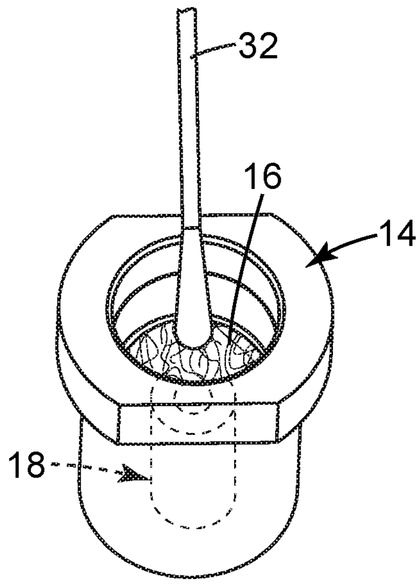


Fig. 8A

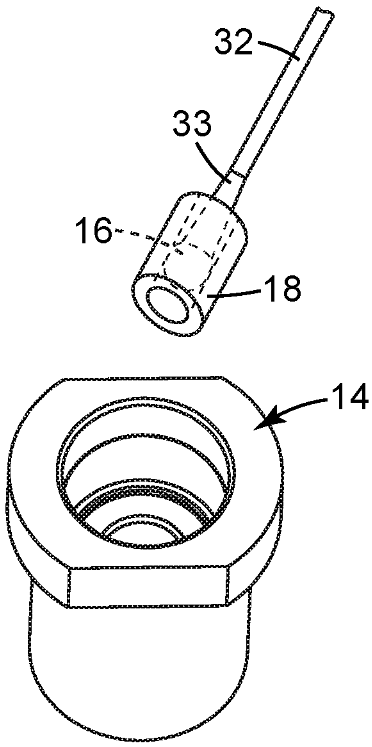


Fig. 8B

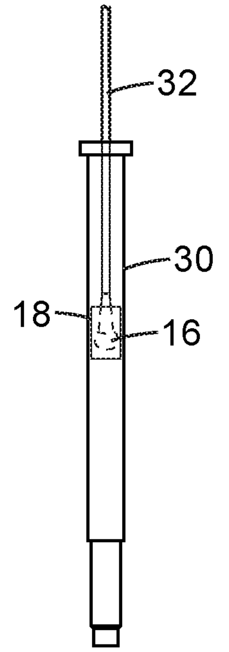


Fig. 8C

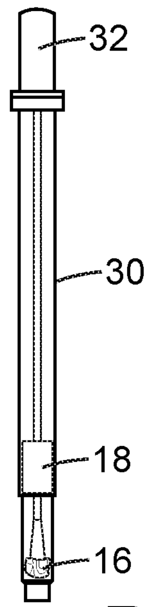


Fig. 8D

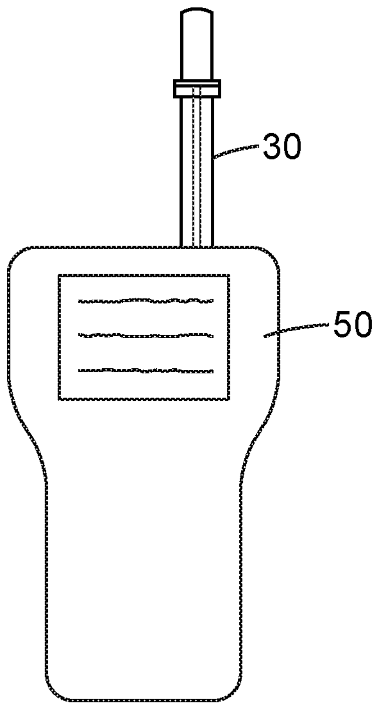


Fig. 8E

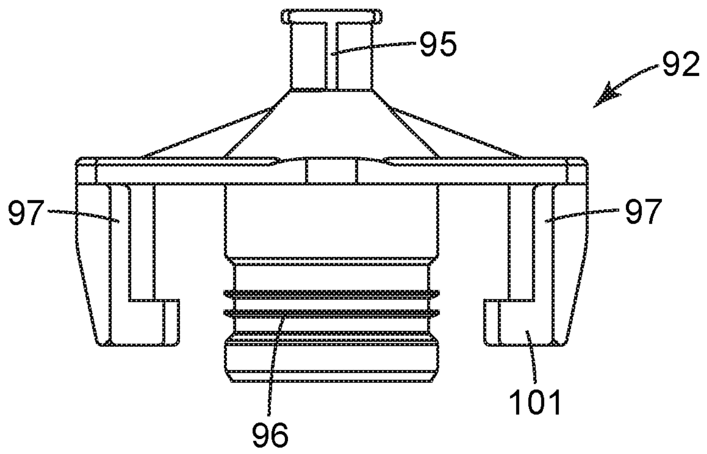


Fig. 9A

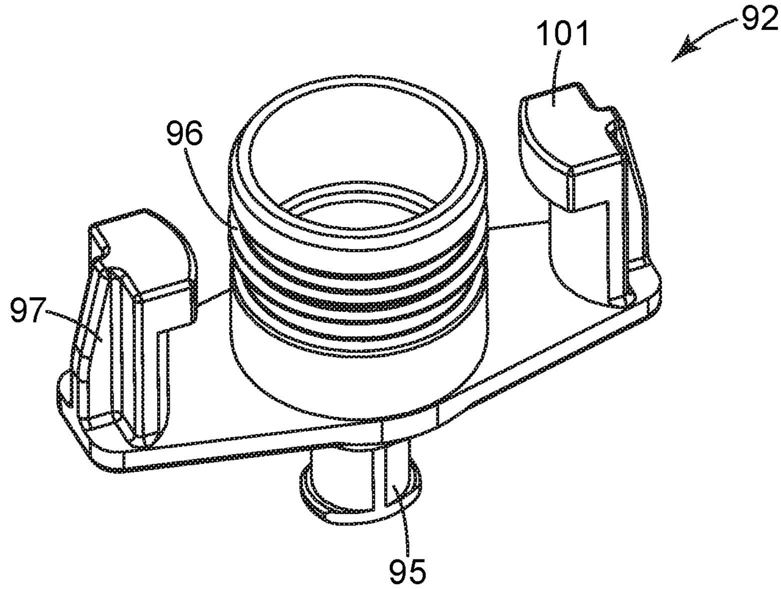


Fig. 9B

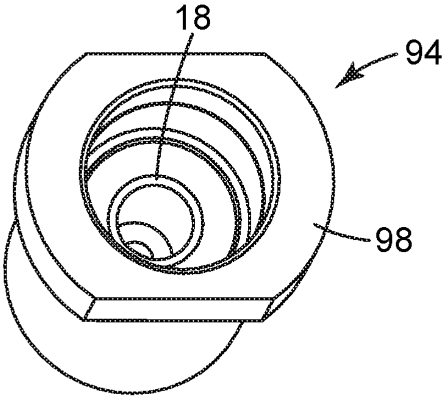


Fig. 9C

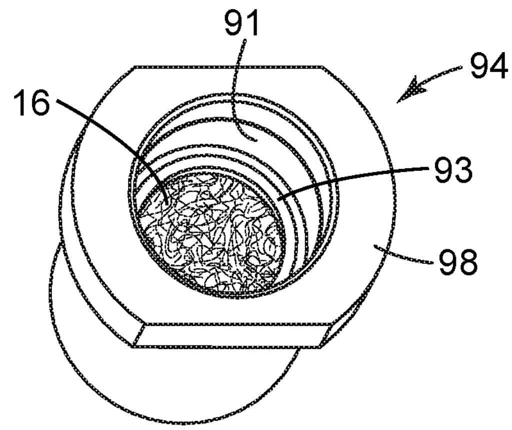


*Fig. 9D*

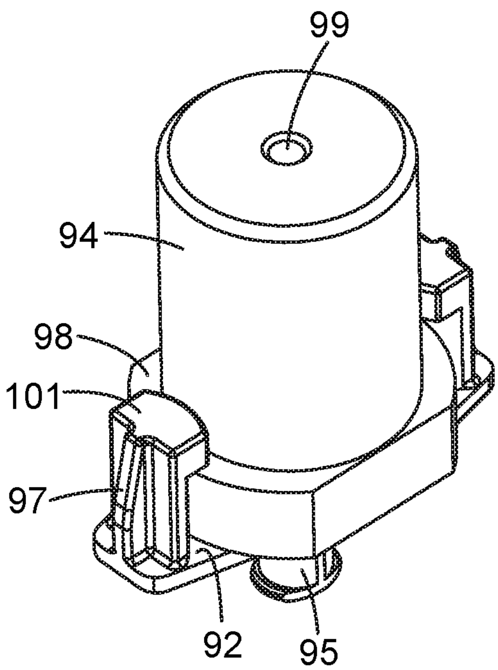


*Fig. 9E*

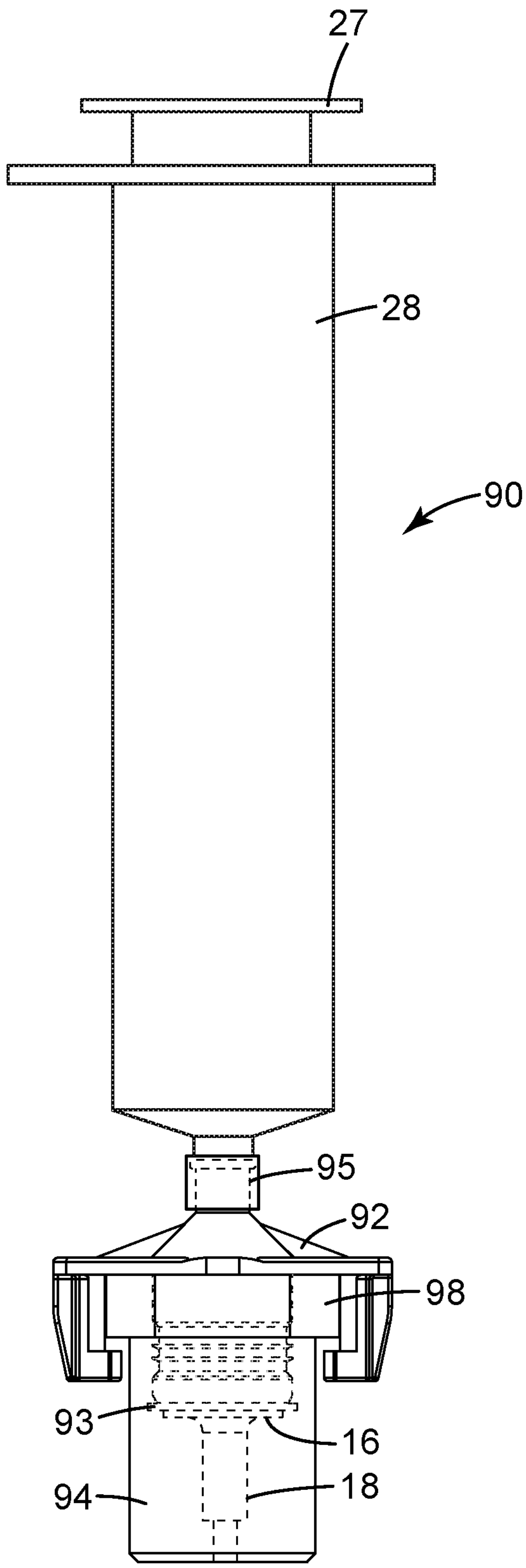


*Fig. 9F*

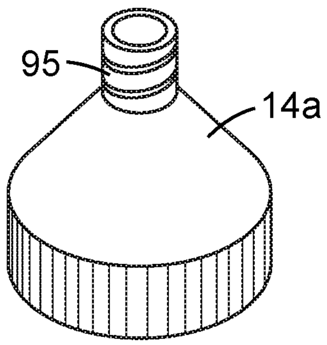


Fig. 10A

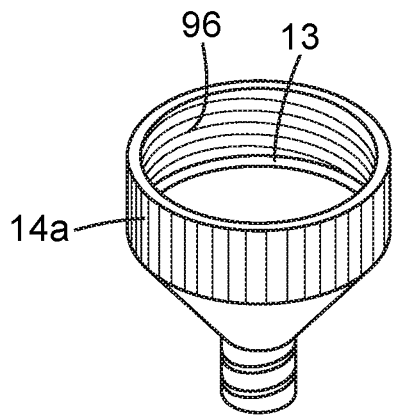


Fig. 10B

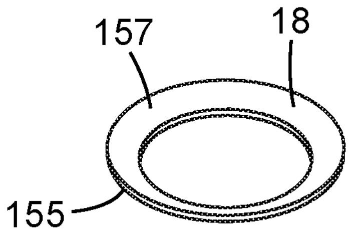


Fig. 10C

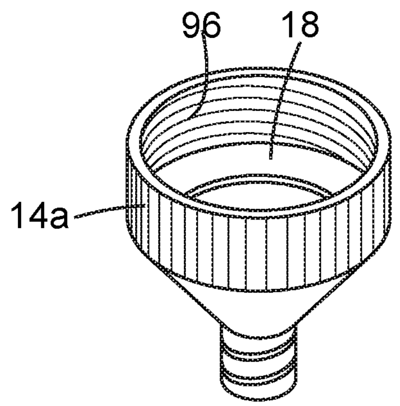


Fig. 10D

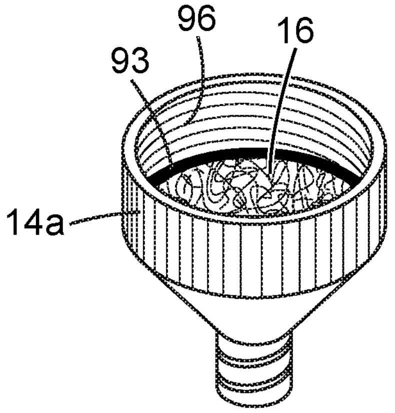


Fig. 10E

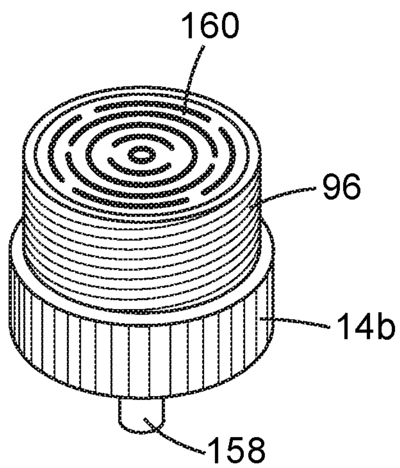


Fig. 10F

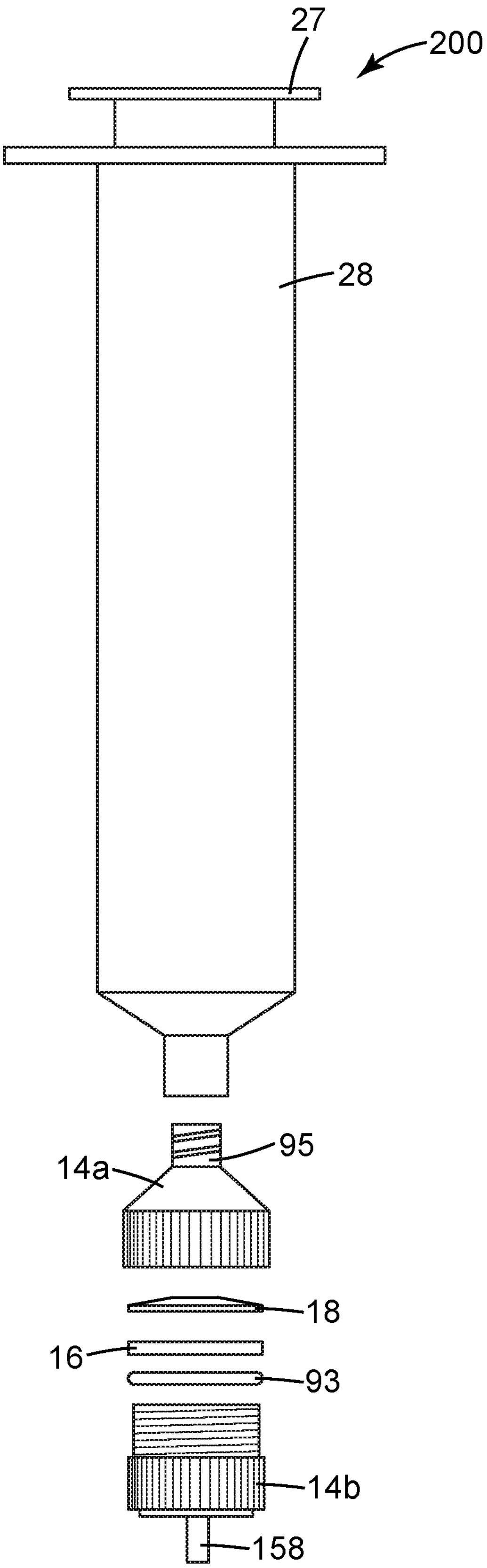


Fig. 10G

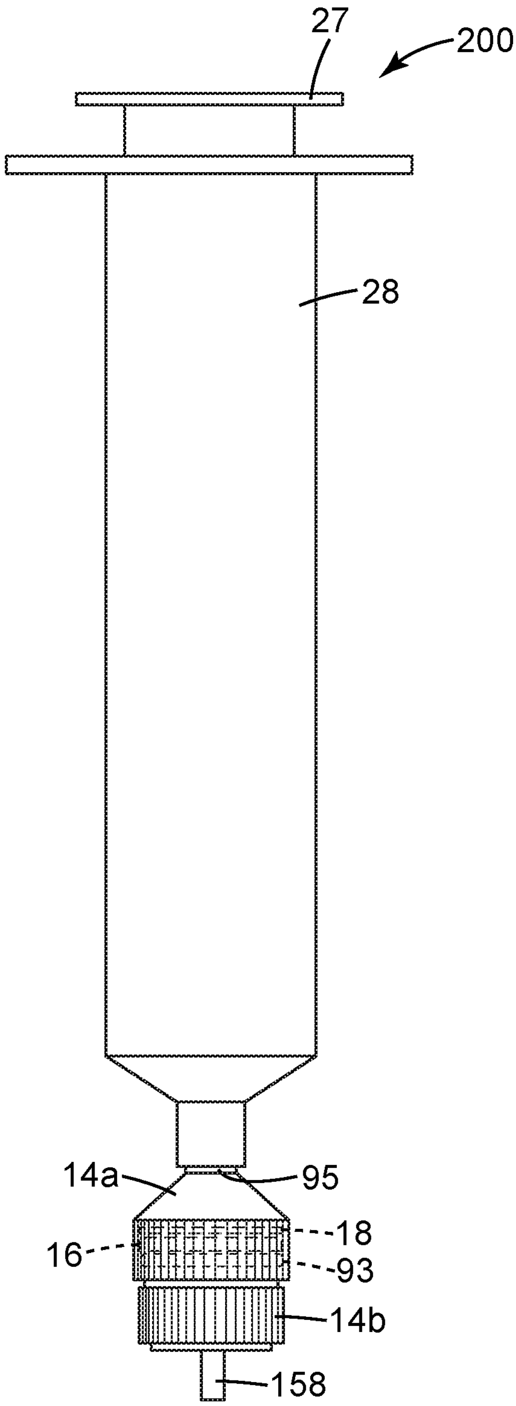


Fig. 10H

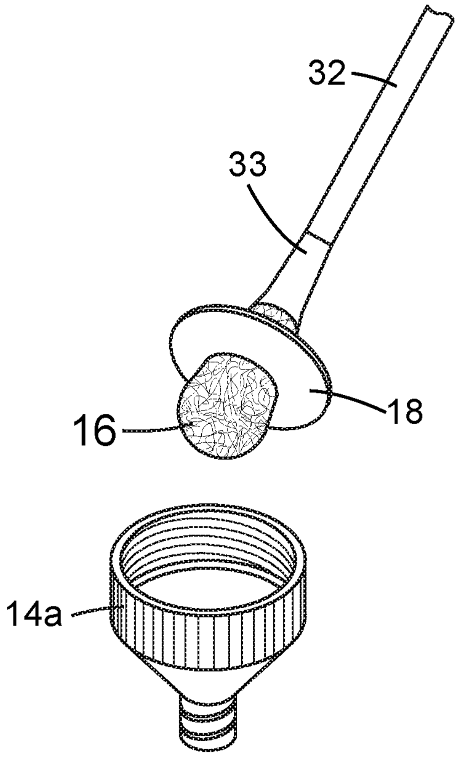


*Fig. 11A*

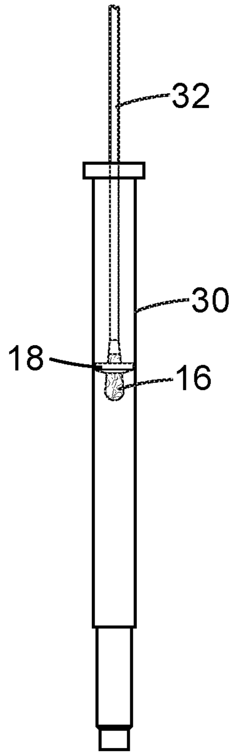


*Fig. 11B*

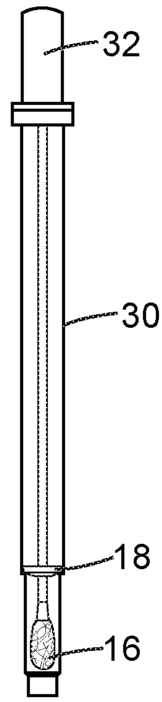


*Fig. 11C*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2013260370 A1
- US 2013244225 A1
- WO 2013184186 A1
- WO 2012012172 A1
- US 7422868 B, Fan
- US 4118531 A, Hauser
- US 5597645 A, Pike
- CA 2612854, Sommer
- US 6045913 A, Castle
- US 2014040861 W, Kshirsagar
- WO 2009046191 A, Kshirsagar
- WO 2009046183 A, Kshirsagar
- US 4729846 A, Matsui
- WO 9832539 A
- US 6536687 B
- US 6588681 B
- US 2004060574 W
- US 2004060575 W
- US 20040164182 A
- US 2004094072 W
- WO 2007079143 A
- WO 2007079188 A
- WO 2009067498 A


### Non-patent literature cited in the description


- **SPYCHALA.** *Environ Tech,* 2015, vol. 36 (8), 937-945